# EUROPEAN PATENT APPLICATION

(11) **EP 4 817 637 A1**
(43) Date of publication of application: **07.10.2026**
(21) Application number: 24896725.9
(22) Date of filing: 29.11.2024
(51) Int. Cl.: A61K 47/68, A61P 35/00, C07K 16/28, C12N 15/85, C12N 5/10, A61K 39/395, A61P 31/00

(54) **COMBINATION OF PD-L1 BINDING MOLECULE AND PROTEIN-DRUG CONJUGATE, AND USE THEREOF**

(30) Priority: 30.11.2023 CN 202311629115; 11.09.2024 CN 202411273758
(71) Applicant: Jiangsu Alphamab Biopharmaceuticals Co., Ltd., Jiangsu 215000 (CN)
(72) Inventor: XU, Ting, Suzhou, Jiangsu 215000 (CN); LIU, Yang, Suzhou, Jiangsu 215000 (CN); MENG, Fanyu, Suzhou, Jiangsu 215000 (CN); ZHAO, Xin, Suzhou, Jiangsu 215000 (CN); YU, Qiuling, Suzhou, Jiangsu 215000 (CN); WANG, Lele, Suzhou, Jiangsu 215000 (CN)
(74) Representative: Latscha Schöllhorn Partner AG
(86) International application number: PCT/CN2024/135699
(87) International publication number: WO 2025/113644

(57) **Abstract**

The present invention relates to the use of a PD-L1 binding molecule in improving the stability of a protein-drug conjugate formulation. The present invention also relates to a pharmaceutical composition consisting of the PD-L1 binding molecule and the protein-drug conjugate, and discloses an anti-tumor use thereof.

## Description

### TECHNICAL FIELD

The present application relates to the field of biopharmaceuticals and particularly to a combination of a PD-L1-binding molecule and a protein-drug conjugate and use thereof.

### BACKGROUND

Antibody drugs are widely used in tumor therapy. Since antibodies can selectively bind to antigens or antigen-positive cells, they show relatively low off-target toxicity. However, antibody drugs alone are often insufficient to effectively combat tumors. Antibody-drug conjugates (ADCs) combine the advantages of antibodies and cytotoxins, enhancing the effectiveness and selectivity of tumor cell killing. Since the approval of the first ADC drug, Mylotarg, in 2000, 15 ADC drugs have been approved, with many more targeting different targets and utilizing different conjugation technologies currently under clinical investigation.

In the process of bringing clinically effective ADCs to the market as products, it is necessary to develop pharmaceutical formulations suitable for storage, transportation, and administration. However, proteins are prone to chemical and physical degradation, and the conjugation of small molecules also introduces more complexity and instability to antibodies. Marketed ADC drugs generally have concentrations lower than 20 mg/mL, in sharp contrast to the concentrations higher than 50 mg/mL in conventional antibody therapies. The lower drug concentrations can reduce the risk of aggregation, especially for drugs carrying hydrophobic payloads. Existing ADC drugs are usually administered by intravenous infusion. In infusion bags, the low ADC concentrations increase the risk of drug loss due to adsorption to plastic. Dilution of the drugs also reduces the levels of stabilizers, which may lead to aggregation and particle formation. In addition, intravenous infusion often requires prolonged administration times. Generally, liquid formulations are less costly to produce and more convenient to use compared to freeze-dried products. However, due to the structural complexity and stability of products, they can only be formulated and used at relatively low concentrations most of the time, and need to be made into freeze-dried powders for long-term storage. Currently, all approved ADC drugs are stored and transported in freeze-dried form and are reconstituted before use.

Programmed death-1 (PD-1) is a member of the CD28 receptor family, which includes CD28, CTLA-4, ICOS, PD-1, and BTLA. The earliest identified members of this family, CD28 and ICOS, were discovered for their function of enhancing T cell proliferation following the addition of monoclonal antibodies. Two cell-surface glycoprotein ligands of PD-1-PD-L1 and PD-L2-have been identified. They have been shown to downregulate T cell activation and cytokine secretion upon binding to PD-1. Both PD-L1 (B7-H1) and PD-L2 (B7-DC) are homologs of B7 that bind to PD-1 but not to other members of the CD28 family. It has also been demonstrated that PD-L1 expression on the cell surface is upregulated through IFN-γ stimulation.

PD-L1 expression has been found in several murine and human cancer types, including human lung cancer, ovarian cancer, colon cancer, melanoma, and various myelomas. Existing results show that PD-L1, highly expressed by tumor cells, plays an important role in tumor immune escape by increasing T cell apoptosis. Researchers have found that P815 tumor cell lines transfected with the PD-L1 gene resisted lysis by specific CTLs *in vitro,* and after being inoculated into mice, these cells exhibited greater tumorigenicity and invasiveness. These biological properties can all be reversed by blocking PD-L1. In PD-1 gene knockout mice, blocking the PD-L1/PD-1 pathway prevents inoculated tumor cells from forming tumors. It has also been suggested that PD-L1 may be involved in intestinal mucosal inflammation, and inhibition of PD-L1 prevents colitis-related atrophy. Patent Application WO2017020801 discloses a class of PD-L1-targeting single-domain antibodies and their anti-tumor use.

In view of the structures and instability of ADC drugs that have been developed in the prior art, there is a need in the prior art to develop techniques for improving the stability of ADC drugs, and/or to obtain ADC drug-containing formulations suitable for long-term stable storage, such as high-concentration injectable solutions.

### SUMMARY

The inventors have surprisingly found that single-domain antibody molecules, such as anti-PD-L1 single-domain antibodies, can improve the stability of ADC drugs. For example, after a single-domain antibody molecule (e.g., an anti-PD-L1 single-domain antibody) is combined with an ADC drug, the stability of the ADC drug is significantly improved, which enables the formulation of a high-concentration injectable solution and long-term stable storage. The high-concentration injectable solution can be used for subcutaneous injection, which greatly facilitates clinical administration to patients and is also conducive to the use of the ADC drug in combination with immunotherapy.

In a first aspect, the present disclosure relates to use of an antigen (e.g., PD-L1)-binding molecule in improving the stability of a protein-drug conjugate formulation.

In a second aspect, the present disclosure further relates to a composition comprising an antigen (e.g., PD-L1)-binding molecule and a protein-drug conjugate. The composition may be in the form of a solid formulation, such as a freeze-dried formulation, or in the form of a liquid formulation, such as an injectable solution, and may further comprise suitable pharmaceutical auxiliary materials. The composition is suitable for clinical use, e.g., for subcutaneous injection.

In a third aspect, the present disclosure further relates to a method for treating advanced or metastatic solid malignancies, comprising administering to a patient in need thereof a pharmaceutical combination of an antigen (e.g., PD-L1)-binding molecule and a protein-drug conjugate.

The specific features of the invention to which the present application relates are shown in the appended claims. The characteristics and advantages of the invention to which the present application relates can be better understood with reference to the exemplary embodiments and drawings described in detail below.

### BRIEF DESCRIPTION OF THE DRAWINGS

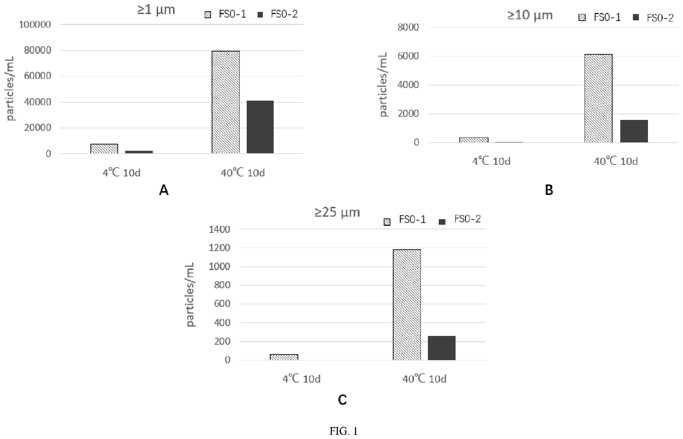

The drawings are briefly described below:
A, B, and C of FIG. 1 show the insoluble microparticle content of JSKN003 alone and a mixed formulation of JSKN003 and KN035.
FIG. 2 shows the aggregate content of JSKN003 alone and a mixed formulation of JSKN003 and KN035.
FIG. 3 shows changes in the drug concentration of JSKN003 in serum samples after intravenous bolus injection or subcutaneous injection, wherein ◆ represents intravenous bolus injection, and ▲ represents subcutaneous injection.
FIG. 4 shows the MS spectrum of LP1.
FIG. 5 shows the reaction scheme for preparing the antibody A-(N₃)₄ and the structure of UDP-GalNAz.
FIG. 6 shows the mass spectrometry data for the antibody A-(N₃)₄.
FIG. 7 shows the reaction scheme for preparing JSKN003.
FIG. 8 shows the mass spectrometry data for JSKN003.
FIG. 9 shows the insoluble microparticle content of JSKN003 alone and a mixture of JSKN003 and KN035 after 4 weeks of standing.
FIGs. 10A-10C show the following test results from a high-temperature stability experiment in the first round of formula screening: SEC-HPLC aggregates (A), nrCE-SDS fragments (B), and antibody moiety relative binding activity (C).
FIGs. 11A-11F show the following test results from a high-temperature stability experiment in the second round of formula screening: insoluble microparticles (A), SEC-HPLC aggregates (B; only the SEC-HPLC test results for FS2-1 were obtained at T0, and these data were used as reference), nrCE-SDS fragments (C), CEX-HPLC main peak (D), and antibody moiety relative binding activity (E), and the test results of viscosity (F) from the second round of formula screening.
FIGs. 12A-12E show the following test results from the third round of formula screening: insoluble microparticles (A), SEC-HPLC aggregates (B; no SEC-HPLC data were obtained at T0, and the test results for the samples before buffer exchange were used as reference), nrCE-SDS fragments (C), CEX-HPLC main peak (D), and antibody moiety relative binding activity (E).
FIG. 13 shows the following test results from an auxiliary material content confirmation experiment: insoluble microparticles, SEC-HPLC, and CEX-HPLC main peak.
FIGs. 14A-14D show the results of a long-term stability experiment for a mixed formulation, including SEC-HPLC (A), CEX-HPLC (B), nrCE-SDS (C), and relative binding activity (D).
FIGs. 15A-15D show the results of an accelerated stability experiment for a mixed formulation, including SEC-HPLC (A), CEX-HPLC (B), nrCE-SDS (C), and relative binding activity (D).
A, B, C, D, E, and F of FIG. 16 show the insoluble microparticle content of DS8201 alone and a mixed formulation of DS8201 and KN035.
A and B of FIG. 17 show the SEC-HPLC aggregate content of a mixed formulation of DS8201.
A, B, and C of FIG. 18 show the insoluble microparticle content of RC48 alone and a mixed formulation of RC48 and KN035.

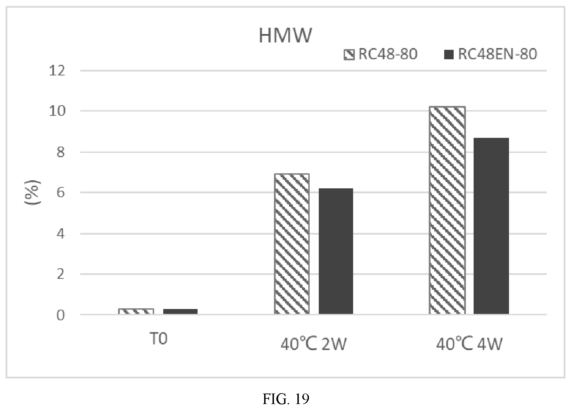

FIG. 19 shows the SEC-HPLC aggregate content of a mixed formulation of RC48.

### DETAILED DESCRIPTION

### Definitions of Terms

Unless otherwise indicated or defined, all terms used herein have the ordinary meanings in the art that would be understood by those skilled in the art. Refer to, for example, standard handbooks, such as Sambrook et al., "Molecular Cloning: A Laboratory Manual" (2nd Edition), Vol. 1-3, Cold Spring Harbor Laboratory Press (1989); Lewin, "Genes IV", Oxford University Press, New York (1990); and Roitt et al., "Immunology" (2nd Edition), Gower Medical Publishing, London, New York (1989), and the general prior art cited herein; in addition, unless otherwise specified, all methods, steps, techniques, and procedures that are not specifically described can be, and have been, performed in a manner that itself is known to those skilled in the art. Also refer to, for example, standard handbooks, the general prior art described above, and the other references cited therein.

The terms "complete antibody", "full-length antibody", and "whole antibody" are used interchangeably herein and generally refer to an immunoglobulin molecule consisting of two identical pairs of polypeptide chains (each pair has one "light" (L) chain and one "heavy" (H) chain). Antibody light chains can be classified as κ and λ light chains. Heavy chains can be classified as µ, δ, γ, α, or ε, and the isotypes of antibodies are defined as IgM, IgD, IgG, IgA, and IgE, respectively. Within a light chain and a heavy chain, the variable regions and the constant regions can be linked by a "J" region of about 12 or more amino acids. The heavy chain can further comprise a "D" region of about 3 or more amino acids. The heavy chain may consist of a heavy chain variable region (VH) and a heavy chain constant region (CH). The heavy chain constant region may consist of 3 domains (CH1, CH2, and CH3). The light chain may consist of a light chain variable region (VL) and a light chain constant region (CL). The light chain constant region may consist of one domain: CL. The constant regions of an antibody can mediate the binding of immunoglobulins to host tissues or factors, including various cells of the immune system (e.g., effector cells) and the first component of the classical complement system (C1q). The VH and VL regions can also be subdivided into regions with high variability (known as complementarity determining regions (CDRs)) interspersed with relatively conserved regions known as framework regions (FRs). For example, the VH and VL may each comprise or consist of 3 CDRs and 4 FRs arranged from the amino-terminus to the carboxy-terminus in the following order: FR1, CDR1, FR2, CDR2, FR3, CDR3, FR4. The variable regions (VH and VL) of each heavy/light chain pair form an antibody binding site.

"Complementarity determining regions" or "CDR regions" or "CDRs" are regions in an antibody variable domain that are highly variable in sequence and form structurally defined loops ("hypervariable loops") and/or comprise antigen contact residues ("antigen contact points"). CDRs are primarily responsible for binding to antigenic epitopes. The CDRs of heavy and light chains are generally referred to as CDR1, CDR2, and CDR3, and are sequentially numbered from the N-terminus. The CDRs located in a heavy chain variable domain of an antibody are referred to as HCDR1, HCDR2, and HCDR3, and the CDRs located in a light chain variable domain of an antibody are referred to as LCDR1, LCDR2, and LCDR3. In the amino acid sequence of a given light or heavy chain variable region, the exact amino acid sequence boundaries of the CDRs can be determined using any one or a combination of many well-known antibody CDR assignment systems, including, e.g., Chothia, which is based on the three-dimensional structures of antibodies and the topology of CDR loops (Chothia et al., (1989) Nature 342: 877-883; Al-Lazikani et al., "Standard conformations for the canonical structures of immunoglobulins", Journal of Molecular Biology, 273: 927-948 (1997)), Kabat, which is based on antibody sequence variability (Kabat et al., Sequences of Proteins of Immunological Interest, 4th Edition, U.S. Department of Health and Human Services, National Institutes of Health (1987)), AbM (University of Bath), Contact (University College London), International ImMunoGeneTics database (IMGT) (imgt.cines.fr/ on the World Wide Web), and the North CDR definition, which is based on an affinity propagation clustering using a large number of crystal structures (North et al., "A New Clustering of Antibody CDR Loop Conformations", Journal of Molecular Biology, 406, 228-256 (2011)).

In the present disclosure, the regional ranges of CDRs defined using the Kabat, AbM, or IMGT scheme are as follows:

| CDR | Kabat scheme | AbM scheme | IMGT scheme | Chothia scheme |
|---|---|---|---|---|
| LCDR1 (Kabat and Chothia numbering systems) | L24-L34 | L24-L34 | L27-L32 | L26-L32 |
| LCDR2 (Kabat and Chothia numbering systems) | L50-L56 | L50-L56 | L50-L52 | L50-L52 |
| LCDR3 (Kabat and Chothia numbering systems) | L89-L97 | L89-L97 | L89-L96 | L91-L96 |
| HCDR1 (Kabat numbering system) | H31-H35B | H26-H35B | H26-H35B | H26-H32 |
| HCDR1 (Chothia numbering system) | H31-H35 | H26-H35 | H26-H35 | H26-H32 |
| HCDR2 (Kabat and Chothia numbering systems) | H50-H65 | H50-H58 | H51-H57 | H53-H55 |
| HCDR3 (Kabat and Chothia numbering systems) | H95-H102 | H95-H102 | H93-H102 | H96-H101 |

Unless otherwise specified, the term "CDR" or "CDR sequence" as used in the present disclosure encompasses CDR sequences determined by any one of the schemes described above.

Herein, when "Kabat CDR" is referred to, it refers to a CDR determined based on the Kabat scheme. Similarly, "Chothia CDR" refers to a CDR determined based on the Chothia scheme, "Abm CDR" refers to a CDR determined based on the Abm scheme, and "IMGT CDR" refers to a CDR determined based on the IMGT scheme.

The term "antibody" is not limited by any particular antibody production method. For example, it includes, in particular, recombinant antibodies, monoclonal antibodies, and polyclonal antibodies. Antibodies may be antibodies of different isotypes, e.g., IgG (e.g., the IgG1, IgG2, IgG3, or IgG4 subtype), IgA1, IgA2, IgD, IgE, or IgM antibodies.

In the present application, the term "bispecific antibody" generally refers to an antibody capable of binding to two antigens or antigenic epitopes. In some embodiments of the present disclosure, the bispecific antibody may comprise a light chain and a heavy chain of an antibody capable of specifically binding to a first antigen or antigenic epitope, and a light chain and a heavy chain of an antibody capable of specifically binding to a second antigen or antigenic epitope. In one embodiment of the present application, in the bispecific antibody, the light chain of the antibody capable of specifically binding to the first antigen or antigenic epitope and the light chain of the antibody capable of specifically binding to the second antigen or antigenic epitope have the same sequence. In one embodiment of the present application, in the bispecific antibody, the heavy chain of the antibody capable of specifically binding to the first antigen or antigenic epitope and the heavy chain of the antibody capable of specifically binding to the second antigen or antigenic epitope have different sequences.

The term "epitope" or "antigenic epitope" generally refers to a site on an antigen to which an immunoglobulin or antibody specifically binds. "Epitope" is also referred to in the art as "antigenic determinant". Epitopes or antigenic determinants usually consist of chemically active surface groups of molecules such as amino acids or carbohydrates or sugar side chains and usually have specific three-dimensional structural characteristics and specific charge characteristics. For example, an epitope usually comprises at least 3, 4, 5, 6, 7, 8, 9, 10, 11, 12, 13, 14, or 15 contiguous or non-contiguous amino acids in a unique spatial conformation, and it may be "linear" or "conformational". See, e.g., Epitope Mapping Protocols in Methods in Molecular Biology, Vol. 66, G. E. Morris, Ed. (1996). In a linear epitope, all points of interaction between a protein and an interacting molecule (e.g., an antibody) are present linearly along the primary amino acid sequence of the protein. In a conformational epitope, the points of interaction are present across amino acid residues on the protein that are separated from one another.

The term "specificity" refers to the number of different types of antigens or epitopes to which a particular antigen-binding molecule or antigen-binding protein can bind. The specificity of an antigen-binding protein can be determined based on its affinity and/or avidity. Affinity, expressed as the dissociation equilibrium constant (KD) of an antigen and an antigen-binding protein, measures the binding strength between an epitope and an antigen-binding site on the antigen-binding protein: the smaller the KD value, the stronger the binding strength between the epitope and the antigen-binding protein (or, the affinity can also be expressed as the association constant (KA), which is 1/KD). As will be appreciated by those skilled in the art, affinity can be determined in a known manner depending on the specific antigen of interest. Avidity measures the binding strength between an antigen-binding protein (e.g., an immunoglobulin, an antibody, an immunoglobulin single variable domain, or a polypeptide comprising same) and a related antigen. Avidity is related to both the affinity between antigen-binding sites on its antigen-binding protein and the number of related binding sites present on the antigen-binding protein.

As used in the present application, the term "domain" (of a polypeptide or protein) refers to a folded protein structure that is capable of maintaining its tertiary structure independently of the rest of the protein. In general, a domain is responsible for a single functional property of a protein and, in many cases, may be added, removed, or transferred to other proteins without compromising the functionality of the rest of the protein and/or the domain.

As used in the present application, the term "immunoglobulin domain" refers to a globular region of an antibody chain (e.g., a chain of a conventional 4-chain antibody or a chain of a heavy-chain antibody) or a polypeptide consisting essentially of such globular regions. An immunoglobulin domain is characterized in that it maintains the immunoglobulin folding characteristics of an antibody molecule.

As used in the present application, the term "immunoglobulin variable domain" refers to an immunoglobulin domain consisting essentially of: four "framework regions" referred to in the art and hereinafter as "framework region 1" or "FR1", "framework region 2" or "FR2", "framework region 3" or "FR3", and "framework region 4" or "FR4", and three "complementarity determining regions" or "CDRs" referred to in the art and hereinafter as "complementarity determining region 1" or "CDR1", "complementarity determining region 2" or "CDR2", and "complementarity determining region 3" or "CDR3" that space the framework regions apart. Thus, the general structure or sequence of an immunoglobulin variable domain can be represented as follows: FR1-CDR1-FR2-CDR2-FR3-CDR3-FR4. An immunoglobulin variable domain imparts specificity for an antigen to an antibody due to the antigen-binding site it has.

As used in the present application, the term "immunoglobulin single variable domain" refers to an immunoglobulin variable domain that is capable of specifically binding to an antigenic epitope without pairing with other immunoglobulin variable domains. An example of the immunoglobulin single variable domain in the context of the present application is the "domain antibody", such as the immunoglobulin single variable domains VH and VL (VH domain and VL domain). Another example of the immunoglobulin single variable domain is a "VHH domain" (or "VHH" for short) of the family Camelidae as defined below.

A "VHH domain", also known as a single-domain antibody, a VHH, a VHH domain, a VHH antibody fragment, and a VHH antibody, is the variable domain of an antigen-binding immunoglobulin known as a "heavy-chain antibody" (Hamers-Casterman C, Atarhouch T, Muyldermans S, Robinson G, Hamers C, Songa EB, Bendahman N, Hamers R.: "Naturally occurring antibodies devoid of light chains"; Nature 363,446-448 (1993)). The term "VHH domain" is used to distinguish the variable domain from the heavy chain variable domain (which is referred to in the present application as a "VH domain") present in a conventional 4-chain antibody. The VHH domain specifically binds to an epitope without requiring additional antigen-binding domains (as opposed to the VH or VL domain in a conventional 4-chain antibody where epitope recognition involves both the VL and VH domains). A VHH domain is a small, stable, and efficient antigen recognition unit formed from a single immunoglobulin domain. A VHH is, for example, derived from an animal of the family Camelidae, such as an alpaca, or is a humanized form thereof or a sequence-optimized form thereof (e.g., an affinity-matured form to increase the binding affinity). In some embodiments, the VHH is a monovalent monospecific polypeptide molecule consisting of, or consisting essentially of, a single heavy chain variable region (e.g., the heavy chain variable region of a heavy-chain antibody).

In the context of the present application, the terms "VHH domain", "VHH", "VHH domain", "VHH antibody fragment", and "VHH antibody" are used interchangeably.

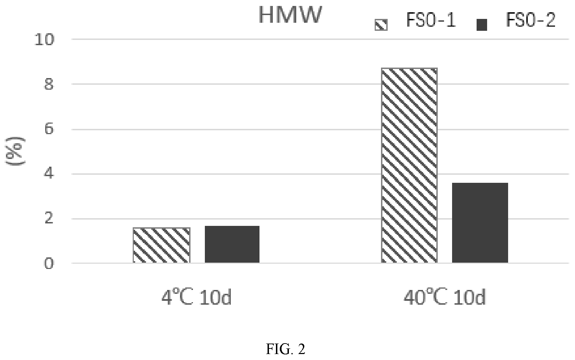

For example, as shown in FIG. 2 in Riechmann and Muyldermans, J. Immunol. Methods 231, 25-38 (1999), the amino acid residues used in VHH domains of the family Camelidae can be numbered according to the general numbering method for VH domains given by Kabat et al. (Kabat et al., Sequences of Proteins of Immunological Interest, 5th Ed. Public Health Service, National Institutes of Health, Bethesda, Md. (1991)).

Alternative methods for numbering the amino acid residues of VH domains are known in the art and can also be similarly applied to VHH domains. For example, Chothia CDRs refer to the positions of structural loops (Chothia and Lesk, J. Mol. Biol. 196: 901-917 (1987)). AbM CDRs represent a compromise between Kabat hypervariable regions and Chothia structure loops, and are used in Oxford Molecular's AbM antibody modeling software. "Contact" CDRs are based on the analysis of available complex crystal structures.

Single-domain antibodies or VHHs may also be contained in larger polypeptides/proteins. Examples of polypeptides/proteins comprising the VHH of the present disclosure include, but are not limited to, a heavy-chain antibody (HcAb).

The "heavy-chain antibody" described in the present disclosure refers to an antibody having no light chain, which may comprise, for example, from the N-segment to the C-segment, VH-Fc or VH-CH2-CH3 or VH-hinge region-CH2-CH3, or may comprise VH-CH1-CH2-CH3. The heavy-chain antibody of the present disclosure may also encompass a homodimer, e.g., a heavy-chain dimer antibody having no light chain. The heavy-chain antibody may comprise a VH from a standard antibody or a VH from a single-domain antibody. For example, the VH in the heavy-chain antibody may be a VHH. For example, the heavy-chain antibody may be a heavy-chain antibody having framework regions and/or a heavy chain constant region derived from animals of the family Camelidae (llamas and camels, particularly alpacas), a humanized form thereof or a sequence-optimized form thereof (affinity-matured form), or a fragment thereof (e.g., a fragment comprising at least a portion of the constant region). The heavy-chain antibody may also encompass an antibody formed by fusing a heavy chain variable region or a VHH to an Fc region (e.g., a human IgG Fc region, e.g., a human IgG1 or IgG4 Fc region).

The term "Fc region" is used herein to define a C-terminal region of an immunoglobulin heavy chain that comprises at least a portion of the constant region. The term includes Fc regions of natural sequences and variant Fc regions. A natural immunoglobulin "Fc domain" comprises two or three constant domains: a CH2 domain, a CH3 domain, and an optional CH4 domain. For example, in natural antibodies, an immunoglobulin Fc domain comprises the second and the third constant domains (the CH2 domain and the CH3 domain) derived from two heavy chains of IgG, IgA, and IgD antibodies, or comprises the second, the third, and the fourth constant domains (the CH2 domain, the CH3 domain, and the CH4 domain) derived from two heavy chains of IgM and IgE antibodies. Unless otherwise specified herein, the amino acid residues in an Fc region or heavy chain constant region are numbered according to the EU numbering system (also known as the EU index) described in, for example, Kabat et al., Sequences of Proteins of Immunological Interes, 5th Edition, Public Health Service, National Institutes of Health, Bethesda, MD, 1991. Herein, the term "Fc region" does not comprise the heavy chain variable region VH and the light chain variable region VL, as well as the heavy chain constant region CH1 and the light chain constant region CL, of an immunoglobulin, but in some cases, it may comprise the hinge region at the N-terminus of the heavy chain constant region. In some embodiments, the Fc region of the present disclosure is from IgG1, IgG2, IgG3, or IgG4. In some embodiments, the Fc region of the present disclosure comprises the amino acid sequence set forth in SEQ ID NO: 2 or 3, or an amino acid sequence having at least 85%, 90%, 91%, 92%, 93%, 94%, 95%, 96%, 97%, 98%, or 99% identity to the amino acid sequence.

The term "protein-drug conjugate" generally refers to a binding protein (e.g., an antibody or an antigen-binding fragment thereof) linked to one or more chemical drugs, such as an antibody-drug conjugate (ADC). The chemical drugs may be any therapeutic agents and/or cytotoxic agents. The antibody-drug conjugate may have anywhere from 1 to 16 drugs conjugated to the antibody; for example, it may include drug-loaded species of 2, 4, 6, or 8. In the present disclosure, the drugs may include mitotic inhibitors, anti-tumor antibiotics, immunomodulators, vectors for gene therapy, alkylating agents, anti-angiogenic agents, anti-metabolites, boron-containing agents, chemoprotective agents, hormones, anti-hormone agents, corticosteroids, photoactive therapeutic agents, oligonucleotides, radionuclide agents, topoisomerase inhibitors, tyrosine kinase inhibitors, and/or radiosensitizers, etc.

In the present application, the term "drug/antibody ratio" or "DAR" generally refers to the number of drugs linked to the antibody (or protein) of the ADC. The DAR of an ADC may range from 1 to 8, or may also be higher (e.g., 10). The range of the DAR can depend on the number of linking sites on the antibody. In the present application, the DAR may be the number of drugs loaded onto an individual antibody. The DAR may also be an average or mean DAR of a group of ADCs.

The term "linker" refers to a structural fragment that links a drug (e.g., a small-molecule drug) and an antibody moiety. It will be appreciated that the linker has a functional group that can form a bond with a functional group of an antibody or an antigen-binding fragment thereof before being linked to the antibody or the antigen-binding fragment thereof.

In the present application, the term "HER2" generally refers to human epidermal growth factor receptor 2 (SwissProt P04626). In the present application, the HER2 may also be referred to as rbB-2, NEU, HER-2, or CD340. The HER2 may include any variants, isoforms, and species homologs of HER2 that are naturally expressed by cells, including tumor cells, or are expressed by cells transfected with the HER2 gene or cDNA.

As used herein, the term "alkyl" refers to a saturated aliphatic hydrocarbon group that is a linear or branched group containing 1 to 20 carbon atoms, preferably an alkyl group containing 1 to 12 carbon atoms, more preferably an alkyl group containing 1 to 10 carbon atoms, and most preferably an alkyl group containing 1 to 6 carbon atoms.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, and more preferably 3 to 10 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl groups include spiro-ring, fused-ring, and bridged-ring cycloalkyl groups.

The term "cycloalkylene" refers to a saturated or partially unsaturated monocyclic or polycyclic hydrocarbon group, which is a residue derived by removing two hydrogen atoms from the same carbon atom or two different carbon atoms of the parental alkane.

The term "heterocycloalkyl" refers to a saturated or partially unsaturated monocyclic or polycyclic hydrocarbon substituent containing 3 to 20 ring atoms, wherein one or more of the ring atoms are heteroatoms selected from nitrogen, oxygen, and S(O)ₘ (where m is an integer from 0 to 2), but a ring moiety of -O-O-, -O-S-, or -S-S- is excluded, and the other ring atoms are carbon atoms.

The term "heterocycloalkylene" refers to a saturated or partially unsaturated monocyclic or polycyclic hydrocarbon group containing 3 to 20 ring atoms, wherein one or more of the ring atoms are heteroatoms selected from nitrogen, oxygen, and S(O)ₘ (where m is an integer from 0 to 2), but a ring moiety of -O-O-, -O-S-, or -S-S- is excluded, and the other ring atoms are carbon atoms. The group is a residue derived by removing two hydrogen atoms from the same carbon atom or two different carbon atoms of the parental alkane on this basis.

The term "alkoxy" refers to -O-(alkyl) and -O-(unsubstituted cycloalkyl), wherein the alkyl or cycloalkyl is defined as described above.

The term "aryl" refers to a 6- to 14-membered, preferably 6- to 10-membered, all-carbon monocyclic or fused polycyclic (i.e., rings that share a pair of adjacent carbon atoms) group having a conjugated π-electron system, such as phenyl and naphthyl, preferably phenyl. The aryl ring may be fused to a heteroaryl, heterocyclyl, or cycloalkyl ring, wherein the ring attached to the parental structure is the aryl ring. Aryl may be substituted or unsubstituted, and when it is substituted, the substituent is preferably one or more groups independently selected from alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "arylene" refers to a 6- to 14-membered all-carbon monocyclic or fused polycyclic ring having a conjugated π-electron system, which is a residue derived by removing two hydrogen atoms from two different carbon atoms of the parental aromatic ring.

The term "heteroaryl" refers to a heteroaromatic system containing 1 to 4 heteroatoms and 5 to 14 ring atoms, wherein the heteroatoms are selected from oxygen, sulfur, and nitrogen. Heteroaryl is preferably 5- to 10-membered, and is more preferably 5-membered or 6-membered, such as furanyl, thienyl, pyridinyl, pyrrolyl, N-alkylpyrrolyl, pyrimidinyl, pyrazinyl, imidazolyl, and tetrazolyl. The heteroaryl ring may be fused to an aryl, heterocyclyl, or cycloalkyl ring, wherein the ring attached to the parental structure is the heteroaryl ring. Heteroaryl may be optionally substituted or unsubstituted, and when it is substituted, the substituent is preferably one or more groups independently selected from alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "heteroarylene" refers to a heteroaromatic polycyclic ring containing 1 to 4 heteroatoms and 5 to 14 ring atoms, which is a residue derived by removing two hydrogen atoms from two different carbon atoms of the parental aromatic ring.

The term "optional" or "optionally" means that the subsequently described event or circumstance occurs or does not occur, and the description includes instances where the event or circumstance occurs and instances where it does not. For example, when a group or structure is "optionally substituted", the group or structure may be substituted or unsubstituted.

The term "pharmaceutically acceptable salt" refers to salts that retain the biological effects and properties of the conjugates of the present disclosure and are not biologically or otherwise undesired. The conjugates of the present disclosure may be present in pharmaceutically acceptable salt forms thereof, including acid addition salts and base addition salts. In the present disclosure, pharmaceutically acceptable non-toxic acid addition salts refer to salts formed from the conjugates of the present disclosure with organic or inorganic acids, wherein the organic or inorganic acids include, but are not limited to, hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propanoic acid, benzoic acid, p-toluenesulfonic acid, malic acid, etc. Pharmaceutically acceptable non-toxic base addition salts refer to salts formed from the conjugates of the present disclosure with organic or inorganic bases, including but not limited to alkali metal salts, such as lithium, sodium, or potassium salts; alkaline earth metal salts, such as calcium or magnesium salts; and organic base salts, such as ammonium salts formed with N group-containing organic bases.

Where there is no contradiction according to the context, "pharmaceutically acceptable" and "pharmaceutical" are used interchangeably herein.

As used herein, the term "and/or" refers to any one of the options or two or more of the options.

As used herein, the term "comprise", "contain", or "include" refers to including the stated elements, integers, or steps, but not excluding any other elements, integers, or steps. As used herein, the term "comprise", "contain", or "include" also encompasses combinations of the stated elements, integers, or steps, unless otherwise indicated.

The term "administer" generally refers to a method of giving a subject (e.g., a patient) a certain dose of a compound or pharmaceutical composition. Administration may be performed by any suitable means, including parenteral, intrapulmonary, and intranasal, as well as (if topical treatment is desired) intralesional administration. Parenteral infusion includes, for example, intramuscular, intravenous, intraarterial, intraperitoneal, or subcutaneous administration.

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value. Unless otherwise specified, all numerical values mentioned in the present application are seen to be modified by "about". If there is a question or there is no common understanding for the margin of error of a particular value or parameter acknowledged in the art, "about" means +5% of that value or parameter.

The term "effective amount" refers to an amount or dose of the antibody or fragment or composition or combination of the present disclosure that produces the expected effects in a patient in need of treatment or prevention after being administered to the patient in a single dose or multiple doses. "Therapeutically effective amount" and "prophylactically effective amount" may be included according to the expected effects.

"Therapeutically effective amount" refers to an amount effective, at dosages and for periods of time necessary, to achieve the desired therapeutic result. A therapeutically effective amount is also one in which any toxic or detrimental effect of the antibody or antibody fragment or composition or combination is outweighed by the therapeutically beneficial effects. "Prophylactically effective amount" refers to an amount effective, at dosages and for periods of time necessary, to achieve the desired prophylactic result. In general, as a prophylactic dose is used in a subject prior to or at an earlier stage of a disease, the prophylactically effective amount will be less than the therapeutically effective amount.

"Individual" or "subject" includes mammals. Mammals include, but are not limited to, domestic animals (e.g., cows, sheep, cats, dogs, and horses), primates (e.g., human and non-human primates such as monkeys), rabbits, and rodents (e.g., mice and rats). In some embodiments, the individual or subject is a human.

The terms "cancer" and "cancerous" refer to or describe physiological conditions in mammals that are typically characterized by unregulated cell growth. Cancer may be at an early, intermediate, or advanced stage or be metastatic cancer.

The term "tumor" refers to all neoplastic cell growth and proliferation, whether malignant or benign, and all pre-cancerous and cancerous cells and tissues. "Tumor" encompasses solid tumors and hematological tumors, as well as metastatic lesions. The terms "cancer", "cancerous", and "tumor" are not mutually exclusive when referred to herein.

The term "pharmaceutical auxiliary material" refers to diluents, adjuvants (e.g., Freund's adjuvants (complete and incomplete)), excipients, carriers, stabilizers, or the like, which are administered together with an active substance.

The term "pharmaceutical composition" refers to a composition that is present in a form that allows the biological activity of an active ingredient contained therein to be effective and does not contain additional ingredients that have unacceptable toxicity to a subject to which the composition is administered.

As used herein, "treat" refers to slowing, interrupting, arresting, alleviating, stopping, reducing, or reversing the progression or severity of an existing symptom, condition, disorder, or disease.

### Detailed Description of the Invention

### Improving Stability of Protein-Drug Conjugate Formulation

In a first aspect, the present application provides use of an antigen-binding molecule in improving the stability of a protein-drug conjugate formulation. The stability includes, but is not limited to, conformational stability, colloidal stability, high-temperature stability, freeze-thaw stability, shaking stability, light-exposure stability, long-term storage stability, etc.

In some embodiments, the formulation is a solid formulation, preferably a freeze-dried formulation.

In some embodiments, the formulation is a liquid formulation, preferably a solution, more preferably an injectable agent, such as a subcutaneous injectable solution or an intravenous injectable solution.

In some embodiments, the antigen-binding molecule is capable of reducing the amount of insoluble microparticles generated in the formulation.

In some embodiments, the antigen-binding molecule is capable of reducing the amount of aggregates generated in the formulation.

In some embodiments, the antigen-binding molecule is a programmed death ligand 1 (PD-L1)-binding molecule.

In some embodiments, the PD-L1-binding molecule of the present disclosure is from Patent WO2017020801, which is incorporated herein in its entirety.

In some embodiments, the PD-L1-binding molecule of the present disclosure comprises or consists of a CDR1, a CDR2, and a CDR3 from a VHH disclosed in WO2017020801, or comprises or consists of a VHH disclosed therein, or comprises or consists of a heavy-chain antibody disclosed therein.

In some embodiments, the PD-L1-binding molecule comprises or consists of an immunoglobulin single variable domain, wherein the immunoglobulin single variable domain comprises a CDR1, a CDR2, and a CDR3 from the VHH set forth in SEQ ID NO: 1. The CDRs may be Kabat CDRs, AbM CDRs, Chothia CDRs, or IMGT CDRs.

In some embodiments, the CDR1, the CDR2, and the CDR3 from the VHH set forth in SEQ ID NO: 1 are selected from any one of the following groups: SEQ ID NOs: 5-7 (Kabat), SEQ ID NOs: 8-10 (AbM), SEQ ID NOs: 11-13 (Chothia), and SEQ ID NOs: 14-16 (IMGT).

In some embodiments, the immunoglobulin single variable domain comprises or consists of the amino acid sequence set forth in SEQ ID NO: 1 or an amino acid sequence having at least 85%, 90%, 91%, 92%, 93%, 94%, 95%, 96%, 97%, 98%, or 99% identity thereto. Preferably, the amino acid sequence of the immunoglobulin single variable domain is set forth in SEQ ID NO: 1.

In some embodiments, the PD-L1-binding molecule is a PD-L1 heavy-chain antibody comprising a VHH, and a constant region of an antibody or a portion thereof, such as an Fc region; for example, the molecule is a heavy-chain antibody comprising a VHH-constant region or VHH-CH1-Fc or VHH-Fc.

Preferably, the immunoglobulin Fc region is a human immunoglobulin Fc region, e.g., an Fc region of human IgG1, human IgG2, human IgG3, or human IgG4, or a variant thereof.

In some specific embodiments, the amino acid sequence of the immunoglobulin Fc region is set forth in SEQ ID NO: 2 or SEQ ID NO: 3.

In some embodiments, the VHH may be located at the N-terminus of the immunoglobulin Fc region or the C-terminus of the immunoglobulin Fc region; the two are linked directly or by a peptide linker.

In some embodiments, the aforementioned peptide chain is selected from one or more of the following amino acid sequences: (GS)ₙ, (GG)ₙ, (GGS)ₙ, (GGGS)ₙ, (GGSG)ₙ, (GGGGS)ₙ, and (GAP)ₙ, wherein n is an integer from 1 to 10.

In some embodiments, the PD-L1-binding molecule comprises the amino acid sequence set forth in SEQ ID NO: 4; preferably, the amino acid sequence of the PD-L1-binding molecule is set forth in SEQ ID NO: 4.

In one embodiment, the PD-L1 heavy-chain antibody according to the present disclosure may be dimerized with another polypeptide chain comprising an Fc region (e.g., another identical or different heavy-chain antibody) through the Fc region. Thus, in one embodiment, the present disclosure further provides a homo- or heteromultimer protein comprising the PD-L1 heavy-chain antibody of the present disclosure. In one preferred embodiment, preferably, the protein comprises a PD-L1 heavy-chain antibody formed by pairing two identical PD-L1 heavy-chain antibody chains.

In some embodiments, the protein-drug conjugate has the structure of formula I:

P-(L-D)ₙ (I),

wherein P is a protein, L is a linking unit, D is a substance having biological activity, and n is an integer from 1 to 20.

In some embodiments, the protein P has desired physiological properties; preferably, the protein P is capable of preventing and/or treating a disease or condition. Specifically, the protein P may be an antibody, a fusion protein, a cytokine, a polypeptide, or the like.

In some embodiments, the protein P is an antibody; the antibody targets one or more of the following antigens: 4-1BB, 4-1BBL, A33, adenosine A2a receptor, Akt, ALK, androgen receptor, Ang-1, Ang-2, Annexin A3, Aurora A, Aurora B, B7-H3, B7-H4, Bcl-2, Bcr-Abl, BRAF, BTK, BTLA, BTN2A1, CA-125, CAIX, CCR4, CD105/endoglin, CD109, CD123, CD155, CD16, CD160, CD19, CD20, CD200, CD200R, CD22, CD24, CD25, CD27, CD28, CD30, CD33, CD36, CD37, CD38, CD40, CD40L, CD47, CD48, CD52, CD70, CD79b, CD80, CD86, CD96, CDK4, CDK6, CDK9, CEA, CEACAM1, ChK1, ChK2, c-KIT, c-Met, COX2, CSF-1R, CSF2, CTLA-4, CXCR2, CXCR4, DDR2, DLL3, DLL4, DNAM-1, DR5, EGFR, EpCAM, EPHA3, EphB4, ERK1, ERK2/p38 MAPK, FAK, FAP, FGF-2, FGFR1, FGFR2, FGFR3, FGFR4, Flt-3, Gal-9, GITR, GITRL, Glypican-3, HDAC1, HDAC2, HDAC3, HDAC4, HDAC5, HDAC7, HDAC8, HDAC9, HER2, HER3, HER4/ERBB4, HGF, HHLA2, HIF-1α, HSP27, HSP90, HVEM, ICOS, ICOS Ligand, IDO, IGF1R, IL-13, IL-6, JAK1, JAK2, JAK3, KRAS, LAG-3, LIGHT, MDM2, MEK1, MEK2, MMP-1, MMP-10, MMP-11, MMP-13, MMP-2, MMP-7, MMP-9, mTOR, Mucin 1, Myc, NF-κB, NKG2A, NRAS, NTRK1, NTRK2, NTRK3, OX40, OX40L, p53, PAF, PARP1, PARP2, PD1, PDGFR-α, PDGFR-β, PD-L1, PD-L2, PI3Kα, PI3Kβ, PI3Kγ, PI3Kδ, PIM1, PIM3, PSMA, PTEN, RAF-1, RANKL, RET, S100A4, SIRPα, SLAMF7, SMO, Src, STAT3, STEAP-1, SYK, TDO, TGFβ, Tie-2, TIGIT, TIM-3, TLR8, TMIGD2, TNF-α, Toll-like receptor 3, TRAIL, TRAILR1, TROP-2, VEGF, VEGF-C, VEGFR-1, VEGFR-2, VEGFR-3, and VISTA.

In some embodiments, the antibody may target HER2; preferably, the antibody may bind to different epitopes of human HER2, e.g., extracellular domain II of human HER2 and/or extracellular domain IV of human HER2.

In some embodiments, the antibody is a bispecific antibody that targets different epitopes of HER2. In some embodiments, the antibody may specifically bind to extracellular domains II and IV of human HER2.

In some embodiments, the bispecific antibody is derived from Patent Application WO2016110267, which is incorporated herein in its entirety.

In some embodiments, the bispecific antibody comprises a bispecific structure shown in WO2016110267.

In some embodiments, the bispecific antibody comprises 1-6 CDRs from a first heavy chain, 1-6 CDRs from a second heavy chain, and 1-6 CDRs from common light chains of the bispecific antibody described in WO2016110267.

In some embodiments, the bispecific antibody comprises a heavy chain variable region and/or a light chain variable region from the first heavy chain, a heavy chain variable region and/or a light chain variable region from the second heavy chain, and a heavy chain variable region and/or a light chain variable region from the common light chain of the bispecific antibody described in WO2016110267.

In some embodiments, the bispecific antibody comprises the first heavy chain, the second heavy chain, and the common light chain of the bispecific antibody described in WO2016110267.

In some embodiments, the antibody has a structure similar to that of a full-length antibody, but has two different heavy chains; that is, the antibody has a first heavy chain, a second heavy chain, and two common light chains. In some embodiments, the first heavy chain comprises a first heavy chain variable region, the second heavy chain comprises a second heavy chain variable region, and/or the common light chains comprise common light chain variable regions.

In some embodiments, the antibody has a first heavy chain, a second heavy chain, and common light chains, wherein the first heavy chain comprises a CDR1, a CDR2, and a CDR3 from the heavy chain variable region set forth in SEQ ID NO: 17, the second heavy chain comprises a CDR1, a CDR2, and a CDR3 from the heavy chain variable region set forth in SEQ ID NO: 18, and the common light chains comprise a CDR1, a CDR2, and a CDR3 from the light chain variable region set forth in SEQ ID NO: 19. The CDRs may be Kabat CDRs, AbM CDRs, Chothia CDRs, or IMGT CDRs.

In some embodiments, the CDR1, the CDR2, and the CDR3 from the heavy chain variable region set forth in SEQ ID NO: 17 are selected from any one of the following groups: SEQ ID NOs: 23-25 (Kabat), SEQ ID NOs: 26-28 (AbM), SEQ ID NOs: 29-31 (Chothia), and SEQ ID NOs: 32-34 (IMGT).

In some embodiments, the CDR1, the CDR2, and the CDR3 from the heavy chain variable region set forth in SEQ ID NO: 18 are selected from any one of the following groups: SEQ ID NOs: 35-37 (Kabat), SEQ ID NOs: 38-40 (AbM), SEQ ID NOs: 41-43 (Chothia), and SEQ ID NOs: 44-46 (IMGT).

In some embodiments, the CDR1, the CDR2, and the CDR3 from the light chain variable region set forth in SEQ ID NO: 19 are selected from any one of the following groups: SEQ ID NOs: 47-49 (Kabat), SEQ ID NOs: 50-52 (AbM), SEQ ID NOs: 53-55 (Chothia), and SEQ ID NOs: 56-58 (IMGT).

In some embodiments, the first heavy chain variable region comprises or consists of the amino acid sequence set forth in SEQ ID NO: 17 or an amino acid sequence having at least 85%, 90%, 91%, 92%, 93%, 94%, 95%, 96%, 97%, 98%, or 99% identity thereto.

In some embodiments, the second heavy chain variable region comprises or consists of the amino acid sequence set forth in SEQ ID NO: 18 or an amino acid sequence having at least 85%, 90%, 91%, 92%, 93%, 94%, 95%, 96%, 97%, 98%, or 99% identity thereto.

In some embodiments, the common light chain variable region comprises or consists of the amino acid sequence set forth in SEQ ID NO: 19 or an amino acid sequence having at least 85%, 90%, 91%, 92%, 93%, 94%, 95%, 96%, 97%, 98%, or 99% identity thereto.

In some embodiments, the antibody comprises a first heavy chain, a second heavy chain, and two common light chains, wherein the first heavy chain comprises a first heavy chain variable region, the second heavy chain comprises a second heavy chain variable region, and each of the common light chains comprises a light chain variable region, wherein:
the first heavy chain variable region comprises or consists of the amino acid sequence set forth in SEQ ID NO: 17;
the first heavy chain variable region comprises or consists of the amino acid sequence set forth in SEQ ID NO: 18; and
the light chain variable region comprises or consists of the amino acid sequence set forth in SEQ ID NO: 19.

In some embodiments, the amino acid sequence of the first heavy chain of the antibody comprises or consists of the amino acid sequence set forth in SEQ ID NO: 20 or an amino acid sequence having at least 85%, 90%, 91%, 92%, 93%, 94%, 95%, 96%, 97%, 98%, or 99% identity thereto.

In some embodiments, the amino acid sequence of the second heavy chain of the antibody comprises or consists of the amino acid sequence set forth in SEQ ID NO: 21 or an amino acid sequence having at least 85%, 90%, 91%, 92%, 93%, 94%, 95%, 96%, 97%, 98%, or 99% identity thereto.

In some embodiments, the amino acid sequences of the common light chains of the antibody comprise or consist of the amino acid sequence set forth in SEQ ID NO: 22 or an amino acid sequence having at least 85%, 90%, 91%, 92%, 93%, 94%, 95%, 96%, 97%, 98%, or 99% identity thereto.

In some embodiments, the antibody comprises a first heavy chain, a second heavy chain, and two common light chains, wherein:
the first heavy chain variable region comprises or consists of the amino acid sequence set forth in SEQ ID NO: 20;
the first heavy chain variable region comprises or consists of the amino acid sequence set forth in SEQ ID NO: 21; and
the light chain variable region comprises or consists of the amino acid sequence set forth in SEQ ID NO: 22.

In some embodiments, the linking unit L in formula I has the structure represented by formula II:

-L1-sp1-L2-sp2- (II)

wherein L1 is a linker for linking to P, sp1 is a first spacing unit, L2 is a cleavable linker, and sp2 is a second spacing unit and is linked to D.

In some embodiments, the linking unit L in formula I has the structure represented by formula II:

-L1-sp1-L2-sp2- (II)

wherein L1 is a linker for linking to P, sp1 is a first spacing unit, L2 is absent, and sp2 is a second spacing unit and is linked to D.

In some embodiments, L1 in formula II is selected from: 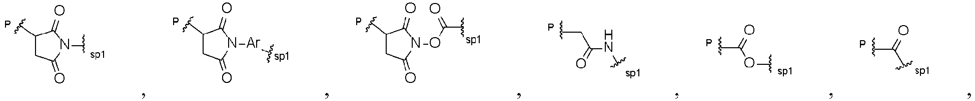 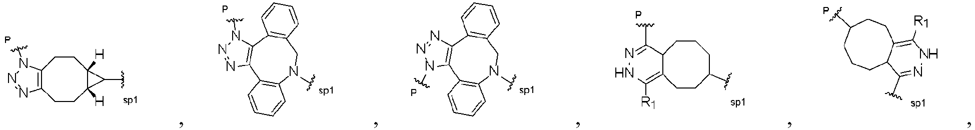 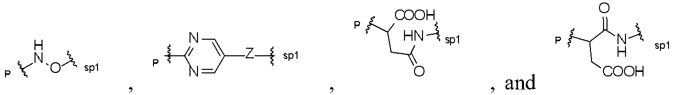 (the side linked to the protein is denoted by P, and the side linked to the first spacing unit is denoted by sp1),
wherein Ar represents C₆₋₁₀ arylene optionally substituted with halogen or C₁₋₆ alkyl; R₁ is selected from hydrogen, halogen, and C₁₋₆ alkyl; Z is selected from a direct bond, C₂₋₆ alkynylene, C₂₋₆ alkenylene, C₆₋₁₀ arylene, 5-10 membered heteroarylene, amido, sulfonamido, imino, and CF₂.

In some embodiments, L1 is

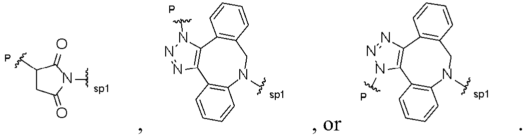

In some embodiments, the first spacing unit sp1 in formula II has the structure of: 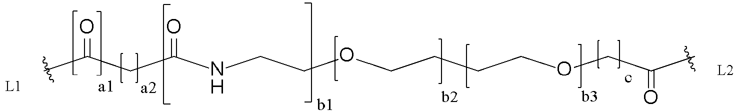 (the side linked to L1 is denoted by L1, and the side linked to L2 is denoted by L2), wherein a1 = 0 or 1, a2 = an integer from 0 to 6, b1 = 0 or 1, b2 = an integer from 0 to 16, b3 = an integer from 0 to 16, and c = an integer from 0 to 6, and at least one of b2 and b3 is 0.

In some embodiments, in the structure of sp1, a1 = 1; in some other embodiments, a1 = 0.

In some embodiments, a2 = 0, 1, 2, 3, 4, 5, or 6.

In some embodiments, b1 = 0 or 1.

In some embodiments, b2 = 0, 1, 2, 3, 4, 5, 6, 7, 8, 9, 10, 11, 12, 13, 14, 15, or 16.

In some embodiments, b3 = 0, 1, 2, 3, 4, 5, 6, 7, 8, 9, 10, 11, 12, 13, 14, 15, or 16.

In some embodiments, c = 0, 1, 2, 3, 4, 5, or 6.

The above optional embodiments of a1, a2, b1, b2, b3, and c can be combined arbitrarily, provided that at least one of b2 and b3 is 0.

In some specific embodiments, the structure of sp1 is selected from the following groups:
(1) a1 = 0, a2 = 2, 3, 4, 5, or 6, b1 = 0, b2 = 0, b3 = 0, and c = 0;
(2) a1 = 0, a2 = 0, b1 = 0, b2 = 0, b3 = 0, and c = 2, 3, 4, 5, or 6;
(3) a1 = 1, a2 = 2, 3, 4, 5, or 6, b1 = 1, b2 = 2, 3, 4, 5, 6, 7, or 8, b3 = 0, and c = 0;
(4) a1 = 0, a2 = 2, 3, 4, 5, or 6, b1 = 1, b2 = 2, 3, 4, 5, 6, 7, or 8, b3 = 0, and c = 0; and
(5) a1 = 1, a2 = 0, b1 = 0, b2 = 0, b3 = 2, 3, 4, 5, 6, 7, or 8, and c = 2, 3, 4, 5, or 6.

In some embodiments, the cleavable linker L2 in formula II is a dipeptide, tripeptide, or tetrapeptide residue.

In some embodiments, L2 is selected from the following dipeptide residues: -Phe-Lys-, -Val-Ala-, -Val-Lys-, -Val-Cit-, -Ala-Lys-, -Phe-Cit-, -Leu-Cit-, -Ile-Cit-, -Phe-Arg-, -Trp-Cit-, -Gly-Gly-, -Ala-Ala-, -Gly-Val-, and -Gly-Glu-; the left side of the dipeptide residue is linked to sp1, and the right side thereof is linked to sp2. Preferably, L2 is selected from -Val-Ala-, -Val-Lys-, and -Val-Cit-.

In some embodiments, L2 is selected from the following tripeptide residues: -Glu-Val-Ala-, -Glu-Val-Cit-, -αGlu-Val-Ala-, -αGlu-Val-Cit-, -Val-Lys-Gly, and -Val-Cit-Gly-; the left side of the tripeptide residue is linked to sp1, and the right side thereof is linked to sp2.

In some embodiments, L2 is selected from the following tetrapeptide residues: -Gly-Gly-Phe-Gly- and -Gly-Phe-Gly-Gly-; the left side of the tetrapeptide residue is linked to sp1, and the right side thereof is linked to sp2.

In some embodiments, sp2 in formula II is absent, or sp2 is selected from: 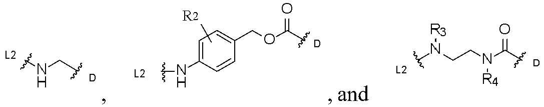 (the side linked to L2 is denoted by L2, and the side linked to the substance having biological activity D is denoted by D), wherein:
R₂ is independently selected from hydrogen, C₁₋₆ alkyl, hydroxy, amino, halogen, nitro, cyano, 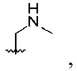 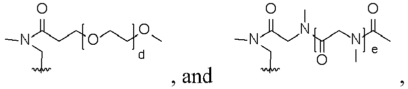 d is an integer from 1 to 20, and e is an integer from 1 to 20; R₃ and R₄ are each independently selected from hydrogen and C₁₋₆ alkyl;
the alkyl may be optionally substituted with hydroxy, amino, halogen, nitro, and cyano.

In some embodiments, sp2 is

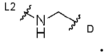

In some embodiments, sp2 is

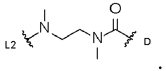

In some embodiments, sp2 is selected from 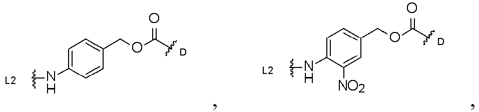 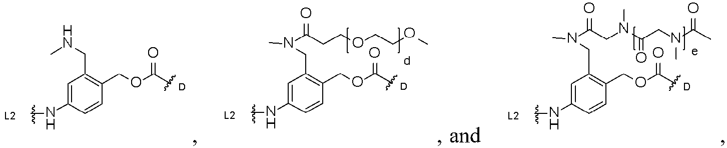 wherein d is an integer from 1 to 10, and e is an integer from 1 to 10.

In some embodiments, the linking unit L may be selected from the following structures: 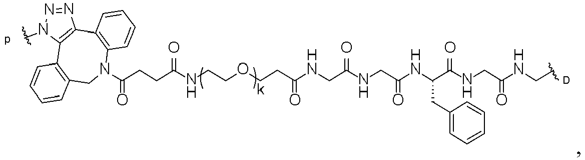 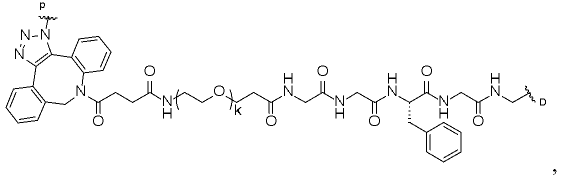 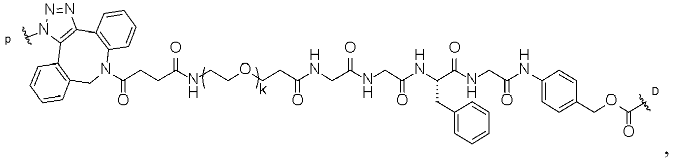 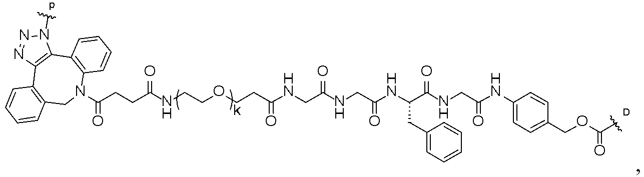 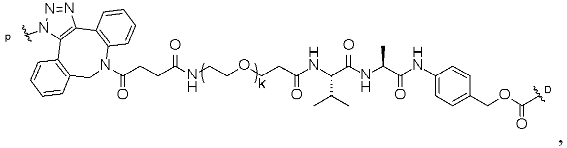 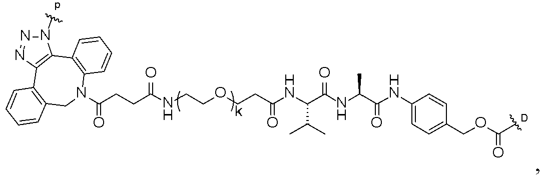 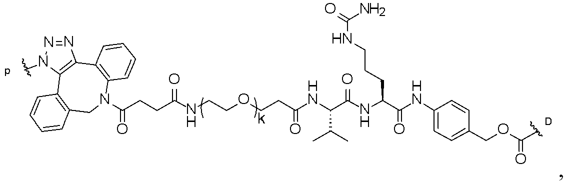 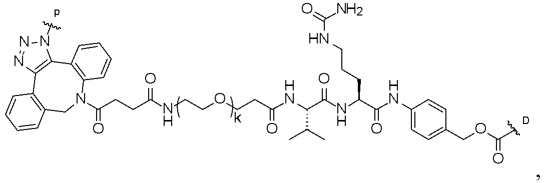 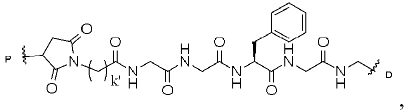 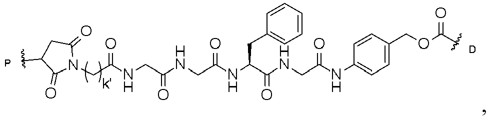 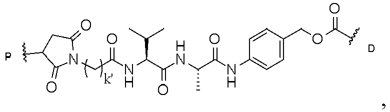 and 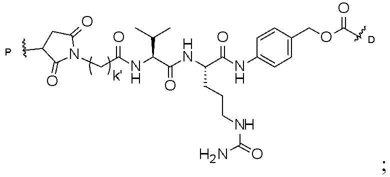 k and k' are each independently an integer from 1 to 20, e.g., 2, 3, 4, 5, 6, 7, 8, 9, or 10 (the side linked to the protein P is denoted by P, and the side linked to the substance having biological activity D is denoted by D).

In some embodiments, k = 2, 4, or 8. In some embodiments, k' = 1, 2, 3, 4, 5, or 6.

In some embodiments, the linking unit L may be selected from the following structures: 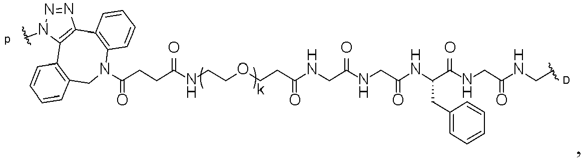 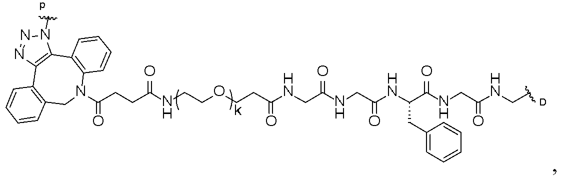 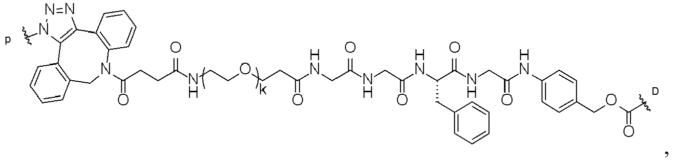 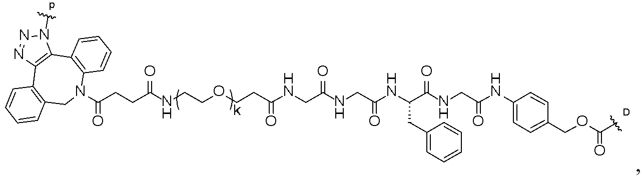 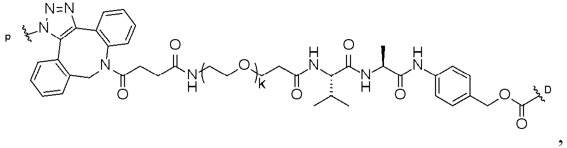 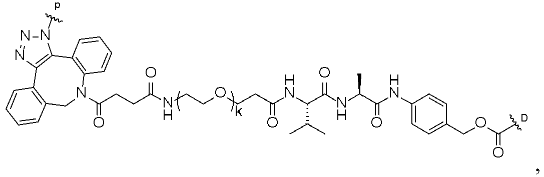 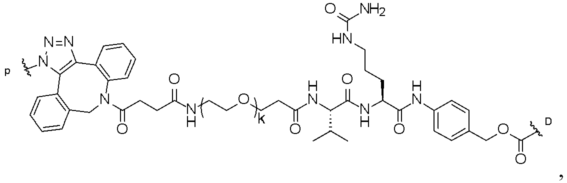 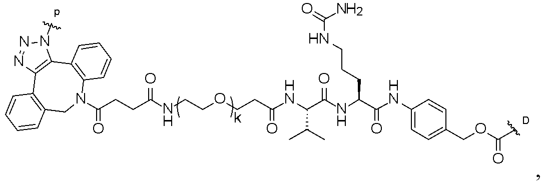 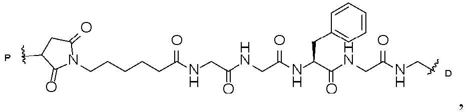 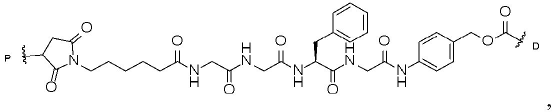 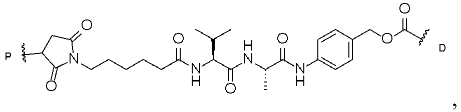 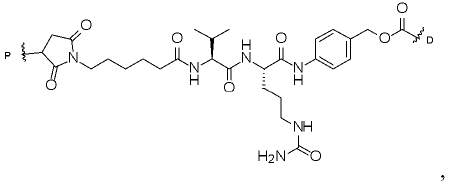 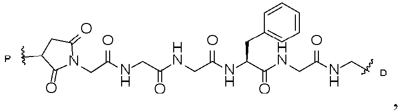 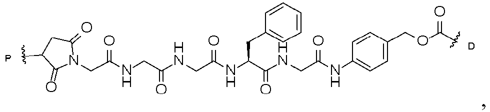 and 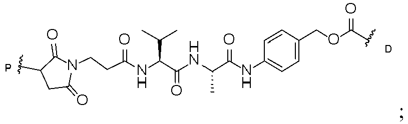 k is an integer from 1 to 20, e.g., 2, 3, 4, 5, 6, 7, 8, 9, or 10 (the side linked to the protein P is denoted by P, and the side linked to the substance having biological activity D is denoted by D). In some embodiments, k = 2, 4, or 8.

The substance having biological activity D in formula I may be selected from a cytotoxin, a protein kinase inhibitor, an immune agonist, a glucocorticoid, an oligonucleotide, a radioisotope, a polypeptide, and any combination thereof.

In some embodiments, D is a cytotoxin selected from a DNA alkylating agent, a DNA deconstructing agent, a topoisomerase I inhibitor, a topoisomerase II inhibitor, a microtubule inhibitor, a ribosome inhibitor, and any combination thereof.

In some specific embodiments, D is selected from an auristatin derivative, a maitansine derivative, an eribulin derivative, a tubulysin derivative, a pyrrolobenzodiazepine (PDB) derivative, a duocarmycin derivative, a calicheamicin derivative, PNU-159682 and a derivative thereof, a camptothecin derivative, an amatoxin derivative, and any combination thereof.

In some embodiments, D is selected from a camptothecin derivative, which refers to a compound having the same 5-membered fused parental core structure as camptothecin of natural origin and having substitution modifications at positions 7, 9, 10, and 11 thereof, which has equivalent or greater inhibitory activity against topoisomerase I than natural camptothecin.

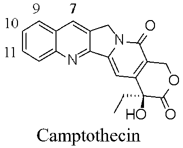

In some embodiments, D has the structure represented by formula III: 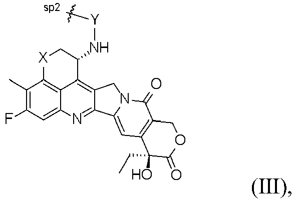
wherein X is selected from CH₂, NH, O, S, and SO₂; Y is absent, or Y has the structure represented by 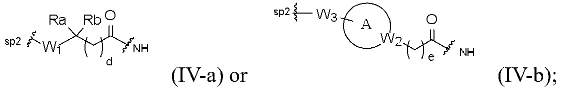
W₁ and W₃ are each independently selected from O, S, and NH, and W₂ is selected from C, CH, and N;
Rₐ and R_{b} are each independently selected from hydrogen, deuterium, halogen, C₁₋₆ alkyl, C₁₋₆ alkoxy, C₃₋₆ cycloalkyl, 3-6 membered heterocycloalkyl, hydroxy, amino, cyano, and nitro; or, Rₐ and R_{b}, together with the carbon atom to which they are attached, form 3-6 membered cycloalkyl, 3-6 membered heterocycloalkyl, or carbonyl; or, Rₐ is linked to the N atom of the amide moiety to form 3-6 membered heterocycloalkyl, and R_{b} is hydrogen;
ring A is selected from the following groups: 5-10 membered cycloalkylene, 5-10 membered heterocycloalkylene, 6-10 membered arylene, and 5-10 membered heteroarylene;
the alkyl, alkoxy, cycloalkyl, heterocycloalkyl, cycloalkylene, heterocycloalkylene, arylene, and heteroarylene are each independently, optionally, and further substituted with a group selected from deuterium, halogen, hydroxy, amino, cyano, carbonyl, and nitro;
d and e are each independently selected from integers from 0 to 5.

In some embodiments, D has the structure represented by formula III-a: 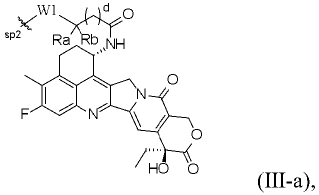 wherein W₁ is O or NH;
Rₐ and R_{b} are each independently selected from hydrogen, deuterium, halogen, C₁₋₆ alkyl, C₁₋₆ alkoxy, C₁₋₆ cycloalkyl, 3-6 membered heterocycloalkyl, hydroxy, amino, cyano, and nitro; or, Rₐ and R_{b}, together with the carbon atom to which they are attached, form 3-6 membered cycloalkyl, 3-6 membered heterocycloalkyl, or carbonyl; or, Rₐ is linked to the N atom of the amide moiety to form 3-6 membered heterocycloalkyl, and R_{b} is hydrogen;
the alkyl, alkoxy, cycloalkyl, and heterocycloalkyl are each independently, optionally, and further substituted with a group selected from deuterium, halogen, hydroxy, amino, cyano, and nitro;
d is an integer from 0 to 5.

In some embodiments, W₁ is O.

In some embodiments, d is 0, 1, or 2.

In some embodiments, Rₐ is selected from hydrogen, deuterium, halogen, C₁₋₆ alkyl, C₁₋₆ alkoxy, C₁₋₆ cycloalkyl, 3-6 membered heterocycloalkyl, hydroxy, and amino, and R_{b} is hydrogen. The alkyl, alkoxy, cycloalkyl, and heterocycloalkyl are each independently, optionally, and further substituted with a group selected from deuterium, halogen, hydroxy, amino, cyano, and nitro.

In some embodiments, Rₐ and R_{b}, together with the carbon atom to which they are attached, form 3-6 membered cycloalkyl or 3-6 membered heterocycloalkyl. The cycloalkyl and heterocycloalkyl are each independently, optionally, and further substituted with a group selected from deuterium, halogen, hydroxy, amino, cyano, and nitro. In some embodiments, D may be selected from the following groups:

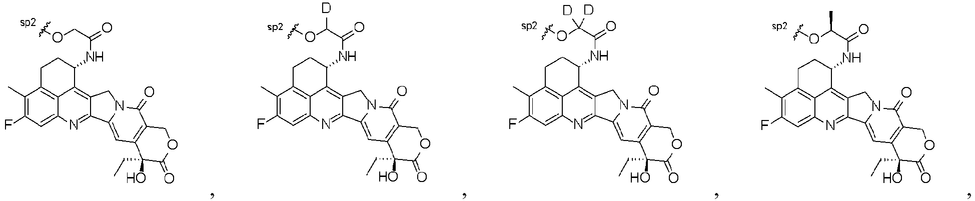

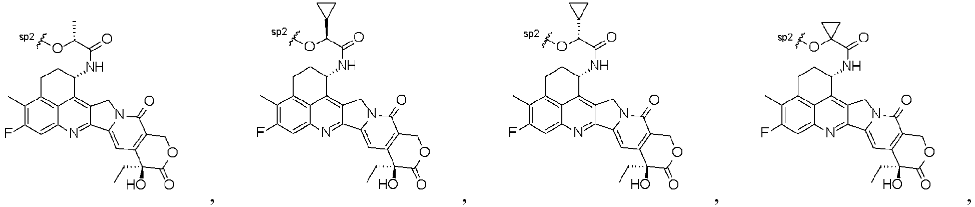

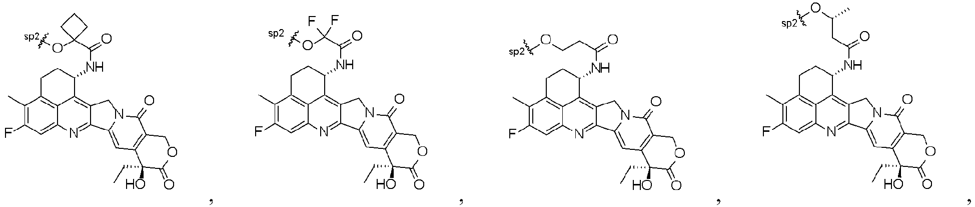

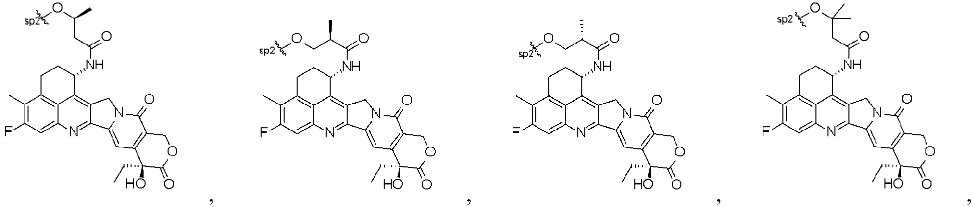

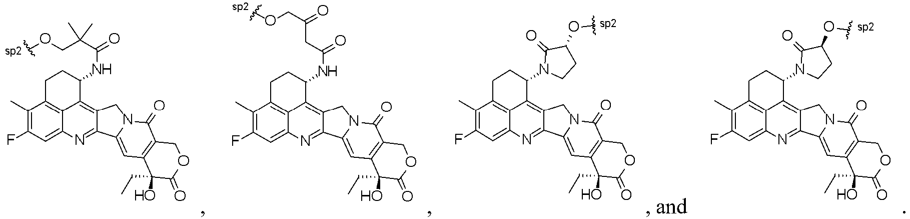

In some other embodiments, D has the structure represented by formula III-b: 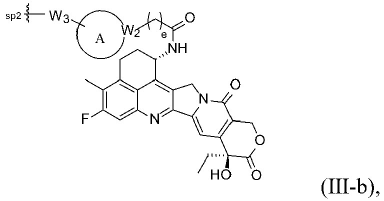
wherein W₃ is O or NH, and W₂ is C, CH, or N;
ring A is selected from the following groups: 5-10 membered cycloalkylene, 5-10 membered heterocycloalkylene, 6-10 membered arylene, and 5-10 membered heteroarylene;
the cycloalkylene, heterocycloalkylene, arylene, and heteroarylene are each independently, optionally, and further substituted with a group selected from deuterium, halogen, hydroxy, amino, cyano, carbonyl, and nitro;
e is an integer from 0 to 5.

In some embodiments, W₃ is O; in some other embodiments, W₂ is CH.

In some embodiments, e is 0, 1, or 2.

In some embodiments, ring A is 5-10 membered cycloalkylene. The cycloalkylene may be further substituted with a group selected from deuterium, halogen, hydroxy, amino, cyano, carbonyl, and nitro.

In some embodiments, D may be selected from the following groups:

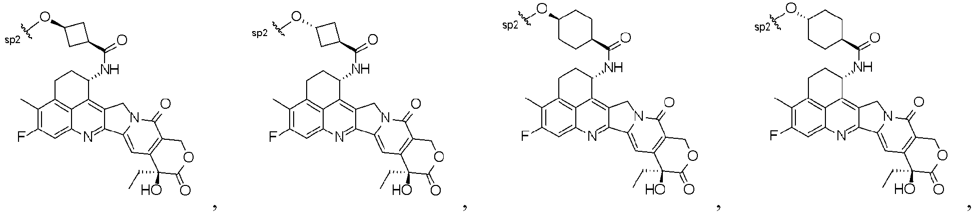

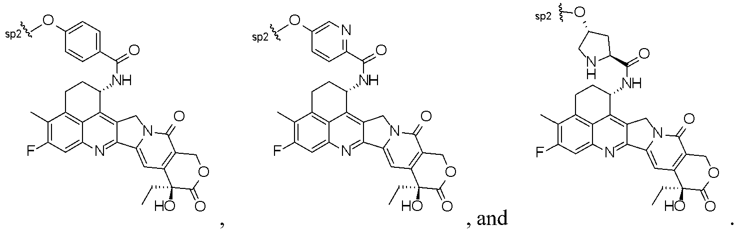

In some embodiments, D, as a camptothecin derivative, may also be selected from the following structures: 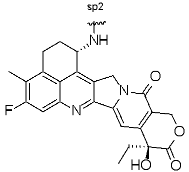 (i.e., exatecan), 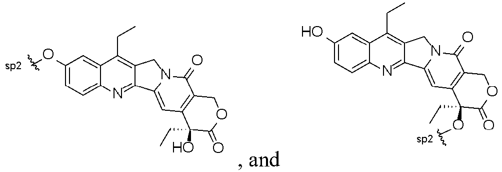 (i.e., SN-38). In some embodiments, D may be an auristatin derivative and is preferably selected from the following structures: 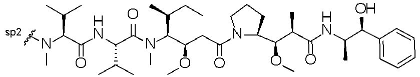 (MMAE), 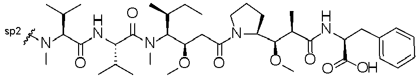 (MMAF), and 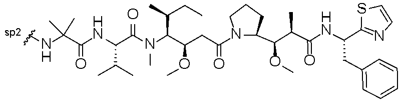 (PF-06380101).

In some embodiments, D may be a maitansine derivative and is preferably selected from the following structures: 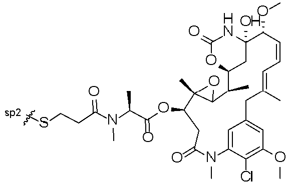 (DM1) and 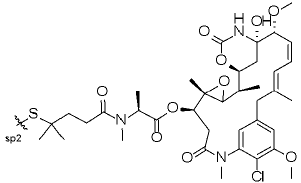 (DM4).

In some embodiments, D is eribulin and a derivative thereof and is preferably selected from the following structures:

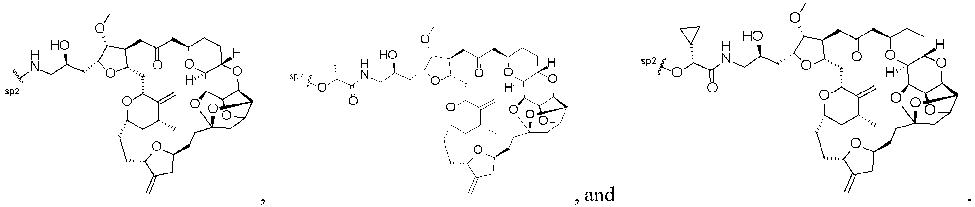

In some embodiments, in formula I, the linking unit L is linked to an antibody by sulfhydryl; preferably, the sulfhydryl is derived from the antibody; more preferably, the sulfhydryl is obtained by reducing a disulfide bond between heavy chains and/or a disulfide bond between a heavy chain and a light chain.

In some other embodiments, the linking unit L is linked to an antibody by an oligosaccharide; preferably, the oligosaccharide is derived from a natural glycan chain of the antibody.

In some embodiments, the oligosaccharide is derived from an N-glycan chain of the antibody.

In some embodiments, the oligosaccharide consists of 2-15 monosaccharides; preferably, the oligosaccharide consists of 2-10 monosaccharides.

In some embodiments, the oligosaccharide has the structure represented by formula V-a or formula V-b: 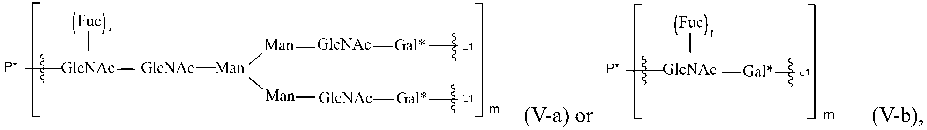
wherein P* is an antibody, GlcNAc is N-acetylglucosamine, Fuc is fucose, Man is mannose, f is 0 or 1, and m is 1 to 20;
Gal* is a modified galactose selected from the following structures: 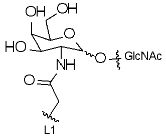 and 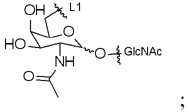
the oligosaccharide is linked to the antibody by the core GlcNAc.

In some embodiments, the modified galactose is linked to GlcNAc by a β-1,4-glycosidic bond.

In some embodiments, the oligosaccharide is linked to the Fc fragment of the antibody, preferably to the CH2 domain of the Fc fragment, and more preferably to Asn297 (numbered according to the EU index of Kabat) of the Fc fragment.

In some specific embodiments, the protein-drug conjugate described in the present application has the structure represented by formula VI: 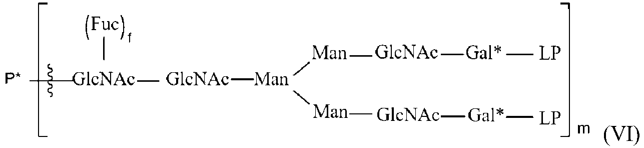
wherein P* is an antibody (the antibody may be as defined above), GlcNAc is N-acetylglucosamine, Fuc is fucose, Man is mannose, f is 0 or 1, and m is 1 to 10;
Gal* is a modified galactose selected from the following structures: 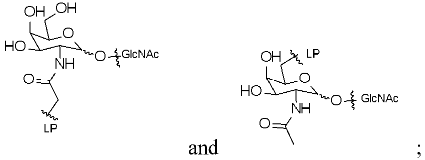
LP is defined in the same way as the aforementioned L-D; the core GlcNAc is directly linked to the antibody.

In some embodiments, LP is selected from one of the following structures (a) to (g):
(a) 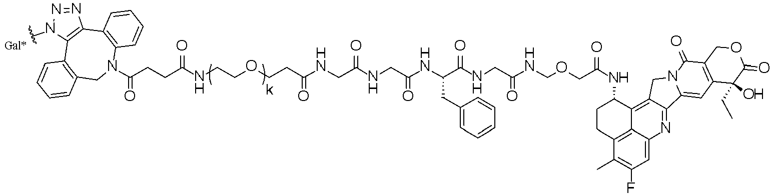 and/or 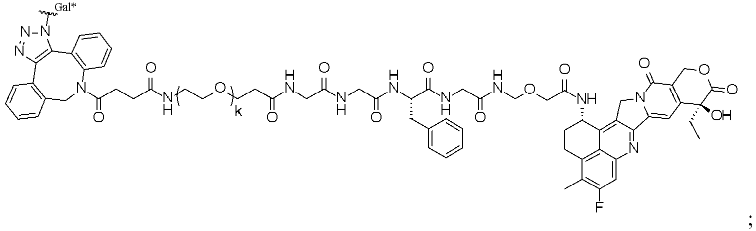
(b) 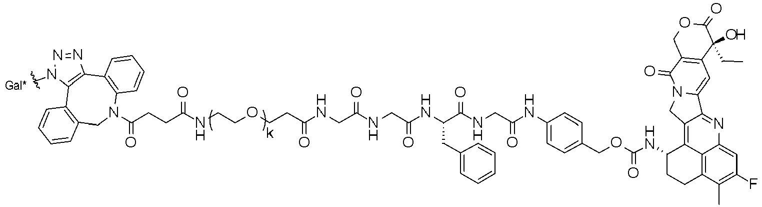 and/or 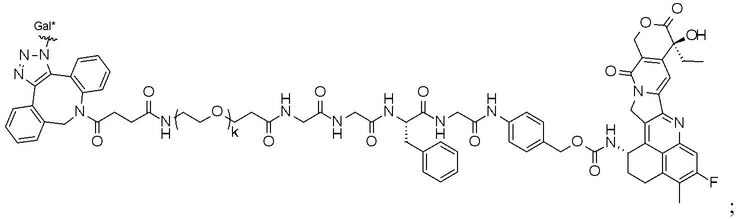
(c) 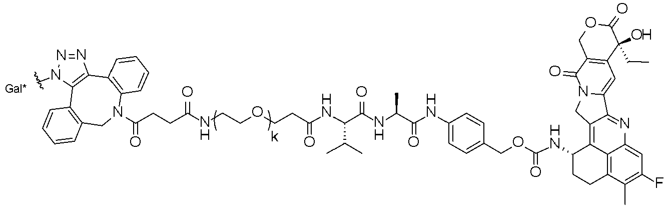 and/or 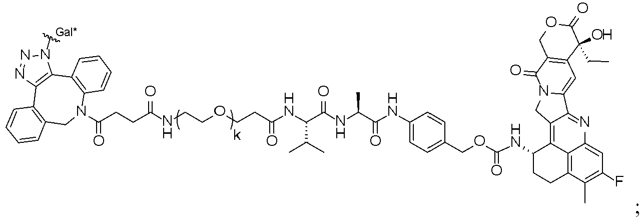
(d) 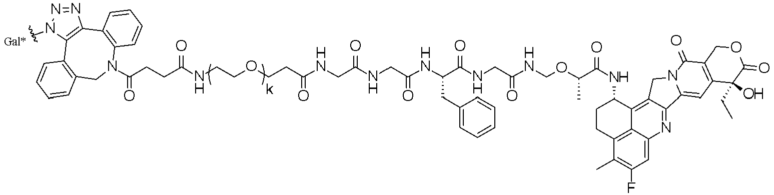 and/or 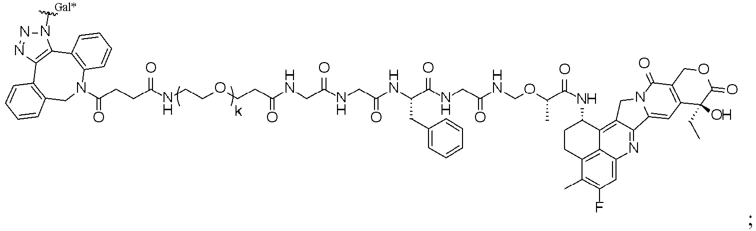
(e) 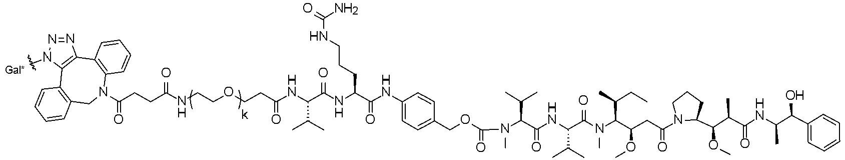 and/or 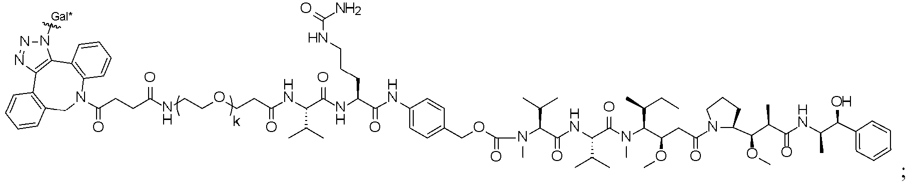
(f) 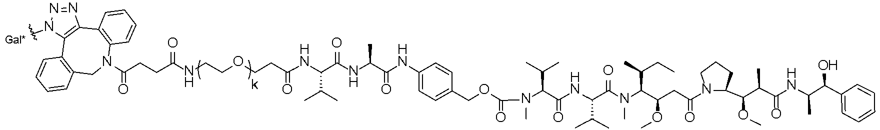 and/or 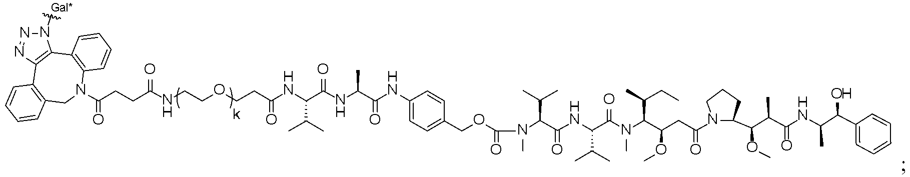 and
(g) 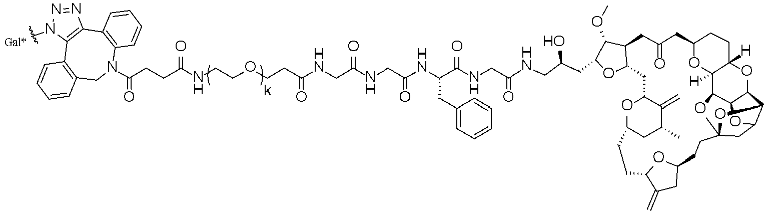 and/or 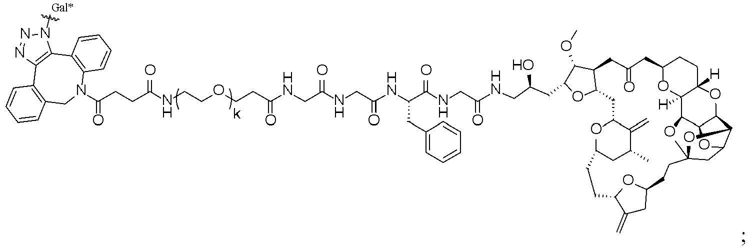 k is an integer from 1 to 20, e.g., 2, 3, 4, 5, 6, 7, 8, 9, or 10.

In some embodiments, m is 1 to 4; in some embodiments, the conjugate has an overall DAR value of 1 to 8.

In some embodiments, the antibody comprises a first heavy chain of the amino acid sequence set forth in SEQ ID NO: 20, a second heavy chain of the amino acid sequence set forth in SEQ ID NO: 21, and common light chains of the amino acid sequence set forth in SEQ ID NO: 22.

### Composition

In a second aspect, the present application further provides a composition comprising an antigen-binding molecule and a protein-drug conjugate.

In some embodiments, the composition is a solid formulation, preferably a freeze-dried formulation.

In some embodiments, the composition is a liquid formulation, preferably a solution, more preferably an injectable agent, such as a subcutaneous injectable solution or an intravenous injectable solution.

For the definitions regarding the antigen-binding molecule and the protein-drug conjugate in the composition, reference can be made to the technical features described in the first aspect. Moreover, the following definitions may be further made:
In some embodiments, the antigen-binding molecule comprises a VHH that binds to PD-L1, wherein the VHH that binds to PD-L1 comprises the amino acid sequence set forth in SEQ ID NO: 1.

In some embodiments, the PD-L1-binding molecule is a heavy-chain antibody comprising an immunoglobulin Fc region.

In some embodiments, the immunoglobulin Fc region is a human immunoglobulin Fc region, preferably, an Fc region of human IgG 1, human IgG2, human IgG3, or human IgG4, or a variant thereof.

In some embodiments, the amino acid sequence of the immunoglobulin Fc region is SEQ ID NO: 2 or SEQ ID NO: 3.

In some embodiments, the immunoglobulin single variable domain (e.g., VHH) is located at the N-terminus of the immunoglobulin Fc region, and the two are linked directly or by a peptide chain selected from one or more of the following amino acid sequences: (GS)ₙ, (GG)ₙ, (GGS)ₙ, (GGGS)ₙ, (GGSG)ₙ, (GGGGS)ₙ, and (GAP)ₙ, wherein n is an integer from 1 to 10.

In some embodiments, the amino acid sequence of the PD-L1-binding molecule is SEQ ID NO: 4.

In some embodiments, the protein-drug conjugate has the following structure: 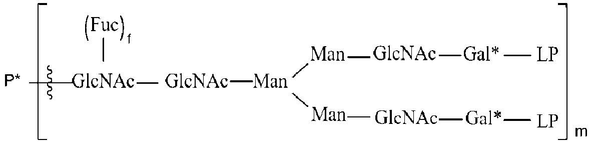
wherein P* is an antibody (the antibody may be as defined above), GlcNAc is N-acetylglucosamine, Fuc is fucose, Man is mannose, f is 0 or 1, and m is 1 to 10;
Gal* is a modified galactose selected from the following structures: 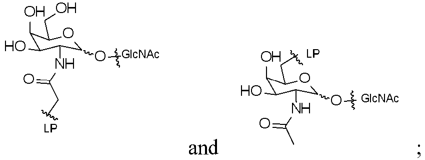
LP is: 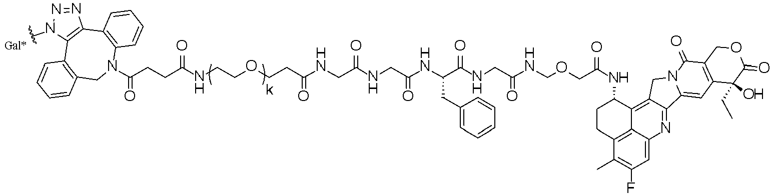 and/or 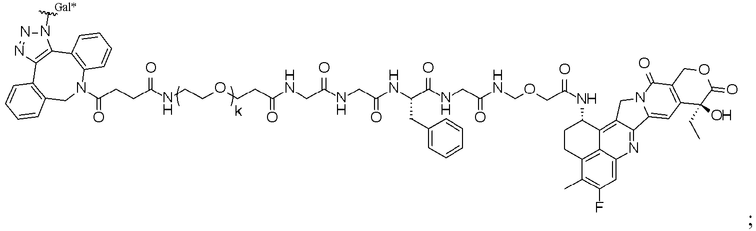
k is an integer from 1 to 20, e.g., 2, 3, 4, 5, 6, 7, 8, 9, or 10;
the core GlcNAc is directly linked to the antibody.

In some embodiments, k = 2, 4, or 8.

In some embodiments, the modified galactose is linked to GlcNAc by a β-1,4-glycosidic bond.

In some embodiments, the core GlcNAc is linked to the Fc fragment of the antibody, preferably to the CH2 domain of the Fc fragment, and more preferably to Asn297 (numbered according to the EU index of Kabat) of the Fc fragment.

In some embodiments, the antibody is a bispecific antibody that targets different epitopes of HER2.

In some embodiments, the antibody has a first heavy chain, a second heavy chain, and common light chains, wherein the first heavy chain comprises the heavy chain variable region set forth in SEQ ID NO: 17, the second heavy chain comprises the heavy chain variable region set forth in SEQ ID NO: 18, and the common light chains comprise the light chain variable regions set forth in SEQ ID NO: 19.

In some embodiments, the antibody comprises a first heavy chain of the amino acid sequence set forth in SEQ ID NO: 20, a second heavy chain of the amino acid sequence set forth in SEQ ID NO: 21, and common light chains of the amino acid sequence set forth in SEQ ID NO: 22.

For the content of each component in the composition, the protein-drug conjugate in the composition may be at a concentration of 20 mg/mL-150 mg/mL, preferably 50 mg/mL-120 mg/mL, and more preferably 60 mg/mL-100 mg/mL; specifically, the protein-drug conjugate may be at a concentration of 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 105 mg/mL, 110 mg/mL, 115 mg/mL, 120 mg/mL, 125 mg/mL, 130 mg/mL, 135 mg/mL, 140 mg/mL, 145 mg/mL, or 150 mg/mL.

In some embodiments, the antigen-binding protein or PD-L1-binding protein in the composition is at a concentration of 20 mg/mL-180 mg/mL, preferably 50 mg/mL-150 mg/mL, and more preferably 80 mg/mL-120 mg/mL; specifically, the antigen-binding protein or PD-L1-binding protein may be at a concentration of 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 105 mg/mL, 110 mg/mL, 115 mg/mL, 120 mg/mL, 125 mg/mL, 130 mg/mL, 135 mg/mL, 140 mg/mL, 145 mg/mL, 150 mg/mL, 155 mg/mL, 160 mg/mL, 165 mg/mL, 170mg/mL, 175 mg/mL, or 180 mg/mL.

In some embodiments, the composition has a ratio of the protein-drug conjugate to the PD-L1-binding molecule of 1:0.5 to 1:2, preferably 1:0.75 to 1:1.75, more preferably 1:0.8 to 1:1.5, and even more preferably 1:1 to 1:1.25.

In some embodiments, the composition has a pH of 4.0 to 7.0, preferably 4.5 to 6.5, and more preferably 5.0 to 6.0; specifically, the composition may have a pH of 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, or 7.0.

The pH of the composition described in the present disclosure may be the pH value measured after the protein-drug conjugate and the PD-L1-binding molecule are mixed with other components, e.g., the pH value of a composition obtained after buffer exchange, and/or the pH value found after the composition is mixed.

In some embodiments, the composition of the present disclosure may comprise a buffering component selected from acetic acid-sodium acetate, histidine-histidine hydrochloride, acetic acid-histidine, phosphoric acid-sodium phosphate, and citric acid-sodium hydroxide; preferably, the buffering component is acetic acid-sodium acetate, histidine-histidine hydrochloride, or acetic acid-histidine.

In some embodiments, the buffering component is at a concentration of 2 mM-50 mM, preferably 5 mM-30 mM or 2 mM-20 mM, and more preferably 10 mM-20 mM or 2 mM-10 mM; specifically, the buffering component may be at a concentration of 2 mM, 3 mM, 4 mM, 5 mM, 10 mM, 15 mM, 20 mM, 25 mM, 30 mM, 35 mM, 40 mM, 45 mM, or 50 mM.

The concentration of the buffering component may be the concentration of the buffering component in a replacement buffer and/or the concentration of the buffering component after buffer exchange, which may be the same or slightly different.

In some embodiments, the concentration of the buffering component is the concentration of the buffering component in the replacement buffer.

In some embodiments, the concentration of the buffering component is the concentration of the buffering component in the composition after buffer exchange.

In some embodiments, the composition may further comprise an auxiliary material comprising a stabilizer, wherein the stabilizer is selected from one or more of sucrose, trehalose, mannitol, sorbitol, proline, glycine, arginine hydrochloride, and glycerol, is preferably selected from one or more of sucrose, trehalose, mannitol, sorbitol, and proline, is more preferably selected from one or more of trehalose, mannitol, sorbitol, and proline, and is even more preferably proline.

In some embodiments, the stabilizer is at a concentration of 50 mM-250 mM, preferably 80 mM-200 mM, and more preferably 100 mM-150 mM; specifically, the stabilizer may be at a concentration of 50 mM, 55 mM, 60 mM, 65 mM, 70 mM, 75 mM, 80 mM, 85 mM, 90 mM, 95 mM, 100 mM, 105 mM, 110 mM, 115 mM, 120 mM, 125 mM, 130 mM, 135 mM, 140 mM, 145 mM, 150 mM, 155 mM, 160 mM, 165 mM, 170 mM, 175 mM, 180 mM, 185 mM, 190 mM, 195 mM, 200 mM, 205 mM, 210 mM, 215 mM, 220 mM, 225 mM, 230 mM, 235 mM, 240 mM, 245 mM, or 250 mM.

In some specific embodiments, the selected stabilizer is proline, trehalose, mannitol, or sorbitol, and the aforementioned stabilizer is at a concentration of 50 mM-250 mM, preferably 80 mM-200 mM, and more preferably 100 mM-150 mM.

In some embodiments, the auxiliary material may also optionally and further comprise an antioxidant, wherein the antioxidant may be selected from methionine, phenol, cresol, ascorbic acid, glutathione, sodium thiosulfate, citric acid, and nicotinamide; preferably, the antioxidant is methionine.

In some embodiments, the antioxidant is at a concentration of 10 mM-100 mM, preferably 15 mM-75 mM, more preferably 20 mM-50 mM, and even more preferably 30 mM-40 mM; specifically, the antioxidant may be at a concentration of 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, or 100 mM.

In some embodiments, the composition further comprises a surfactant selected from polysorbate 20, polysorbate 80, and poloxamer 188. The concentration of the surfactant is expressed in mg/mL or % (w/v) in the present application, and it will be appreciated by those skilled in the art that the two can be converted to each other. For example, 0.02% surfactant can be converted to 0.2 mg/mL.

In some embodiments, the surfactant is at a concentration of 0 mg/mL-0.8 mg/mL, preferably 0.05 mg/mL-0.75 mg/mL, more preferably 0.1 mg/mL-0.5 mg/mL, and even more preferably 0.2 mg/mL-0.4 mg/mL or 0.1 mg/mL-0.3 mg/mL; specifically, the surfactant may be at a concentration of 0 mg/mL, 0.01 mg/mL, 0.02 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.55 mg/mL, 0.6 mg/mL, 0.65 mg/mL, 0.7 mg/mL, 0.75 mg/mL, or 0.8 mg/mL.

In some specific embodiments, the composition may comprise a formula selected from the following:
(1) 50 mg/mL-120 mg/mL protein-drug conjugate, 50 mg/mL-150 mg/mL PD-L1-binding protein, 5 mM-30 mM histidine-histidine hydrochloride, 80 mM-200 mM proline, 20 mM-50 mM methionine, and 0.1 mg/mL-0.5 mg/mL polysorbate 80, pH 5.0 to 6.0;
(2) 50 mg/mL-120 mg/mL protein-drug conjugate, 50 mg/mL-150 mg/mL PD-L1-binding protein, 5 mM-30 mM histidine-histidine hydrochloride, 80 mM-200 mM sucrose, 20 mM-50 mM methionine, and 0.1 mg/mL-0.5 mg/mL polysorbate 80, pH 5.0 to 6.0;
(3) 50 mg/mL-120 mg/mL protein-drug conjugate, 50 mg/mL-150 mg/mL PD-L1-binding protein, 5 mM-30 mM histidine-histidine hydrochloride, 80 mM-200 mM trehalose, 20 mM-50 mM methionine, and 0.1 mg/mL-0.5 mg/mL polysorbate 80, pH 5.0 to 6.0;
(4) 50 mg/mL-120 mg/mL protein-drug conjugate, 50 mg/mL-150 mg/mL PD-L1-binding protein, 5 mM-30 mM histidine-histidine hydrochloride, 80 mM-200 mM mannitol, 20 mM-50 mM methionine, and 0.1 mg/mL-0.5 mg/mL polysorbate 80, pH 5.0 to 6.0;
(5) 50 mg/mL-120 mg/mL protein-drug conjugate, 50 mg/mL-150 mg/mL PD-L1-binding protein, 5 mM-30 mM histidine-histidine hydrochloride, 80 mM-200 mM sorbitol, 20 mM-50 mM methionine, and 0.1 mg/mL-0.5 mg/mL polysorbate 80, pH 5.0 to 6.0;
(6) 50 mg/mL-120 mg/mL protein-drug conjugate, 50 mg/mL-150 mg/mL PD-L1-binding protein, 5 mM-30 mM acetic acid-sodium acetate, 80 mM-200 mM proline, 20 mM-50 mM methionine, and 0.1 mg/mL-0.5 mg/mL polysorbate 80, pH 5.0 to 6.0;
(7) 50 mg/mL-120 mg/mL protein-drug conjugate, 50 mg/mL-150 mg/mL PD-L1-binding protein, 5 mM-30 mM acetic acid-sodium acetate, 80 mM-200 mM sucrose, 20 mM-50 mM methionine, and 0.1 mg/mL-0.5 mg/mL polysorbate 80, pH 5.0 to 6.0;
(8) 50 mg/mL-120 mg/mL protein-drug conjugate, 50 mg/mL-150 mg/mL PD-L1-binding protein, 5 mM-30 mM acetic acid-sodium acetate, 80 mM-200 mM trehalose, 20 mM-50 mM methionine, and 0.1 mg/mL-0.5 mg/mL polysorbate 80, pH 5.0 to 6.0;
(9) 50 mg/mL-120 mg/mL protein-drug conjugate, 50 mg/mL-150 mg/mL PD-L1-binding protein, 5 mM-30 mM acetic acid-sodium acetate, 80 mM-200 mM mannitol, 20 mM-50 mM methionine, and 0.1 mg/mL-0.5 mg/mL polysorbate 80, pH 5.0 to 6.0;
(10) 50 mg/mL-120 mg/mL protein-drug conjugate, 50 mg/mL-150 mg/mL PD-L1-binding protein, 5 mM-30 mM acetic acid-sodium acetate, 80 mM-200 mM sorbitol, 20 mM-50 mM methionine, and 0.1 mg/mL-0.5 mg/mL polysorbate 80, pH 5.0 to 6.0;
(11) 50 mg/mL-120 mg/mL protein-drug conjugate, 50 mg/mL-150 mg/mL PD-L1-binding protein, 5 mM-30 mM histidine-acetic acid, 80 mM-200 mM proline, 20 mM-50 mM methionine, and 0.1 mg/mL-0.5 mg/mL polysorbate 80, pH 5.0 to 6.0;
(12) 50 mg/mL-120 mg/mL protein-drug conjugate, 50 mg/mL-150 mg/mL PD-L1-binding protein, 2 mM-20 mM histidine-acetic acid, 80 mM-200 mM proline, 20 mM-50 mM methionine, and 0.1 mg/mL-0.5 mg/mL polysorbate 80, pH 5.0 to 6.0; and
(13) 50 mg/mL-120 mg/mL protein-drug conjugate, 50 mg/mL-150 mg/mL PD-L1-binding protein, 2 mM-20 mM histidine-histidine hydrochloride, 80 mM-200 mM proline, 20 mM-50 mM methionine, and 0.1 mg/mL-0.5 mg/mL polysorbate 80, pH 5.0 to 6.0.

The concentration of the buffering component may be the concentration in a replacement buffer before buffer exchange or the concentration in the composition after buffer exchange.

In some specific embodiments, the composition may comprise a formula selected from the following:
(a) 80 mg/mL protein-drug conjugate, 100 mg/mL PD-L1-binding protein, 10 mM histidine-histidine hydrochloride, 140 mM proline, 30 mM methionine, and 0.2 mg/mL polysorbate 80, pH 5.0 to 6.0;
(b) 80 mg/mL protein-drug conjugate, 100 mg/mL PD-L1-binding protein, 10 mM histidine-acetic acid, 140 mM proline, 30 mM methionine, and 0.2 mg/mL polysorbate 80, pH 5.0 to 6.0;
(c) 100 mg/mL protein-drug conjugate, 100 mg/mL PD-L1-binding protein, 10 mM histidine-histidine hydrochloride, 180 mM proline, 30 mM methionine, and 0.2 mg/mL polysorbate 80, pH 5.0 to 6.0;
(d) 80 mg/mL protein-drug conjugate, 100 mg/mL PD-L1-binding protein, 10 mM histidine-histidine hydrochloride, 180 mM sucrose, 30 mM methionine, and 0.2 mg/mL polysorbate 80, pH 5.0 to 6.0;
(e) 80 mg/mL protein-drug conjugate, 100 mg/mL PD-L1-binding protein, 10 mM histidine-histidine hydrochloride, 180 mM trehalose, 30 mM methionine, and 0.2 mg/mL polysorbate 80, pH 5.0 to 6.0;
(f) 80 mg/mL protein-drug conjugate, 100 mg/mL PD-L1-binding protein, 10 mM histidine-histidine hydrochloride, 180 mM mannitol, 30 mM methionine, and 0.2 mg/mL polysorbate 80, pH 5.0 to 6.0;
(g) 80 mg/mL protein-drug conjugate, 100 mg/mL PD-L1-binding protein, 10 mM histidine-histidine hydrochloride, 180 mM sorbitol, 30 mM methionine, and 0.2 mg/mL polysorbate 80, pH 5.0 to 6.0; and
(h) 60 mg/mL protein-drug conjugate, 90 mg/mL PD-L1-binding protein, 10 mM histidine-histidine hydrochloride, 180 mM sorbitol, 30 mM methionine, and 0.2 mg/mL polysorbate 80, pH 5.0 to 6.0.

In some embodiments, the concentration of the buffering component may be the concentration in a replacement buffer before buffer exchange.

In some other specific embodiments, the composition may comprise a formula selected from the following:
(i) 80 mg/mL protein-drug conjugate, 100 mg/mL PD-L1-binding protein, 2 mM-5 mM histidine-histidine hydrochloride, 140 mM proline, 30 mM methionine, and 0.2 mg/mL polysorbate 80, pH 5.0 to 6.0;
(j) 80 mg/mL protein-drug conjugate, 100 mg/mL PD-L1-binding protein, 2 mM-5 mM histidine-acetic acid, 140 mM proline, 30 mM methionine, and 0.2 mg/mL polysorbate 80, pH 5.0 to 6.0;
(k) 100 mg/mL protein-drug conjugate, 100 mg/mL PD-L1-binding protein, 2 mM-5 mM histidine-histidine hydrochloride, 180 mM proline, 30 mM methionine, and 0.2 mg/mL polysorbate 80, pH 5.0 to 6.0;
(l) 80 mg/mL protein-drug conjugate, 100 mg/mL PD-L1-binding protein, 2 mM-5 mM histidine-histidine hydrochloride, 180 mM sucrose, 30 mM methionine, and 0.2 mg/mL polysorbate 80, pH 5.0 to 6.0;
(m) 80 mg/mL protein-drug conjugate, 100 mg/mL PD-L1-binding protein, 2 mM-5 mM histidine-histidine hydrochloride, 180 mM trehalose, 30 mM methionine, and 0.2 mg/mL polysorbate 80, pH 5.0 to 6.0;
(n) 80 mg/mL protein-drug conjugate, 100 mg/mL PD-L1-binding protein, 2 mM-5 mM histidine-histidine hydrochloride, 180 mM mannitol, 30 mM methionine, and 0.2 mg/mL polysorbate 80, pH 5.0 to 6.0;
(o) 80 mg/mL protein-drug conjugate, 100 mg/mL PD-L1-binding protein, 2 mM-5 mM histidine-histidine hydrochloride, 180 mM sorbitol, 30 mM methionine, and 0.2 mg/mL polysorbate 80, pH 5.0 to 6.0; and
(p) 60 mg/mL protein-drug conjugate, 90 mg/mL PD-L1-binding protein, 2 mM-5 mM histidine-histidine hydrochloride, 180 mM sorbitol, 30 mM methionine, and 0.2 mg/mL polysorbate 80, pH 5.0 to 6.0.

In some embodiments, the concentration of the buffering component may be the concentration in the composition after buffer exchange.

In some embodiments, the composition of the present disclosure has an osmotic pressure suitable for subcutaneous injection, e.g., 200 mOsmo/kg-400 mOsmo/kg, preferably 250 mOsmo/kg-350 mOsmo/kg, and more preferably 280 mOsmo/kg-320 mOsmo/kg.

In some embodiments, the composition of the present disclosure has a viscosity suitable for subcutaneous injection, e.g., 2 cP-25 cP, preferably 5 cP-20 cP, and more preferably 8 cP-15 cP.

In some embodiments, the PD-L1-binding protein in the composition of the present disclosure is KN035 (envafolimab).

In some embodiments, the protein-drug conjugate in the composition of the present disclosure is JSKN003.

In some embodiments, the composition of the present disclosure does not comprise sodium chloride.

### Pharmaceutical Combination

The term "pharmaceutical combination" or "combination product" refers to a non-fixed combination product or a fixed combination product, including but not limited to a kit. The term "non-fixed combination" means that the active ingredients (e.g., (i) the programmed death ligand 1 (PD-L1)-binding molecule of the present disclosure and (ii) the protein-drug conjugate of the present disclosure) are administered, either simultaneously or sequentially (without a specific time limitation or at identical or different time intervals), to a patient as separate entities, wherein such administration provides prophylactically or therapeutically effective levels of two or more active agents in the patient. The term "fixed combination" means that two or more active agents are simultaneously administered in the form of a single entity to a patient. The dose and/or time interval of two or more active agents are/is preferably selected such that the combined use of the components can result in a therapeutic effect on the disease or condition greater than that achieved by the use of any one of the components alone. Each of the components may be in a separate formulation form, and their formulation forms may be identical or different.

Thus, in a third aspect, the present application further provides a pharmaceutical combination or combination product comprising the antigen-binding molecule and the protein-drug conjugate of the present disclosure, wherein for the definitions regarding the antigen-binding molecule and the protein-drug conjugate in the combination, reference can be made to the technical features described in the first aspect. In some embodiments, the pharmaceutical combination or combination product may further comprise one or more additional therapeutic agents, e.g., a chemotherapeutic agent.

The present application further provides a kit of parts comprising the pharmaceutical combination. For example, the kit of parts comprises, within the same package:
- a first container containing the antigen-binding molecule of the present disclosure or a pharmaceutical composition comprising the antigen-binding molecule, and
- a second container containing the protein-drug conjugate of the present disclosure or a pharmaceutical composition comprising the protein-drug conjugate,
wherein for the definitions regarding the antigen-binding molecule and the protein-drug conjugate, reference can be made to the technical features described in the first aspect.

In some embodiments, the kit of parts further comprises, within the same package, an additional container containing an additional therapeutic agent or comprising the additional therapeutic agent. In some embodiments, the additional therapeutic agent is a chemotherapeutic agent.

### Treatment Method and Use

In a fourth aspect, the present application further provides a method for treating a tumor or cancer, e.g., an advanced or metastatic solid malignancy, comprising administering to a patient in need thereof a pharmaceutical combination of a programmed death ligand 1 (PD-L1)-binding molecule and a protein-drug conjugate.

In some embodiments, the present application further provides a method for treating a tumor or cancer, e.g., an advanced or metastatic solid malignancy, comprising administering to a patient in need thereof a programmed death ligand 1 (PD-L1)-binding molecule in combination with a protein-drug conjugate.

The description of "administering...a pharmaceutical combination" or "...in combination with..." in the present application includes both the case where a plurality of drugs are administered simultaneously and the case where a plurality of drugs are administered sequentially. When administered sequentially, the plurality of drugs are administered at intervals of no more than 24 h, e.g., no more than 18 h, no more than 15 h, no more than 12 h, no more than 10 h, no more than 8 h, no more than 5 h, no more than 3 h, no more than 2 h, no more than 1 h, or no more than 0.5 h.

In a fifth aspect, the present application further provides a method for treating a tumor or cancer, e.g., an advanced or metastatic solid malignancy, comprising administering to a patient in need thereof the pharmaceutical composition of the present application.

For the definitions regarding the PD-L1-binding molecule and the protein-drug conjugate in the treatment method, reference can be made to the technical features described in the first aspect.

In some embodiments, the malignancy is an advanced/unresectable or metastatic solid malignancy and is HER2-expressing. HER2-expressing is defined as immunohistochemistry (IHC) ≥ 1+. HER2 mutations in NSCLC patients are considered HER2 expression.

In some embodiments, the solid malignancy is a gastrointestinal tumor or non-small cell lung cancer.

In some embodiments, the pharmaceutical combination has an administration ratio of the protein-drug conjugate to the PD-L1-binding molecule of 1:0.5 to 1:2, preferably 1:0.75 to 1:1.75, more preferably 1:0.8 to 1:1.5, and even more preferably 1:1 to 1:1.25.

In some embodiments, the pharmaceutical composition has a ratio of the protein-drug conjugate to the PD-L1-binding molecule of 1:0.5 to 1:2, preferably 1:0.75 to 1:1.75, more preferably 1:0.8 to 1:1.5, and even more preferably 1:1 to 1:1.25.

In some embodiments, the treatment method comprises administering to the patient in need thereof the pharmaceutical combination or the pharmaceutical composition once weekly, once every two weeks, once every three weeks, or once every four weeks.

In some embodiments, the pharmaceutical combination or the pharmaceutical composition is administered by subcutaneous injection or intravenous injection, preferably by subcutaneous injection.

In some embodiments, the pharmaceutical combination may be administered each time at a dose of 0.5 mg/kg-10.0 mg/kg, preferably 0.8 mg/kg-7.5 mg/kg, and more preferably 1.0 mg/kg-7.0 mg/kg, for example, at a dose selected from 1.1 mg/kg, 2.3 mg/kg, 4.5 mg/kg, 5.6 mg/kg, and 6.7 mg/kg, as calculated based on the body weight of the patient, wherein the dose refers to the sum of the dose of the protein-drug conjugate and the dose of the PD-L1-binding molecule.

For example, the aforementioned dose of the pharmaceutical combination comprises 0.49 mg/kg of the protein-drug conjugate and 0.61 mg/kg of the PD-L1-binding molecule (the dose is 1.1 mg/kg), 1.02 mg/kg of the protein-drug conjugate and 1.28 mg/kg of the PD-L1-binding molecule (the dose is 2.3 mg/kg), 2.0 mg/kg of the protein-drug conjugate and 2.5 mg/kg of the PD-L1-binding molecule (the dose is 4.5 mg/kg), 2.49 mg/kg of the protein-drug conjugate and 3.11 mg/kg of the PD-L1-binding molecule (the dose is 5.6 mg/kg), or 2.98 mg/kg of the protein-drug conjugate and 3.72 mg/kg of the PD-L1-binding molecule (the dose is 6.7 mg/kg).

In some embodiments, the pharmaceutical composition may be administered each time at a dose of 0.5 mg/kg-10.0 mg/kg, preferably 0.8 mg/kg-7.5 mg/kg, and more preferably 1.0 mg/kg-7.0 mg/kg, for example, at a dose selected from 1.1 mg/kg, 2.3 mg/kg, 4.5 mg/kg, 5.6 mg/kg, and 6.7 mg/kg, as calculated based on the body weight of the patient, wherein the dose refers to the sum of the dose of the protein-drug conjugate and the dose of the PD-L1-binding molecule in the composition.

For example, the aforementioned dose of the pharmaceutical composition comprises 0.49 mg/kg of the protein-drug conjugate and 0.61 mg/kg of the PD-L1-binding molecule (the dose is 1.1 mg/kg), 1.02 mg/kg of the protein-drug conjugate and 1.28 mg/kg of the PD-L1-binding molecule (the dose is 2.3 mg/kg), 2.0 mg/kg of the protein-drug conjugate and 2.5 mg/kg of the PD-L1-binding molecule (the dose is 4.5 mg/kg), 2.49 mg/kg of the protein-drug conjugate and 3.11 mg/kg of the PD-L1-binding molecule (the dose is 5.6 mg/kg), or 2.98 mg/kg of the protein-drug conjugate and 3.72 mg/kg of the PD-L1-binding molecule (the dose is 6.7 mg/kg).

In some specific embodiments, the treatment method comprises administering to the patient in need thereof the pharmaceutical combination by subcutaneous injection at a dose of 1.1 mg/kg, 2.3 mg/kg, 4.5 mg/kg, 5.6 mg/kg, or 6.7 mg/kg once weekly. Preferably, the pharmaceutical combination has a ratio of the protein-drug conjugate to the PD-L1-binding molecule of 1:1.25.

In some specific embodiments, the treatment method comprises administering to the patient in need thereof the pharmaceutical composition by subcutaneous injection at a dose of 1.1 mg/kg, 2.3 mg/kg, 4.5 mg/kg, 5.6 mg/kg, or 6.7 mg/kg once weekly. Preferably, the pharmaceutical composition has a ratio of the protein-drug conjugate to the PD-L1-binding molecule of 1:1.25.

In a fourth aspect, the present application further relates to a pharmaceutical combination or pharmaceutical composition of a programmed death ligand 1 (PD-L1)-binding molecule and a protein-drug conjugate, which can be used for treating an advanced or metastatic solid malignancy.

For the definitions regarding the PD-L1-binding molecule and the protein-drug conjugate in the pharmaceutical combination or pharmaceutical composition, reference can be made to the technical features described in the first aspect.

In some embodiments, the malignancy is an advanced/unresectable or metastatic solid malignancy and is HER2-expressing. HER2-expressing is defined as immunohistochemistry (IHC) ≥ 1+. HER2 mutations in NSCLC patients are considered HER2 expression. Optionally, the solid malignancy is a gastrointestinal tumor or non-small cell lung cancer.

In some embodiments, the pharmaceutical combination has an administration ratio of the protein-drug conjugate to the PD-L1-binding molecule of 1:0.5 to 1:2, preferably 1:0.75 to 1:1.75, more preferably 1:0.8 to 1:1.5, and even more preferably 1:1 to 1:1.25.

In some embodiments, the pharmaceutical composition has a ratio of the protein-drug conjugate to the PD-L1-binding molecule of 1:0.5 to 1:2, preferably 1:0.75 to 1:1.75, more preferably 1:0.8 to 1:1.5, and even more preferably 1:1 to 1:1.25.

In some embodiments, the treating comprises administering to a patient in need thereof the pharmaceutical combination or pharmaceutical composition once weekly, once every two weeks, once every three weeks, or once every four weeks.

In some embodiments, the pharmaceutical combination or the pharmaceutical composition is administered by subcutaneous injection or intravenous injection, preferably by subcutaneous injection.

In some embodiments, the pharmaceutical combination may be administered each time at a dose of 0.5 mg/kg-10.0 mg/kg, preferably 0.8 mg/kg-7.5 mg/kg, and more preferably 1.0 mg/kg-7.0 mg/kg, for example, at a dose selected from 1.1 mg/kg, 2.3 mg/kg, 4.5 mg/kg, 5.6 mg/kg, and 6.7 mg/kg, as calculated based on the body weight of the patient, wherein the dose refers to the sum of the dose of the protein-drug conjugate and the dose of the PD-L1-binding molecule.

For example, the aforementioned dose of the pharmaceutical combination comprises 0.49 mg/kg of the protein-drug conjugate and 0.61 mg/kg of the PD-L1-binding molecule (the dose is 1.1 mg/kg), 1.02 mg/kg of the protein-drug conjugate and 1.28 mg/kg of the PD-L1-binding molecule (the dose is 2.3 mg/kg), 2.0 mg/kg of the protein-drug conjugate and 2.5 mg/kg of the PD-L1-binding molecule (the dose is 4.5 mg/kg), 2.49 mg/kg of the protein-drug conjugate and 3.11 mg/kg of the PD-L1-binding molecule (the dose is 5.6 mg/kg), or 2.98 mg/kg of the protein-drug conjugate and 3.72 mg/kg of the PD-L1-binding molecule (the dose is 6.7 mg/kg).

In some embodiments, the pharmaceutical composition may be administered each time at a dose of 0.5 mg/kg-10.0 mg/kg, preferably 0.8 mg/kg-7.5 mg/kg, and more preferably 1.0 mg/kg-7.0 mg/kg, for example, at a dose selected from 1.1 mg/kg, 2.3 mg/kg, 4.5 mg/kg, 5.6 mg/kg, and 6.7 mg/kg, as calculated based on the body weight of the patient, wherein the dose refers to the sum of the dose of the protein-drug conjugate and the dose of the PD-L1-binding molecule.

For example, the aforementioned dose of the pharmaceutical composition comprises 0.49 mg/kg of the protein-drug conjugate and 0.61 mg/kg of the PD-L1-binding molecule (the dose is 1.1 mg/kg), 1.02 mg/kg of the protein-drug conjugate and 1.28 mg/kg of the PD-L1-binding molecule (the dose is 2.3 mg/kg), 2.0 mg/kg of the protein-drug conjugate and 2.5 mg/kg of the PD-L1-binding molecule (the dose is 4.5 mg/kg), 2.49 mg/kg of the protein-drug conjugate and 3.11 mg/kg of the PD-L1-binding molecule (the dose is 5.6 mg/kg), or 2.98 mg/kg of the protein-drug conjugate and 3.72 mg/kg of the PD-L1-binding molecule (the dose is 6.7 mg/kg).

In some specific embodiments, the treating comprises administering to the patient in need thereof the pharmaceutical combination by subcutaneous injection at a dose of 1.1 mg/kg, 2.3 mg/kg, 4.5 mg/kg, 5.6 mg/kg, or 6.7 mg/kg once weekly. Preferably, the pharmaceutical combination has a ratio of the protein-drug conjugate to the PD-L1-binding molecule of 1:1.25.

In some specific embodiments, the treating comprises administering to the patient in need thereof the pharmaceutical composition by subcutaneous injection at a dose of 1.1 mg/kg, 2.3 mg/kg, 4.5 mg/kg, 5.6 mg/kg, or 6.7 mg/kg once weekly. Preferably, the pharmaceutical composition has a ratio of the protein-drug conjugate to the PD-L1-binding molecule of 1:1.25.

In a fifth aspect, the present disclosure relates to use of a programmed death ligand 1 (PD-L1)-binding molecule in combination with a protein-drug conjugate in the preparation of a medicament for treating an advanced or metastatic solid malignancy.

The present disclosure further relates to use of a programmed death ligand 1 (PD-L1)-binding molecule in the preparation of a medicament for use in combination with a protein-drug conjugate to treat an advanced or metastatic solid malignancy.

The present disclosure further relates to use of a protein-drug conjugate in the preparation of a medicament for use in combination with a programmed death ligand 1 (PD-L1)-binding molecule to treat an advanced or metastatic solid malignancy.

The present disclosure further relates to use of a pharmaceutical combination or pharmaceutical composition of a programmed death ligand 1 (PD-L1)-binding molecule and a protein-drug conjugate in the preparation of a medicament for treating an advanced or metastatic solid malignancy.

For the definitions regarding the PD-L1-binding molecule and the protein-drug conjugate in the pharmaceutical combination or pharmaceutical composition, reference can be made to the technical features described in the first aspect.

In some embodiments, the malignancy is an advanced/unresectable or metastatic solid malignancy and is HER2-expressing. HER2-expressing is defined as immunohistochemistry (IHC) ≥ 1+. HER2 mutations in NSCLC patients are considered HER2 expression. Optionally, the solid malignancy is a gastrointestinal tumor or non-small cell lung cancer.

In some embodiments, the pharmaceutical combination has an administration ratio of the protein-drug conjugate to the PD-L1-binding molecule of 1:0.5 to 1:2, preferably 1:0.75 to 1:1.75, more preferably 1:0.8 to 1:1.5, and even more preferably 1:1 to 1:1.25.

In some embodiments, the pharmaceutical composition has a ratio of the protein-drug conjugate to the PD-L1-binding molecule of 1:0.5 to 1:2, preferably 1:0.75 to 1:1.75, more preferably 1:0.8 to 1:1.5, and even more preferably 1:1 to 1:1.25.

### Antigen-Binding Molecule and Protein-Drug Conjugate

In a sixth aspect, for the antigen-binding molecule and the protein-drug conjugate described in the first to fifth aspects, the following definitions may be further made:
In some embodiments, the antigen-binding molecule is a programmed death ligand 1 (PD-L1)-binding molecule.

In some embodiments, the PD-L1-binding molecule comprises an immunoglobulin single variable domain, wherein the immunoglobulin single variable domain comprises a CDR1, a CDR2, and a CDR3 from the VHH set forth in SEQ ID NO: 1. The CDRs may be Kabat CDRs, AbM CDRs, Chothia CDRs, or IMGT CDRs.

In some embodiments, the CDR1, the CDR2, and the CDR3 from the VHH set forth in SEQ ID NO: 1 are selected from any one of the following groups: SEQ ID NOs: 5-7 (Kabat), SEQ ID NOs: 8-10 (AbM), SEQ ID NOs: 11-13 (Chothia), and SEQ ID NOs: 14-16 (IMGT).

In some embodiments, the immunoglobulin single variable domain comprises the amino acid sequence set forth in SEQ ID NO: 1. Preferably, the amino acid sequence of the immunoglobulin single variable domain is set forth in SEQ ID NO: 1.

Optionally, the aforementioned PD-L1-binding molecule further comprises an immunoglobulin Fc region. Preferably, the immunoglobulin Fc region is a human immunoglobulin Fc region, e.g., an Fc region of human IgG1, human IgG2, human IgG3, or human IgG4, or a variant thereof.

In some specific embodiments, the amino acid sequence of the immunoglobulin Fc region is set forth in SEQ ID NO: 2 or SEQ ID NO: 3.

In some embodiments, the immunoglobulin single variable domain may be located at the N-terminus of the immunoglobulin Fc region or the C-terminus of the immunoglobulin Fc region; the two are linked directly or by a peptide chain.

In some embodiments, the aforementioned peptide chain is selected from one or more of the following amino acid sequences: (GS)ₙ, (GG)ₙ, (GGS)ₙ, (GGGS)ₙ, (GGSG)ₙ, (GGGGS)ₙ, and (GAP)ₙ, wherein n is an integer from 1 to 10.

In some embodiments, the PD-L1-binding molecule comprises the amino acid sequence set forth in SEQ ID NO: 4; preferably, the amino acid sequence of the PD-L1-binding molecule is set forth in SEQ ID NO: 4.

In some embodiments, the protein-drug conjugate has the structure of formula I:
P-(L-D)n (I), wherein P is a protein, L is a linking unit, D is a substance having biological activity, and n is an integer from 1 to 20.

In some embodiments, the protein P has desired physiological properties; preferably, the protein P is capable of preventing and/or treating a disease or condition. Specifically, the protein P may be an antibody, a fusion protein, a cytokine, a polypeptide, or the like.

In some embodiments, the protein P is an antibody; the antibody targets one or more of the following antigens: 4-1BB, 4-1BBL, A33, adenosine A2a receptor, Akt, ALK, androgen receptor, Ang-1, Ang-2, Annexin A3, Aurora A, Aurora B, B7-H3, B7-H4, Bcl-2, Bcr-Ab1, BRAF, BTK, BTLA, BTN2A1, CA-125, CAIX, CCR4, CD105/endoglin, CD109, CD123, CD155, CD16, CD160, CD19, CD20, CD200, CD200R, CD22, CD24, CD25, CD27, CD28, CD30, CD33, CD36, CD37, CD38, CD40, CD40L, CD47, CD48, CD52, CD70, CD79b, CD80, CD86, CD96, CDK4, CDK6, CDK9, CEA, CEACAM1, ChK1, ChK2, c-KIT, c-Met, COX2, CSF-1R, CSF2, CTLA-4, CXCR2, CXCR4, DDR2, DLL3, DLL4, DNAM-1, DR5, EGFR, EpCAM, EPHA3, EphB4, ERK1, ERK2/p38 MAPK, FAK, FAP, FGF-2, FGFR1, FGFR2, FGFR3, FGFR4, Flt-3, Gal-9, GITR, GITRL, Glypican-3, HDAC1, HDAC2, HDAC3, HDAC4, HDAC5, HDAC7, HDAC8, HDAC9, HER2, HER3, HER4/ERBB4, HGF, HHLA2, HIF-1α, HSP27, HSP90, HVEM, ICOS, ICOS Ligand, IDO, IGF1R, IL-13, IL-6, JAK1, JAK2, JAK3, KRAS, LAG-3, LIGHT, MDM2, MEK1, MEK2, MMP-1, MMP-10, MMP-11, MMP-13, MMP-2, MMP-7, MMP-9, mTOR, Mucin 1, Myc, NF-κB, NKG2A, NRAS, NTRK1, NTRK2, NTRK3, OX40, OX40L, p53, PAF, PARP1, PARP2, PD1, PDGFR-α, PDGFR-β, PD-L1, PD-L2, PI3Kα, PI3Kβ, PI3Kγ, PI3Kδ, PIM1, PIM3, PSMA, PTEN, RAF-1, RANKL, RET, S100A4, SIRPα, SLAMF7, SMO, Src, STAT3, STEAP-1, SYK, TDO, TGFβ, Tie-2, TIGIT, TIM-3, TLR8, TMIGD2, TNF-α, Toll-like receptor 3, TRAIL, TRAILR1, TROP-2, VEGF, VEGF-C, VEGFR-1, VEGFR-2, VEGFR-3, and VISTA.

In some embodiments, the antibody may target HER2; preferably, the antibody may bind to different epitopes of human HER2, e.g., extracellular domain II of human HER2 and/or extracellular domain IV of human HER2.

In some embodiments, the antibody is a bispecific antibody that targets different epitopes of HER2. In some embodiments, the antibody may specifically bind to extracellular domains II and IV of human HER2.

In some embodiments, the antibody has a first heavy chain, a second heavy chain, and common light chains, wherein the first heavy chain comprises a CDR1, a CDR2, and a CDR3 from the heavy chain variable region set forth in SEQ ID NO: 17, the second heavy chain comprises a CDR1, a CDR2, and a CDR3 from the heavy chain variable region set forth in SEQ ID NO: 18, and the common light chains comprise a CDR1, a CDR2, and a CDR3 from the light chain variable region set forth in SEQ ID NO: 19. The CDRs may be Kabat CDRs, AbM CDRs, Chothia CDRs, or IMGT CDRs.

The CDR1, the CDR2, and the CDR3 from the heavy chain variable region set forth in SEQ ID NO: 17 are selected from any one of the following groups: SEQ ID NOs: 23-25 (Kabat), SEQ ID NOs: 26-28 (AbM), SEQ ID NOs: 29-31 (Chothia), and SEQ ID NOs: 32-34 (IMGT).

The CDR1, the CDR2, and the CDR3 from the heavy chain variable region set forth in SEQ ID NO: 18 are selected from any one of the following groups: SEQ ID NOs: 35-37 (Kabat), SEQ ID NOs: 38-40 (AbM), SEQ ID NOs: 41-43 (Chothia), and SEQ ID NOs: 44-46 (IMGT).

The CDR1, the CDR2, and the CDR3 from the light chain variable region set forth in SEQ ID NO: 19 are selected from any one of the following groups: SEQ ID NOs: 47-49 (Kabat), SEQ ID NOs: 50-52 (AbM), SEQ ID NOs: 53-55 (Chothia), and SEQ ID NOs: 56-58 (IMGT).

In some embodiments, the antibody comprises a first heavy chain variable region set forth in SEQ ID NO: 17.

In some embodiments, the antibody comprises a second heavy chain variable region set forth in SEQ ID NO: 18.

In some embodiments, the antibody comprises common light chain variable regions set forth in SEQ ID NO: 19.

In some embodiments, the amino acid sequence of the first heavy chain of the antibody is set forth in SEQ ID NO: 20.

In some embodiments, the amino acid sequence of the second heavy chain of the antibody is set forth in SEQ ID NO: 21.

In some embodiments, the amino acid sequence of the common light chain of the antibody is set forth in SEQ ID NO: 22.

In some embodiments, the linking unit L in formula I has the structure represented by formula II:

-L1-sp1-L2-sp2- (II)

wherein L1 is a linker for linking to P, sp1 is a first spacing unit, L2 is a cleavable linker, and sp2 is a second spacing unit and is linked to D.

In some embodiments, the linking unit L in formula I has the structure represented by formula II:

-L1-sp1-L2-sp2- (II)

wherein L1 is a linker for linking to P, sp1 is a first spacing unit, L2 is absent, and sp2 is a second spacing unit and is linked to D.

In some embodiments, L1 in formula II is selected from: 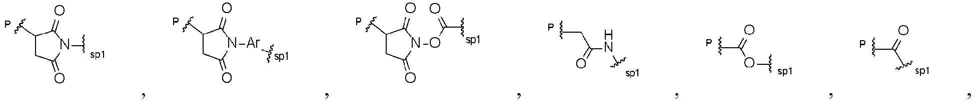 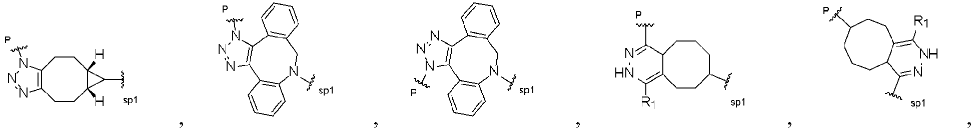 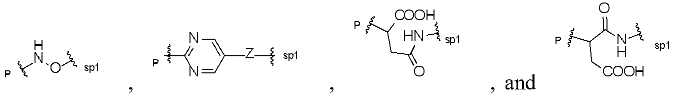 (the side linked to the protein is denoted by P, and the side linked to the first spacing unit is denoted by sp1),
wherein Ar represents C₆₋₁₀ arylene optionally substituted with halogen or C₁₋₆ alkyl; R₁ is selected from hydrogen, halogen, and C₁₋₆ alkyl; Z is selected from a direct bond, C₂₋₆ alkynylene, C₂₋₆ alkenylene, C₆₋₁₀ arylene, 5-10 membered heteroarylene, amido, sulfonamido, imino, and CF₂.

In some embodiments, L1 is

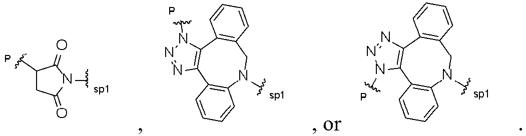

In some embodiments, the first spacing unit sp1 in formula II has the structure of: 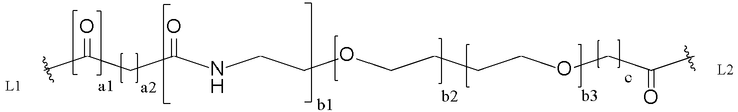 (the side linked to L1 is denoted by L1, and the side linked to L2 is denoted by L2), wherein a1 = 0 or 1, a2 = an integer from 0 to 6, b1 = 0 or 1, b2 = an integer from 0 to 16, b3 = an integer from 0 to 16, and c = an integer from 0 to 6, and at least one of b2 and b3 is 0.

In some embodiments, in the structure of sp1, a1 = 1; in some other embodiments, a1 = 0.

In some embodiments, a2 = 0, 1, 2, 3, 4, 5, or 6.

In some embodiments, b1 = 0 or 1.

In some embodiments, b2 = 0, 1, 2, 3, 4, 5, 6, 7, 8, 9, 10, 11, 12, 13, 14, 15, or 16.

In some embodiments, b3 = 0, 1, 2, 3, 4, 5, 6, 7, 8, 9, 10, 11, 12, 13, 14, 15, or 16.

In some embodiments, c = 0, 1, 2, 3, 4, 5, or 6.

The above optional embodiments of a1, a2, b1, b2, b3, and c can be combined arbitrarily, provided that at least one of b2 and b3 is 0.

In some specific embodiments, the structure of sp1 is selected from the following groups:
(1) a1 = 0, a2 = 2, 3, 4, 5, or 6, b1 = 0, b2 = 0, b3 = 0, and c = 0;
(2) a1 = 0, a2 = 0, b1 = 0, b2 = 0, b3 = 0, and c = 2, 3, 4, 5, or 6;
(3) a1 = 1, a2=2, 3, 4, 5, or 6, b1 = 1, b2 = 2, 3, 4, 5, 6, 7, or 8, b3 = 0, and c = 0;
(4) a1 = 0, a2 = 2, 3, 4, 5, or 6, b1= 1, b2 = 2, 3, 4, 5, 6, 7, or 8, b3 = 0, and c = 0; and
(5) a1 = 1, a2 = 0, b1 = 0, b2 = 0, b3 = 2, 3, 4, 5, 6, 7, or 8, and c = 2, 3, 4, 5, or 6.

In some embodiments, the structure of sp1 is specifically selected from the following groups:
(1.1) a1 = 0, a2 = 2, b1 = 0, b2 = 0, b3 = 0, and c = 0;
(1.2) a1 = 0, a2 = 3, b1 = 0, b2 = 0, b3 = 0, and c = 0;
(1.3) a1 = 0, a2 = 4, b1 = 0, b2 = 0, b3 = 0, and c = 0;
(1.4) a1 = 0, a2 = 5, b1 = 0, b2 = 0, b3 = 0, and c = 0;
(1.5) a1 = 0, a2 = 6, b1 = 0, b2 = 0, b3 = 0, and c = 0;
(2.1) a1 = 0, a2 = 0, b1 = 0, b2 = 0, b3 = 0, and c = 2;
(2.2) a1 = 0, a2 = 0, b1 = 0, b2 = 0, b3 = 0, and c = 3;
(2.3) a1 = 0, a2 = 0, b1 = 0, b2 = 0, b3 = 0, and c = 4;
(2.4) a1 = 0, a2 = 0, b1 = 0, b2 = 0, b3 = 0, and c = 5;
(2.5) a1 = 0, a2 = 0, b1 = 0, b2 = 0, b3 = 0, and c = 6;
(3.1) a1 = 1, a2 = 2, b1 = 1, b2 = 2, b3 = 0, and c = 0;
(3.2) a1 = 1, a2 = 2, b1 = 1, b2 = 3, b3 = 0, and c = 0;
(3.3) a1 = 1, a2 = 2, b1 = 1, b2 = 4, b3 = 0, and c = 0;
(3.4) a1 = 1, a2 = 2, b1 = 1, b2 = 5, b3 = 0, and c = 0;
(3.5) a1 = 1, a2 = 2, b1 = 1, b2 = 6, b3 = 0, and c = 0;
(3.6) a1 = 1, a2 = 2, b1 = 1, b2 = 7, b3 = 0, and c = 0;
(3.7) a1 = 1, a2 = 2, b1 = 1, b2 = 8, b3 = 0, and c = 0;
(3.8) a1 = 1, a2 = 3, b1 = 1, b2 = 2, b3 = 0, and c = 0;
(3.9) a1 = 1, a2 = 3, b1 = 1, b2 = 3, b3 = 0, and c = 0;
(3.10) a1 = 1, a2 = 3, b1 = 1, b2 = 4, b3 = 0, and c = 0;
(3.11) a1 = 1, a2 = 3, b1 = 1, b2 = 5, b3 = 0, and c = 0;
(3.12) a1 = 1, a2 = 3, b1 = 1, b2 = 6, b3 = 0, and c = 0;
(3.13) a1 = 1, a2 = 3, b1 = 1, b2 = 7, b3 = 0, and c = 0;
(3.14) a1 = 1, a2 = 3, b1 = 1, b2 = 8, b3 = 0, and c = 0;
(3.15) a1 = 1, a2 = 4, b1 = 1, b2 = 2, b3 = 0, and c = 0;
(3.16) a1 = 1, a2 = 4, b1 = 1, b2 = 3, b3 = 0, and c = 0;
(3.17) a1 = 1, a2 = 4, b1 = 1, b2 = 4, b3 = 0, and c = 0;
(3.18) a1 = 1, a2 = 4, b1 = 1, b2 = 5, b3 = 0, and c = 0;
(3.19) a1 = 1, a2 = 4, b1 = 1, b2 = 6, b3 = 0, and c = 0;
(3.20) a1 = 1, a2 = 4, b1 = 1, b2 = 7, b3 = 0, and c = 0;
(3.21) a1 = 1, a2 = 4, b1 = 1, b2 = 8, b3 = 0, and c = 0;
(4.1) a1 = 0, a2 = 2, b1 = 1, b2 = 2, b3 = 0, and c = 0;
(4.2) a1 = 0, a2 = 2, b1 = 1, b2 = 3, b3 = 0, and c = 0;
(4.3) a1 = 0, a2 = 2, b1 = 1, b2 = 4, b3 = 0, and c = 0;
(4.4) a1 = 0, a2 = 2, b1 = 1, b2 = 5, b3 = 0, and c = 0;
(4.5) a1 = 0, a2 = 2, b1 = 1, b2 = 6, b3 = 0, and c = 0;
(4.6) a1 = 0, a2 = 2, b1 = 1, b2 = 7, b3 = 0, and c = 0;
(4.7) a1 = 0, a2 = 2, b1 = 1, b2 = 8, b3 = 0, and c = 0;
(4.8) a1 = 0, a2 = 3, b1 = 1, b2 = 2, b3 = 0, and c = 0;
(4.9) a1 = 0, a2 = 3, b1 = 1, b2 = 3, b3 = 0, and c = 0;
(4.10) a1 = 0, a2 = 3, b1 = 1, b2 = 4, b3 = 0, and c = 0;
(4.11) a1 = 0, a2 = 3, b1 = 1, b2 = 5, b3 = 0, and c = 0;
(4.12) a1 = 0, a2 = 3, b1 = 1, b2 = 6, b3 = 0, and c = 0;
(4.13) a1 = 0, a2 = 3, b1 = 1, b2 = 7, b3 = 0, and c = 0;
(4.14) a1 = 0, a2 = 3, b1 = 1, b2 = 8, b3 = 0, and c = 0;
(4.15) a1 = 0, a2 = 4, b1 = 1, b2 = 2, b3 = 0, and c = 0;
(4.16) a1 = 0, a2 = 4, b1 = 1, b2 = 3, b3 = 0, and c = 0;
(4.17) a1 = 0, a2 = 4, b1 = 1, b2 = 4, b3 = 0, and c = 0;
(4.18) a1 = 0, a2 = 4, b1 = 1, b2 = 5, b3 = 0, and c = 0;
(4.19) a1 = 0, a2 = 4, b1 = 1, b2 = 6, b3 = 0, and c = 0;
(4.20) a1 = 0, a2 = 4, b1 = 1, b2 = 7, b3 = 0, and c = 0;
(4.21) a1 = 0, a2 = 4, b1 = 1, b2 = 8, b3 = 0, and c = 0;
(5.1) a1 = 1, a2 = 0, b1 = 0, b2 = 0, b3 = 2, and c = 2;
(5.2) a1 = 1, a2 = 0, b1 = 0, b2 = 0, b3 = 3, and c = 2;
(5.3) a1 = 1, a2 = 0, b1 = 0, b2 = 0, b3 = 4, and c = 2;
(5.4) a1 = 1, a2 = 0, b1 = 0, b2 = 0, b3 = 5, and c = 2;
(5.5) a1 = 1, a2 = 0, b1 = 0, b2 = 0, b3 = 6, and c = 2;
(5.6) a1 = 1, a2 = 0, b1 = 0, b2 = 0, b3 = 7, and c = 2; and
(5.7) a1 = 1, a2 = 0, b1 = 0, b2 = 0, b3 = 8, and c = 2.

In some embodiments, the cleavable linker L2 in formula II is a dipeptide, tripeptide, or tetrapeptide residue.

In some embodiments, L2 is selected from the following dipeptide residues: -Phe-Lys-, -Val-Ala-, -Val-Lys-, -Val-Cit-, -Ala-Lys-, -Phe-Cit-, -Leu-Cit-, -Ile-Cit-, -Phe-Arg-, -Trp-Cit-, -Gly-Gly-, -Ala-Ala-, -Gly-Val-, and -Gly-Glu-; the left side of the dipeptide residue is linked to sp1, and the right side thereof is linked to sp2. Preferably, L2 is selected from -Val-Ala-, -Val-Lys-, and -Val-Cit-.

In some embodiments, L2 is selected from the following tripeptide residues: -Glu-Val-Ala-, -Glu-Val-Cit-, -αGlu-Val-Ala-, -αGlu-Val-Cit-, -Val-Lys-Gly, and -Val-Cit-Gly-; the left side of the tripeptide residue is linked to sp1, and the right side thereof is linked to sp2.

In some embodiments, L2 is selected from the following tetrapeptide residues: -Gly-Gly-Phe-Gly- and -Gly-Phe-Gly-Gly-; the left side of the tetrapeptide residue is linked to sp1, and the right side thereof is linked to sp2.

In some embodiments, sp2 in formula II is absent, or sp2 is selected from: 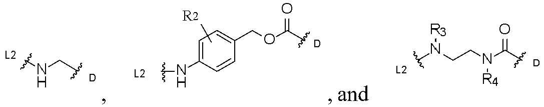 (the side linked to L2 is denoted by L2, and the side linked to the substance having biological activity D is denoted by D), wherein:
R₂ is independently selected from hydrogen, C₁₋₆ alkyl, hydroxy, amino, halogen, nitro, cyano, 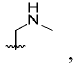 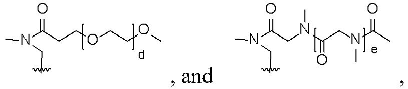 d is an integer from 1 to 20, and e is an integer from 1 to 20; R₃ and R₄ are each independently selected from hydrogen and C₁₋₆ alkyl;
the alkyl may be optionally substituted with hydroxy, amino, halogen, nitro, and cyano.

In some embodiments, sp2 is

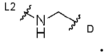

In some embodiments, sp2 is

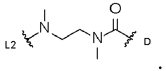

In some embodiments, sp2 is selected from 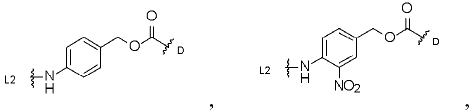 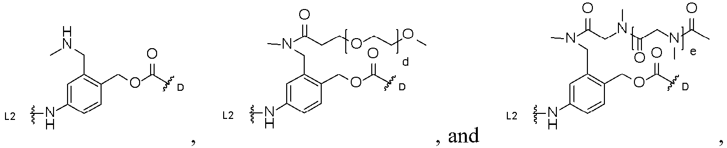 wherein d is an integer from 1 to 10, and e is an integer from 1 to 10.

In some embodiments, the linking unit L may be selected from the following structures: 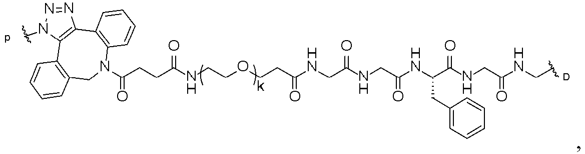 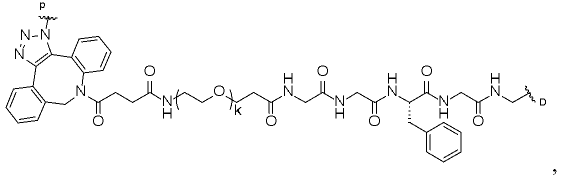 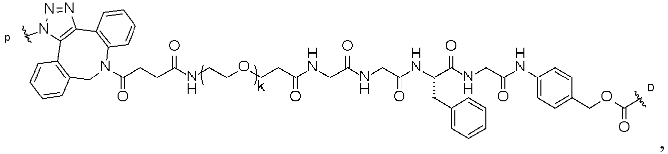 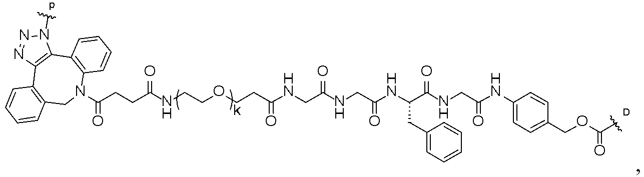 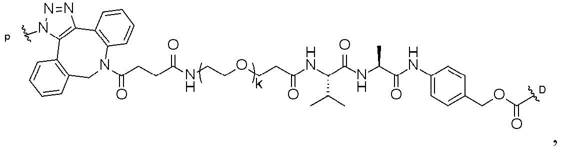 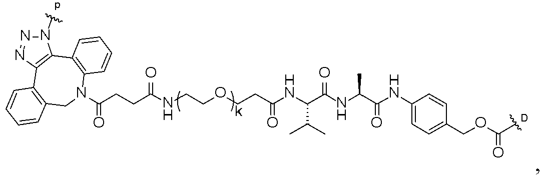 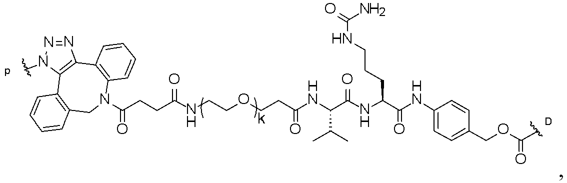 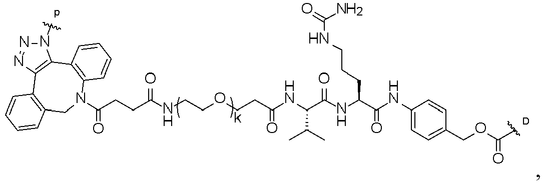 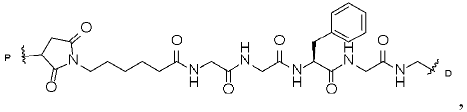 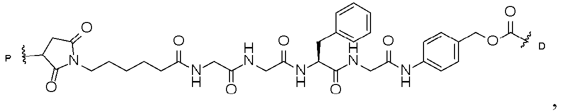 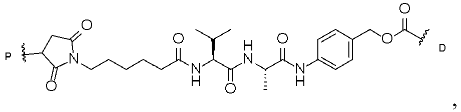 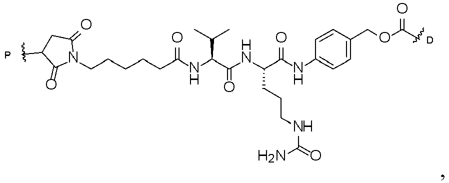 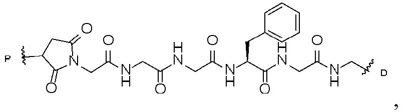 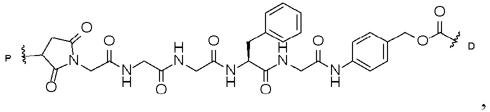 and 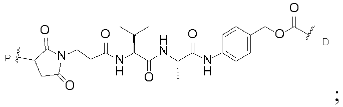 k is an integer from 1 to 20, e.g., 2, 3, 4, 5, 6, 7, 8, 9, or 10. (the side linked to the protein P is denoted by P, and the side linked to the substance having biological activity D is denoted by D).

In some embodiments, k = 2, 4, or 8.

The substance having biological activity D in formula I may be selected from a cytotoxin, a protein kinase inhibitor, an immune agonist, a glucocorticoid, an oligonucleotide, a radioisotope, a polypeptide, and any combination thereof.

In some embodiments, D is a cytotoxin selected from a DNA alkylating agent, a DNA deconstructing agent, a topoisomerase I inhibitor, a topoisomerase II inhibitor, a microtubule inhibitor, a ribosome inhibitor, and any combination thereof.

In some specific embodiments, D is selected from an auristatin derivative, a maitansine derivative, an eribulin derivative, a tubulysin derivative, a pyrrolobenzodiazepine (PDB) derivative, a duocarmycin derivative, a calicheamicin derivative, PNU-159682 and a derivative thereof, a camptothecin derivative, an amatoxin derivative, and any combination thereof.

In some embodiments, D is selected from a camptothecin derivative, which refers to a compound having the same 5-membered fused parental core structure as camptothecin of natural origin and having substitution modifications at positions 7, 9, 10, and 11 thereof, which has equivalent or greater inhibitory activity against topoisomerase I than natural camptothecin.

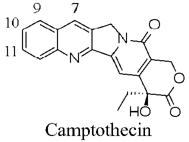

An optional camptothecin derivative may be derived from the entirety of the prior art, for example, the patent application documents WO2014057687, WO2020063676, CN111689980A, WO2022068878, WO2022068878, WO2020259258, WO2020219287, WO2022121981, WO2021173773, WO2019195665, WO2021067861, WO2022170971, WO2020200880, WO2021148501, WO2023109965, and WO2022015656, which are incorporated in the present application in their entirety.

In some embodiments, D has the structure represented by formula III: 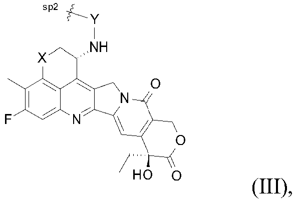
wherein X is selected from CH₂, NH, O, S, and SO₂;
Y is absent, or Y has the structure represented by 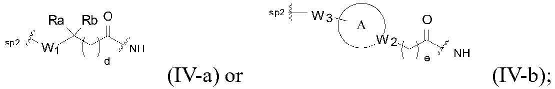
W₁ and W₃ are each independently selected from O, S, and NH, and W₂ is selected from C, CH, and N;
Rₐ and R_{b} are each independently selected from hydrogen, deuterium, halogen, C₁₋₆ alkyl, C₁₋₆ alkoxy, C₃₋₆ cycloalkyl, 3-6 membered heterocycloalkyl, hydroxy, amino, cyano, and nitro; or, Rₐ and R_{b}, together with the carbon atom to which they are attached, form 3-6 membered cycloalkyl, 3-6 membered heterocycloalkyl, or carbonyl; or, Rₐ is linked to the N atom of the amide moiety to form 3-6 membered heterocycloalkyl, and R_{b} is hydrogen;
ring A is selected from the following groups: 5-10 membered cycloalkylene, 5-10 membered heterocycloalkylene, 6-10 membered arylene, and 5-10 membered heteroarylene;
the alkyl, alkoxy, cycloalkyl, heterocycloalkyl, cycloalkylene, heterocycloalkylene, arylene, and heteroarylene are each independently, optionally, and further substituted with a group selected from deuterium, halogen, hydroxy, amino, cyano, carbonyl, and nitro;
d and e are each independently selected from integers from 0 to 5.

In some embodiments, D has the structure represented by formula III-a: 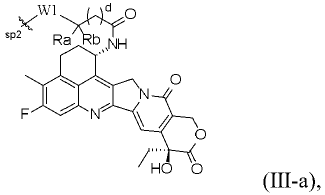
wherein W₁ is O or NH;
Rₐ and R_{b} are each independently selected from hydrogen, deuterium, halogen, C₁₋₆ alkyl, C₁₋₆ alkoxy, C₁₋₆ cycloalkyl, 3-6 membered heterocycloalkyl, hydroxy, amino, cyano, and nitro; or, Rₐ and R_{b}, together with the carbon atom to which they are attached, form 3-6 membered cycloalkyl, 3-6 membered heterocycloalkyl, or carbonyl; or, Rₐ is linked to the N atom of the amide moiety to form 3-6 membered heterocycloalkyl, and R_{b} is hydrogen;
the alkyl, alkoxy, cycloalkyl, and heterocycloalkyl are each independently, optionally, and further substituted with a group selected from deuterium, halogen, hydroxy, amino, cyano, and nitro;
d is an integer from 0 to 5.

In some embodiments, W₁ is O.

In some embodiments, d is 0, 1, or 2.

In some embodiments, Rₐ is selected from hydrogen, deuterium, halogen, C₁₋₆ alkyl, C₁₋₆ alkoxy, C₁₋₆ cycloalkyl, 3-6 membered heterocycloalkyl, hydroxy, and amino, and R_{b} is hydrogen. The alkyl, alkoxy, cycloalkyl, and heterocycloalkyl are each independently, optionally, and further substituted with a group selected from deuterium, halogen, hydroxy, amino, cyano, and nitro.

In some embodiments, Rₐ and R_{b}, together with the carbon atom to which they are attached, form 3-6 membered cycloalkyl or 3-6 membered heterocycloalkyl. The cycloalkyl and heterocycloalkyl are each independently, optionally, and further substituted with a group selected from deuterium, halogen, hydroxy, amino, cyano, and nitro.

In some embodiments, D may be selected from the following groups:

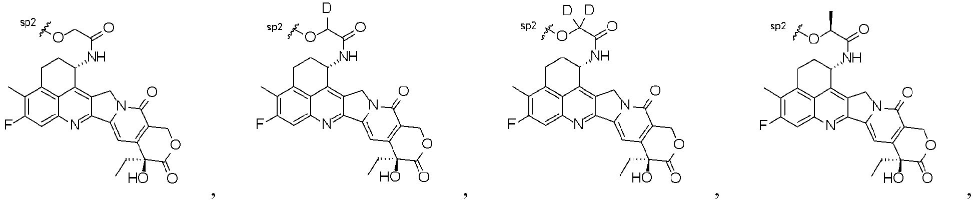

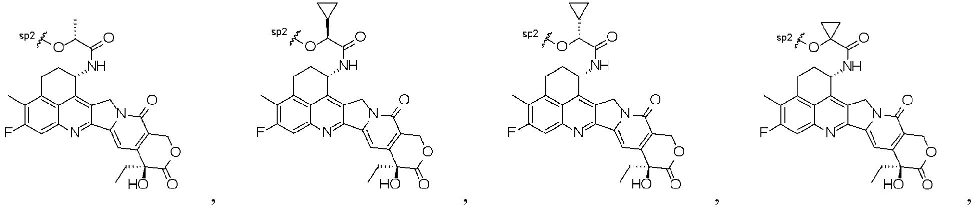

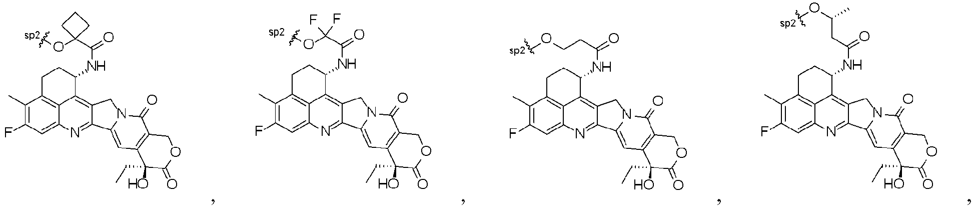

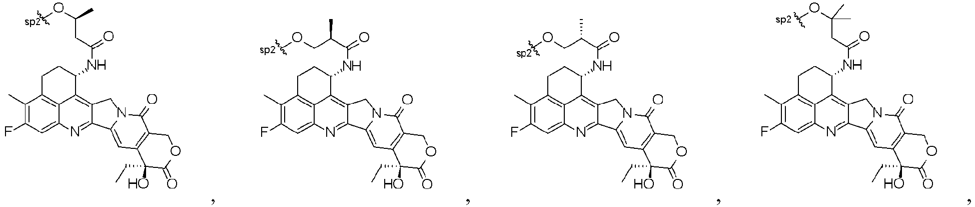

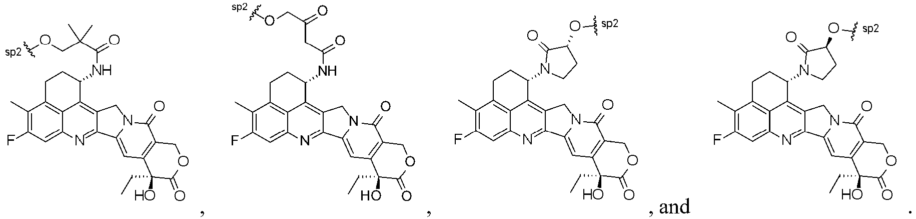

In some other embodiments, D has the structure represented by formula III-b: 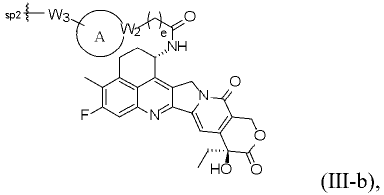
wherein W₃ is O or NH, and W₂ is C, CH, or N;
ring A is selected from the following groups: 5-10 membered cycloalkylene, 5-10 membered heterocycloalkylene, 6-10 membered arylene, and 5-10 membered heteroarylene;
the cycloalkylene, heterocycloalkylene, arylene, and heteroarylene are each independently, optionally, and further substituted with a group selected from deuterium, halogen, hydroxy, amino, cyano, carbonyl, and nitro;
e is an integer from 0 to 5.

In some embodiments, W₃ is O; in some other embodiments, W₂ is CH.

In some embodiments, e is 0, 1, or 2.

In some embodiments, ring A is 5-10 membered cycloalkylene. The cycloalkylene may be further substituted with a group selected from deuterium, halogen, hydroxy, amino, cyano, carbonyl, and nitro.

In some embodiments, D may be selected from the following groups:

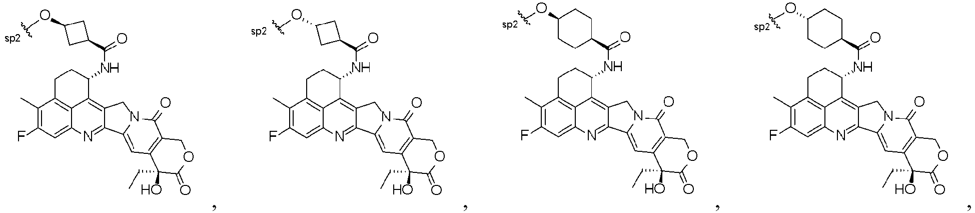

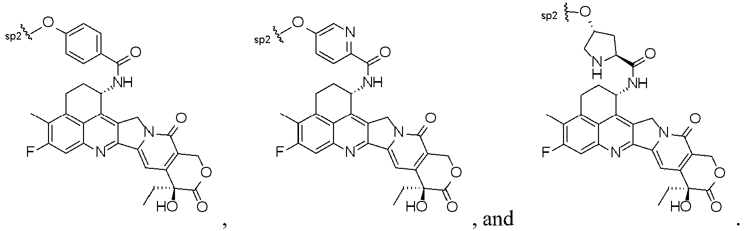

In some embodiments, D, as a camptothecin derivative, may also be selected from the following structures: 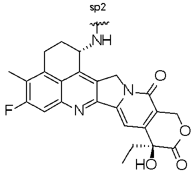 (i.e., exatecan), 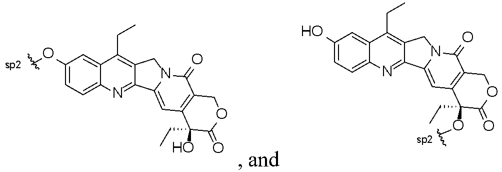 (i.e., SN-38).

In some embodiments, D may be an auristatin derivative and is preferably selected from the following structures: 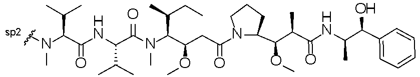 (MMAE), 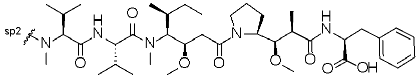 (MMAF), and 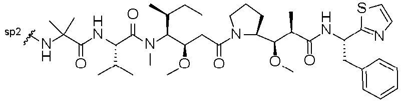 (PF-06380101).

In some embodiments, D may be a maitansine derivative and is preferably selected from the following structures: 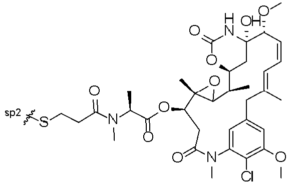 (DM1) and 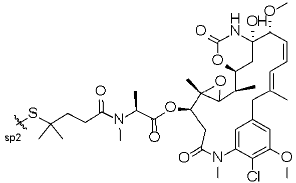 (DM4).

In some embodiments, D is eribulin and a derivative thereof and is preferably selected from the following structures:

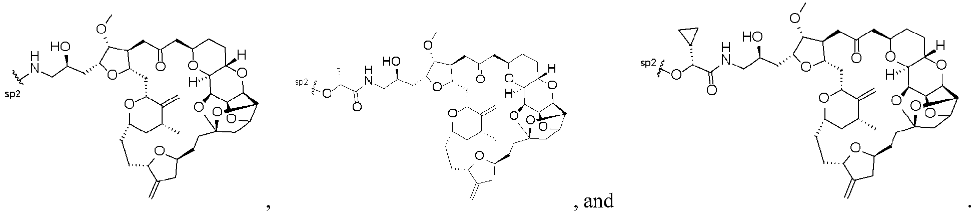

In some embodiments, in formula I, the linking unit L is linked to an antibody by sulfhydryl; preferably, the sulfhydryl is derived from the antibody; more preferably, the sulfhydryl is obtained by reducing a disulfide bond between heavy chains and/or a disulfide bond between a heavy chain and a light chain.

In some other embodiments, the linking unit L is linked to an antibody by an oligosaccharide; preferably, the oligosaccharide is derived from a natural glycan chain of the antibody.

In some embodiments, the oligosaccharide is derived from an N-glycan chain of the antibody.

In some embodiments, the oligosaccharide consists of 2-15 monosaccharides; preferably, the oligosaccharide consists of 2-10 monosaccharides.

In some embodiments, the oligosaccharide has the structure represented by formula V-a or formula V-b: 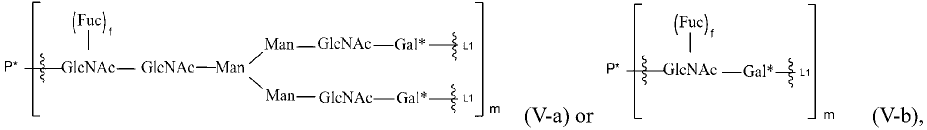
wherein P* is an antibody (the antibody may be as defined above), GlcNAc is N-acetylglucosamine, Fuc is fucose, Man is mannose, f is 0 or 1, and m is 1 to 20;
Gal* is a modified galactose selected from the following structures: 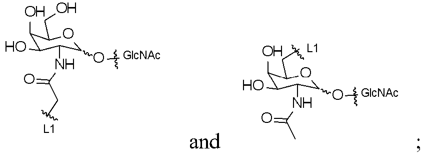
the oligosaccharide is linked to the antibody by the core GlcNAc.

In some embodiments, the modified galactose is linked to GlcNAc by a β-1,4-glycosidic bond.

In some embodiments, the oligosaccharide is linked to the Fc fragment of the antibody, preferably to the CH2 domain of the Fc fragment, and more preferably to Asn297 (numbered according to the EU index of Kabat) of the Fc fragment.

As described above, the oligosaccharide may be derived from a natural glycan chain of an antibody. Specifically, a preparation method for a precursor of the oligosaccharide comprises the following step: reacting an antibody whose N-glycoform is mainly G0F with UDP-GalNAz or a salt thereof in the presence of a catalyst to give the precursor of the aforementioned oligosaccharide.

Methods for obtaining antibodies of the G0F glycoform are well-known in the art. For example, antibodies expressed by eukaryotic cells are post-translationally modified, and the glycan chains can be converted to the G0F form by treatment with galactosidase, which removes any terminal galactose residue and leaves the terminal N-acetylglucosamine residues.

The precursor of the oligosaccharide has the structure represented by formula V-c: 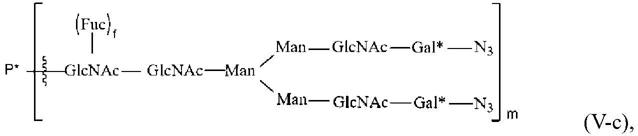 wherein P*, GlcNAc, Fuc, Man, f, and m are defined as described above, and Gal*-N₃ has the following structure:

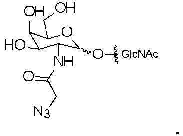

UDP-GalNAz has the following structure:

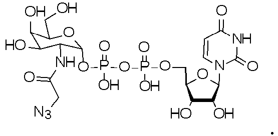

In some embodiments, the aforementioned catalyst is a galactosyltransferase or a functional variant or fragment thereof.

In some embodiments, the catalyst is β-1,4-galactosyltransferase or a functional variant or fragment thereof.

In some embodiments, the catalyst is bovine β-1,4-galactosyltransferase or human β-1,4-galactosyltransferase or a functional variant or fragment thereof.

In some embodiments, the catalyst is human β-(1,4)-GalT1 having mutation Y285L or bovine β-(1,4)-GalT1 having mutation Y289L.

In some embodiments, the catalyst is the β-1,4-acetylgalactosyltransferase disclosed in Patent Application WO2016170186.

In some embodiments, the catalyst comprises the sequence set forth in any one of SEQ ID NOs: 59-61.

In some specific embodiments, the protein-drug conjugate described in the present application has the structure represented by formula VI: 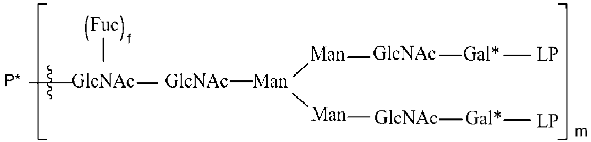
wherein P* is an antibody (the antibody may be as defined above), GlcNAc is N-acetylglucosamine, Fuc is fucose, Man is mannose, f is 0 or 1, and m is 1 to 10;
Gal* is a modified galactose selected from the following structures: 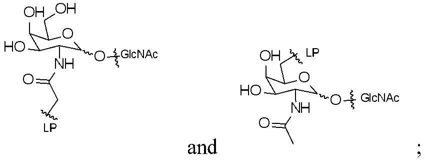
LP is selected from one of the following structures (a) to (g):
   (a) 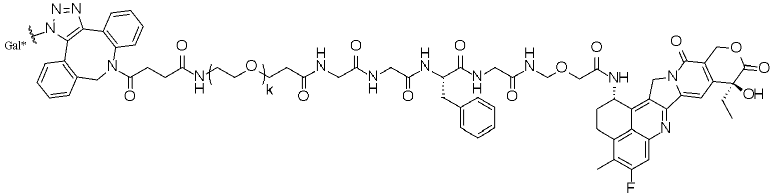 and/or 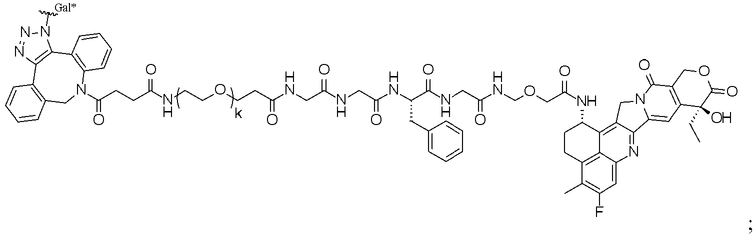
   (b) 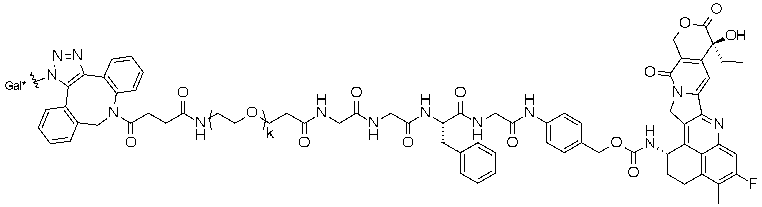 and/or 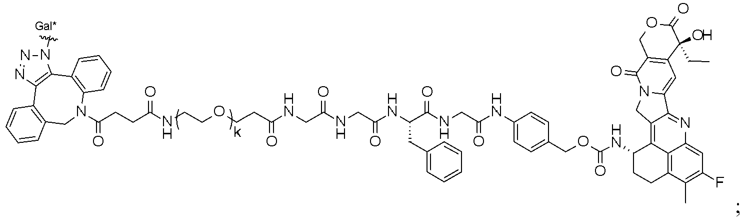
   (c) 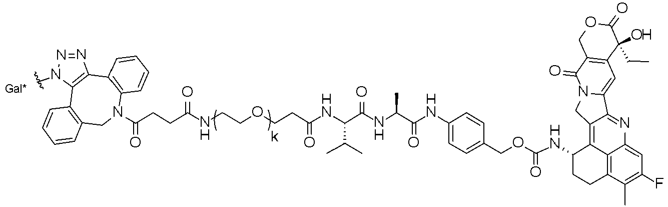 and/or 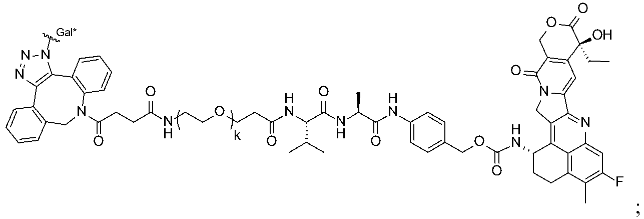
   (d) 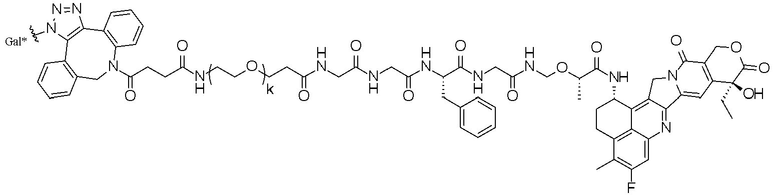 and/or 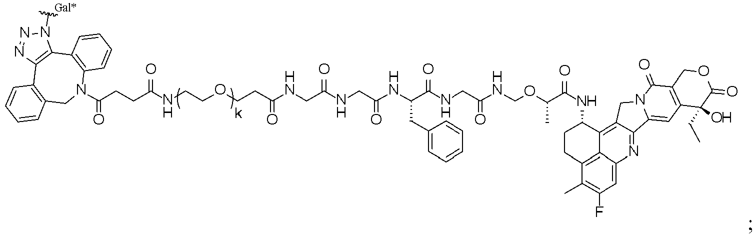
   (e) 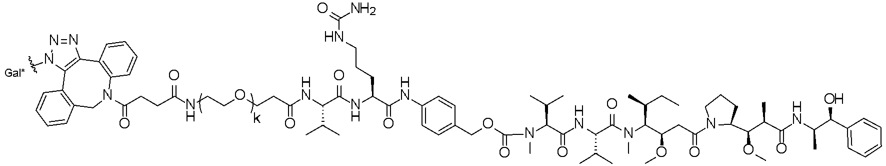 and/or 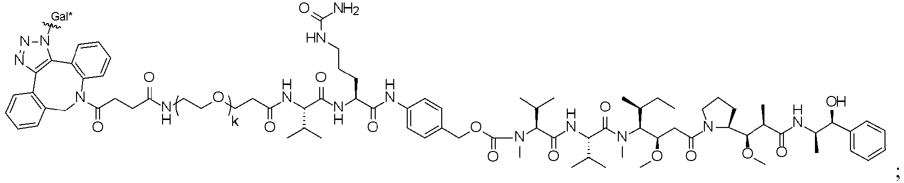
   (f) 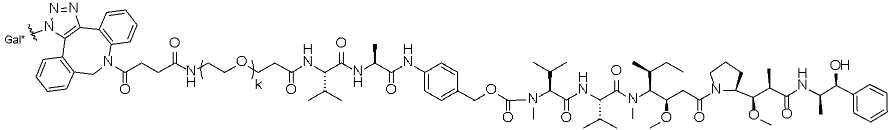 and/or 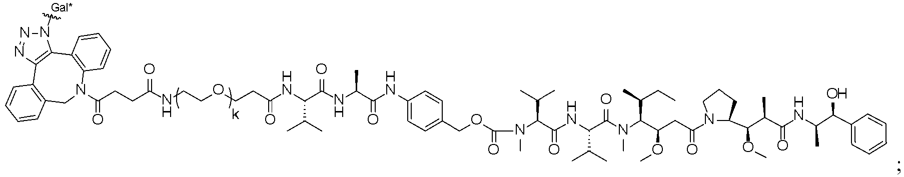 and
   (g) 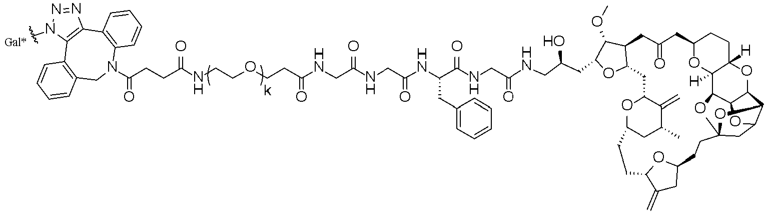 and/or 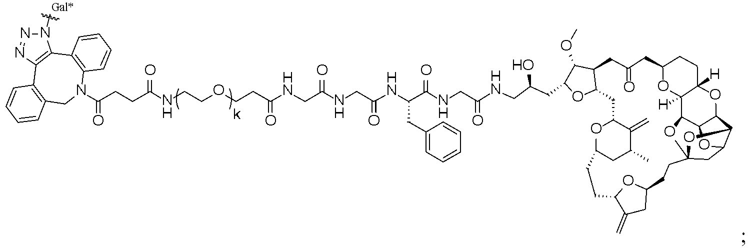 k is an integer from 1 to 20,
e.g., 2, 3, 4, 5, 6, 7, 8, 9, or 10;
the core GlcNAc is directly linked to the antibody.

In some embodiments, k = 2, 4, or 8.

In some embodiments, m is 1 to 4; in some embodiments, the conjugate has an overall DAR value of 1 to 8.

In some embodiments, m is about 1.2-3.5, e.g., 1.2, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, or 3.5.

In some embodiments, the conjugate has an overall DAR value of about 1.5 to 8, e.g., 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0.

In some embodiments, the antibody comprises a first heavy chain of the amino acid sequence set forth in SEQ ID NO: 20, a second heavy chain of the amino acid sequence set forth in SEQ ID NO: 21, and common light chains of the amino acid sequence set forth in SEQ ID NO: 22.

It will be appreciated that technical solutions obtained from any combination of any technical feature described in the sixth aspect and any technical feature of the technical solutions described in the first to fifth aspects are also included in the present application.

### Examples

The present disclosure is further illustrated below with reference to examples, and the following examples should not be construed as limiting the present disclosure.

### Terms and Abbreviations

Tm: Melting Temperature
Tagg: Aggregation Temperature
kD: Diffusion Interaction Parameter
rH: Hydrated Radius
PDI: Polymer Dispersity Index
Turbidity: Turbidity
DSF: Differential Scanning Fluorimetry
DLS: Dynamic Light Scattering
SEC-HPLC: Size Exclusion High Performance Liquid Chromatography
nrCE-SDS: non-reduced Capillary Electrophoresis-Sodium Dodecyl Sulfate
CEX-HPLC: Cation Exchange High Performance Liquid Chromatography
RH: Relative Humidity

### Example 1. Preparation of KN035 and JSKN003

### 1.1. Preparation of KN035

KN035 is an anti-PD-L1 single-domain antibody-Fc fusion protein obtained by screening from a camel immune repertoire. It is a homodimer formed from 2 identical peptide chains. The amino acid sequences of the peptide chains are set forth in SEQ ID NO: 4.

A vector containing a nucleic acid sequence capable of encoding the fusion protein was transfected into cells, and after expression and purification, the corresponding fusion protein was obtained. For the specific preparation process, refer to Patent Application WO2017020801.

### 1.2. Preparation of JSKN003

JSKN003 is an antibody-drug conjugate composed of a HER2-targeting bispecific antibody (hereinafter referred to as antibody A) and a linker-toxin.

### 1.2.1. Preparation of antibody A

Antibody A contains a first heavy chain, a second heavy chain, and common light chains. The amino acid sequence of the first heavy chain is set forth in SEQ ID NO: 20, the amino acid sequence of the second heavy chain is set forth in SEQ ID NO: 21, and the amino acid sequences of the common light chains are set forth in SEQ ID NO: 22.

Vectors containing nucleic acid sequences capable of encoding the first heavy chain, the second heavy chain, and the common light chains described above are transfected into cells, and after expression and purification, antibody A was obtained. For the specific preparation process, refer to Patent Application WO2016110267.

### 1.2.2. Preparation of linker-toxin

The following linker-toxin (LP1) was prepared according to the process described in Patent Application CN113264983A:

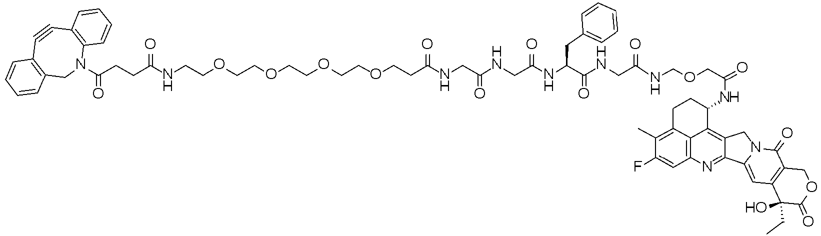

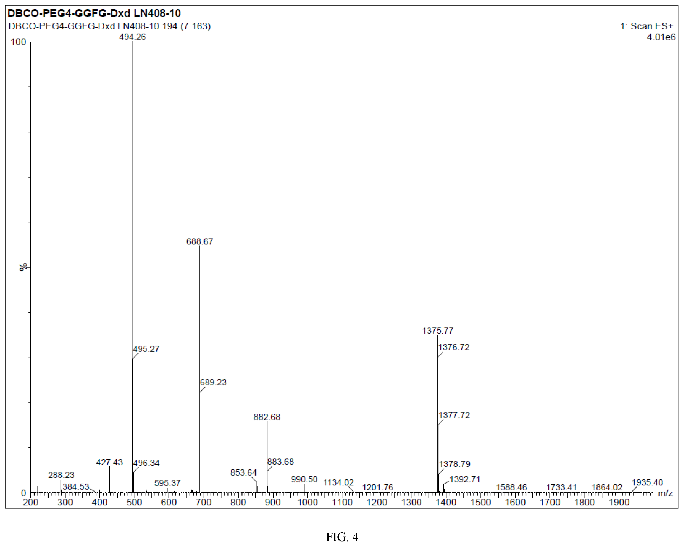

Analysis showed LP1 MS m/z (ESI): 1375.77. The MS spectrum is shown in FIG. 4.

### 1.2.3. Conjugation of antibody A to linker-toxin

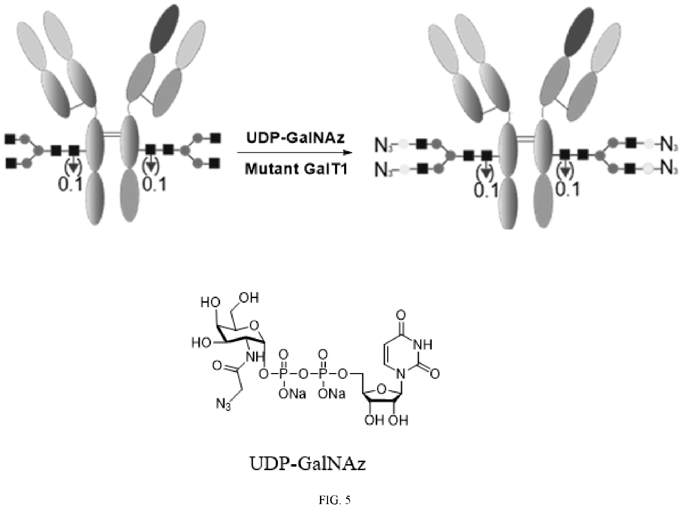

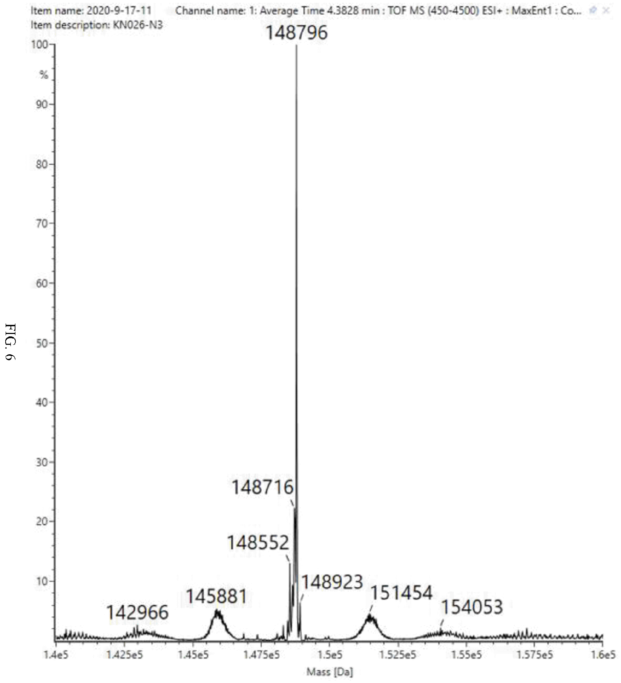

Step 1: Synthesis of antibody A-(N₃)₄: A stock solution of antibody A (mainly G0F) was desalted into an HEPES buffer, and the resulting solution was set aside for later use. Ultrapure water, MnCl₂ (10 mM), UDP-GalNAz (50 mM), Tris-HCl (10 mM), antibody A (10 mg/mL), and GalT1 (0.125 mg/mL, SEQ ID NO: 59) were added to a centrifuge tube, and the mixture was left to react at room temperature at 400 rpm for 12 h. After the reaction was complete, the reaction mixture was purified by Protein A, concentrated by ultrafiltration, and desalted into DPBS to give antibody A-(N₃)₄ (148,796 Da). The reaction scheme for this step and the structure of UDP-GalNAz are shown in FIG. 5, and the mass spectrum of the product is shown in FIG. 6.

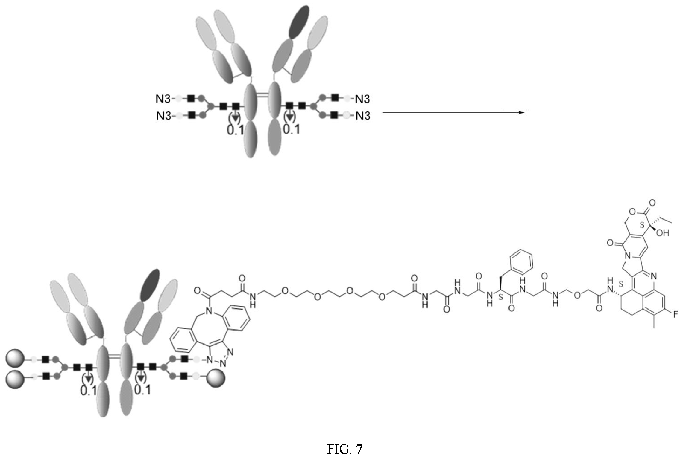

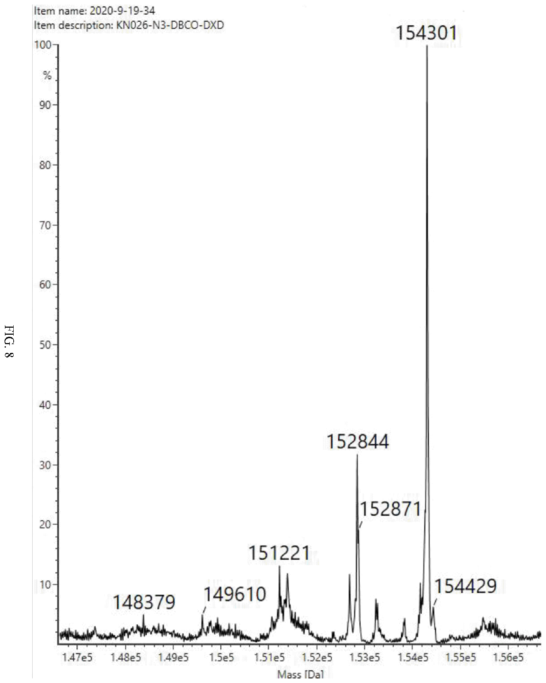

Step 2: Synthesis of antibody-drug conjugate: LP1 (950 µM), DMSO, antibody A-(N₃)₄ (63 µM), and ultrapure water were added to a centrifuge tube, and the mixture was left to react at room temperature at 400 rpm for 12 h. After the reaction was complete, the reaction mixture was desalted into DPBS to give JSKN003 (DAR = 4, 154,301 Da). The reaction scheme is shown in FIG. 7, and the mass spectrum of the product is shown in FIG. 8.

### Example 2. Study on Stability of Mixed Formulations of JSKN003 and KN035

(1)
FS0-1: A JSKN003 sample (30.75 mg/mL JSKN003, 20 mM histidine-acetic acid, pH 5.0) was concentrated by ultrafiltration to 80 mg/mL.
FS0-2: A JSKN003 sample (30.75 mg/mL JSKN003, 20 mM histidine-acetic acid, pH 5.0) was concentrated by ultrafiltration to 150 mg/mL and mixed with a KN035 sample (200 mg/mL KN035, 20 mM sodium acetate-acetic acid, 220 mM proline, 0.02% PS20, pH 6.0) in a 1:1 ratio.

**Table 1. Formulation formulas**

| No. | JSKN003 content | KN035 content | pH | Buffer system | Proline concentration | Surfactant |
|---|---|---|---|---|---|---|
| FS0-1 | 80 mg/mL | / | 5.0 | 20 mM histidine-acetic acid | / | / |
| FS0-2 | 75 mg/mL | 100 mg/mL | 5.7 | 10 mM histidine-acetic acid | 110 mM | 0.01% |
| | | | | 10 mM sodium acetate-acetic acid | | PS80 |

The test results show that when JSKN003 was present alone, after 10 days of standing at 40 °C, there were significant increases in insoluble microparticle content and aggregate content, and that when KN035 was present, under the same test conditions, the increases in total insoluble microparticle content and aggregate content were significantly slowed down (Table 2 and FIGs. 1 and 2).

**Table 2. Test results for mixed formulations**

| No. | Protein content | Insoluble microparticles (particles/mL) | 4 °C 10 d | 40 °C 10 d | SEC-HPLC (%) | 4 °C 10 d | 40 °C 10 d |
|---|---|---|---|---|---|---|---|
| FS0-1 | 80 mg/mL JSKN003 | ≥1 µm | 7515.09 | 79481.13 | HMW | 1.6 | 8.7 |
| | | ≥10 µm | 362.80 | 6114.79 | Main | 98.0 | 89.7 |
| | | ≥25 µm | 62.19 | 1184.95 | | | |
| | | | | | LMW | 0.4 | 1.6 |
| FS0-2 | 75 mg/mL JSKN003 | ≥1 µm | 2529.22 | 40890.54 | HMW | 1.7 | 3.6 |
| | | ≥10 µm | 51.83 | 1568.23 | Main | 98.2 | 96.2 |
| | 100 mg/mL KN035 | ≥25 µm | 0 | 264.90 | | | |
| | | | | | LMW | 0.1 | 0.2 |

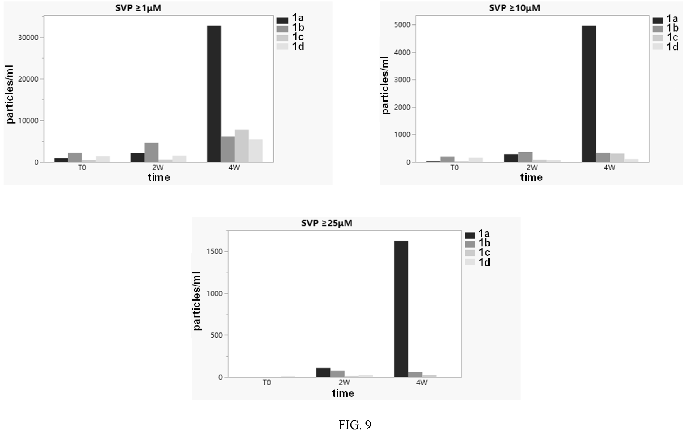

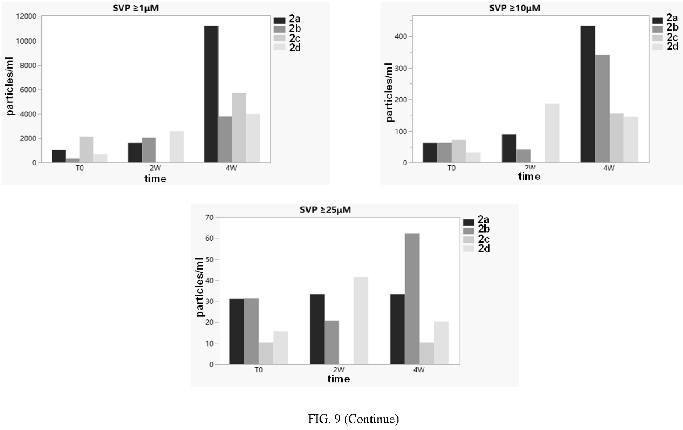

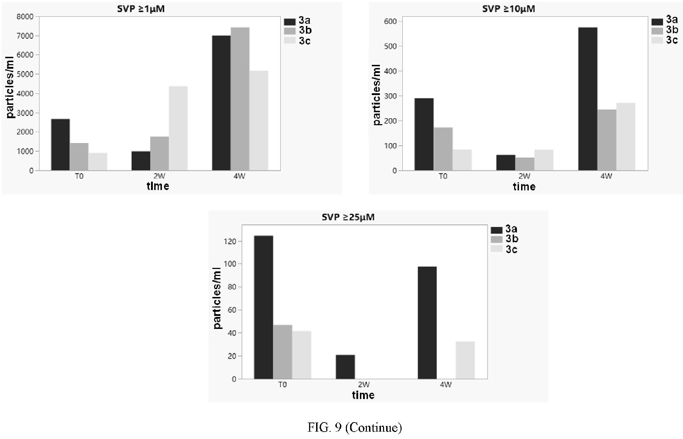

(2)
JSKN003 and KN035 samples were mixed at expected concentrations, subjected to buffer exchange using dialysis cassettes, and concentrated using ultrafiltration tubes. Then, buffer systems were added, and adjustments were made to obtain the formulas shown in Table 3. After the preparation was completed, samples of the formulas were placed in a constant temperature and humidity chamber (40 + 2 °C, 70% RH) for 4 weeks, and the insoluble microparticle content of each formula was determined. The results are shown in FIG. 9.
The test results show that in the histidine-histidine hydrochloride buffer system, when JSKN003 was present alone at a high concentration, after 4 weeks of standing at 40 °C, there was a significant increase in insoluble microparticle content, and when KN035 was present, the increase in insoluble microparticle content was significantly slowed down; in the sodium acetate-acetic acid buffer system, when KN035 was present, the increase in insoluble microparticle content also tended to slow down.

**Table 3. Formulas for mixtures of JSKN003 and KN035**

| No. | JSKN003 content | KN035 content | Buffer system | pH |
|---|---|---|---|---|
| 1a | 80 mg/mL | / | 10 mM histidine-histidine hydrochloride | 5.5 |
| 1b | 80 mg/mL | 100 mg/mL | 10 mM histidine-histidine hydrochloride | 5.5 |
| 1c | 80 mg/mL | 50 mg/mL | 10 mM histidine-histidine hydrochloride | 5.5 |
| 1d | 120 mg/mL | 80 mg/mL | 10 mM histidine-histidine hydrochloride | 5.5 |
| 2a | 80 mg/mL | / | 10 mM sodium acetate-acetic acid | 6.0 |
| 2b | 80 mg/mL | 100 mg/mL | 10 mM sodium acetate-acetic acid | 6.0 |
| 2c | 80 mg/mL | 50 mg/mL | 10 mM sodium acetate-acetic acid | 6.0 |
| 2d | 120 mg/mL | 80 mg/mL | 10 mM sodium acetate-acetic acid | 6.0 |
| 3a | 25 mg/mL | / | 10 mM histidine-histidine hydrochloride | 5.5 |
| 3b | 25 mg/mL | 25 mg/mL | 10 mM histidine-histidine hydrochloride | 5.5 |
| 3c | 25 mg/ml | 50 mg/mL | 10 mM histidine-histidine hydrochloride | 5.5 |

### Example 3. Study on Formulas for Mixed Formulations of JSKN003 and KN035

JSKN003 and KN035 samples were mixed at expected concentrations, subjected to buffer exchange using dialysis cassettes, and concentrated using ultrafiltration tubes. Then, a stabilizer, a surfactant, etc., were added, and adjustments were made to obtain the final desired formulas.

### 3.1. First round of formula screening

The objective of the first round of formula screening was to screen for a suitable pH, a suitable buffer system, and a suitable ionic strength. JSKN003 and KN035 samples were mixed, and the resulting mixtures were buffer-exchanged into the formulas shown in Table 4, respectively. The colloidal stability and high-temperature stability (40 + 2 °C, RH 70 + 5%, 11 d) were investigated, and the appearance, insoluble microparticles, SEC-HPLC, nrCE-SDS, and relative binding activity (HER2) were assessed.

**Table 4. A scheme for the first round of formula screening**

| **No.** | **JSKN003 content** | **KN035 content** | **pH*** | **Buffer system** | **NaCl concentration** | **Surfactant** |
|---|---|---|---|---|---|---|
| FS1-1 | | | 5.0 | 20 mM acetic acid/sodium acetate | 0 | |
| FS1-1-L | | | 5.0 | 20 mM acetic acid/sodium acetate | 20 mM | |
| FS1-1-M | | | 5.5 | 20 mM acetic acid/sodium acetate | 60 mM | |
| FS1-2 | | | 5.5 | 20 mM acetic acid/sodium acetate | 0 | 0.02% polysorbate 80 |
| FS1-3 | 60 mg/mL | 90 mg/mL | 6.0 | 20 mM histidine/histidine hydrochloride | 0 | |
| FS1-3-L | | | 6.0 | 20 mM histidine/histidine hydrochloride | 20 mM | |
| FS1-3-M | | | 6.0 | 20 mM histidine/histidine hydrochloride | 60 mM | |
| FS1-4 | | | 6.5 | 20 mM histidine/histidine hydrochloride | 0 | |
| FS1-5 | | | 7.0 | 20 mM histidine/histidine hydrochloride | 0 | |
| FS1-5-L | | | 7.0 | 20 mM histidine/histidine hydrochloride | 20 mM | |
| FS1-5-M | | | 7.0 | 20 mM histidine/histidine hydrochloride | 60 mM | |

| | | | | | | |
|---|---|---|---|---|---|---|
| *: the found pH value of the sample obtained after buffer exchange; the same applies hereinafter. | | | | | | |

### 3.1.1. FS1-colloidal stability

Samples of the different formulas were serially diluted, with the sample concentrations being 1.25 mg/mL, 2.5 mg/mL, 5 mg/mL, 10 mg/mL, and 20 mg/mL. The kD values were determined using a DLS module of Panta. A kD of >0 indicates that the protein tends to repel, suggesting good colloidal stability. A kD of <0 indicates that the protein tends to aggregate. The test results are shown in Table 5. The results show that the higher the NaCl concentration, the smaller the kD value; the kD values of FS 1-5-L and FS 1-5-M were <0, indicating that the high-pH formulas exhibited relatively poor colloidal stability.

**Table 5. FS 1-colloidal stability test results**

| Formula No. | kD (g/mL⁻¹) |
|---|---|
| FS1-1 | 66.26 |
| FS1-1-L | 27.11 |
| FS1-1-M | 5.39 |
| FS1-2 | 42.23 |
| FS1-3 | 36.34 |
| FS1-3-L | 31.23 |
| FS1-3-M | 8.42 |
| FS1-4 | 35.14 |
| FS1-5 | 34.61 |
| FS1-5-L | -24.17 |
| FS1-5-M | -9.74 |

### 3.1.2. FS1-high-temperature stability

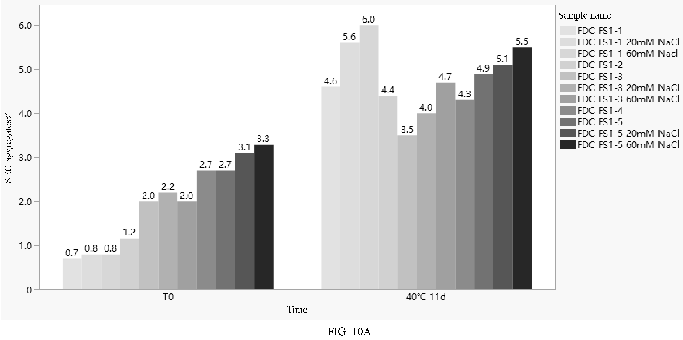

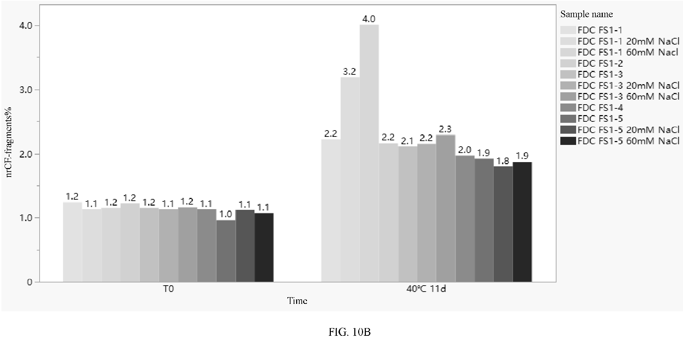

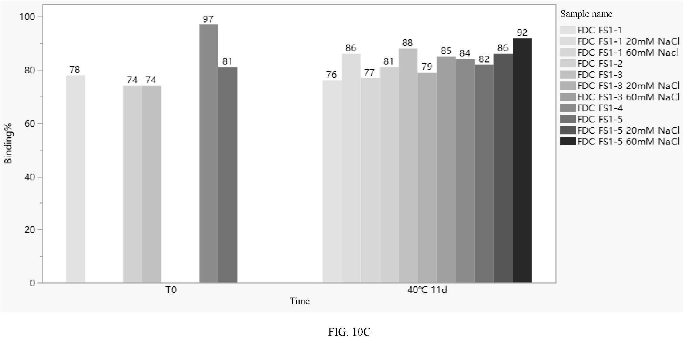

Samples of the formulas were placed in a constant temperature and humidity chamber (40 + 2 °C, 70% RH), and the appearance, insoluble microparticles, rH and PDI, SEC-HPLC, and nrCE-SDS were assessed. The test results are shown in Table 6 and FIGs. 10A-10C.

**Table 6. FS 1-high-temperature stability test results**

| Formula No. | Appearance | | 40 °C, 11 d, insoluble microparticles (particles/mL) | | | |
|---|---|---|---|---|---|---|
| | T0 | 40 °C 11 d | Total | ≥10 µm | | ≥25 µm |
| FS1-1 | Yellow | Yellow | 1369.00 | 62.94 | | 0.00 |
| FS1-1-L | Yellow | Yellow | 1653.12 | 87.01 | | 21.75 |
| FS1-1-M | Yellow | Yellow | 990.40 | 32.65 | | 0.00 |
| FS1-2 | Yellow | Yellow | 232.95 | 21.18 | | 0.00 |
| FS1-3 | Yellowish | Yellow, pinkish | 763.98 | 106.11 | | 10.61 |
| FS1-3-L | Yellowish | Yellow, pinkish | 738.32 | 10.70 | | 0.00 |
| FS1-3-M | Yellowish, slightly opalescent | Yellow, pinkish, opalescent | 717.42 | 0.00 | | 0.00 |
| FS1-4 | Yellowish | Yellow | 510.74 | 10.64 | | 10.64 |
| FS1-5 | Yellowish | Yellow, relatively light | 13825.72 | 288.70 | | 10.69 |
| FS1-5-L | Yellowish | Yellow, relatively light, opalescent | 3412.41 | 21.60 | | 0.00 |
| FS1-5-M | Yellowish, slightly opalescent | Yellow, relatively light, opalescent | 2086.55 | 21.40 | | 0.00 |

| Formula No. | rH (nm) | | PDI | | | |
|---|---|---|---|---|---|---|
| | T0 | 40 °C 11 d | T0 | | 40 °C 11 d | |
| FS1-1 | 2.04 | 3.36 | 0.15 | | 0.59 | |
| FS1-1-L | 3.07 | 6.18 | 0.12 | | 0.46 | |
| FS1-1-M | 4.21 | 10.53 | 0.16 | | 0.36 | |
| FS1-2 | 2.63 | 3.04 | 0.15 | | 0.27 | |
| FS1-3 | 2.60 | 2.94 | 0.12 | | 0.21 | |
| FS1-3-L | 2.81 | 6.09 | 0.12 | | 0.31 | |
| FS1-3-M | 3.76 | 4.13 | 0.15 | | 0.24 | |
| FS 1-4 | 2.62 | 2.75 | 0.16 | | 0.19 | |
| FS1-5 | 2.61 | 2.79 | 0.21 | | 0.24 | |
| FS1-5-L | 8.39 | 5.13 | 0.39 | | 0.25 | |
| FS1-5-M | 5.49 | 9.65 | 0.26 | 0.40 | | |

| Formula No. | SEC-aggregates (%) | SEC-JSKN003 main peak (%) | SEC-KN035 main peak (%) | | SEC-fragments (%) | |
|---|---|---|---|---|---|---|
| FS1-1 | 4.6 | 41.8 | 52.7 | | 0.8 | |
| FS1-1-L | 5.6 | 41.4 | 52.0 | | 1.1 | |
| FS1-1-M | 6.0 | 41.8 | 51.1 | | 1.1 | |
| FS1-2 | 4.4 | 42.2 | 52.7 | | 0.7 | |
| FS1-3 | 3.5 | 43.2 | 52.9 | | 0.3 | |
| FS1-3-L | 4.0 | 43.7 | 51.9 | | 0.3 | |
| FS1-3-M | 4.7 | 43.1 | 51.9 | | 0.4 | |
| FS1-4 | 4.3 | 42.4 | 53.0 | | 0.3 | |
| FS1-5 | 4.9 | 42.0 | 52.8 | | 0.3 | |
| FS1-5-L | 5.1 | 41.9 | 52.7 | | 0.3 | |
| FS1-5-M | 5.5 | 41.7 | 52.4 | | 0.3 | |

| Formula No. | nrCE-aggregates (%) | nrCE-JSKN003 main peak (%) | nrCE-KN035 main peak (%) | | nrCE-fragments (%) | |
|---|---|---|---|---|---|---|
| FS1-1 | 2.3 | 41.8 | 53.8 | | 2.2 | |
| FS1-1-L | 2.6 | 41.2 | 53.1 | | 3.2 | |
| FS1-1-M | 2.9 | 40.3 | 52.8 | | 4.0 | |
| FS1-2 | 2.6 | 41.9 | 53.4 | | 2.2 | |
| FS1-3 | 3.4 | 41.8 | 52.6 | | 2.1 | |
| FS1-3-L | 3.7 | 41.8 | 52.3 | | 2.2 | |
| FS1-3-M | 4.0 | 41.5 | 52.2 | | 2.3 | |
| FS1-4 | 5.0 | 40.2 | 52.9 | | 2.0 | |
| FS1-5 | 8.0 | 39.3 | 50.8 | | 1.9 | |
| FS1-5-L | 7.5 | 39.3 | 51.4 | | 1.8 | |
| FS1-5-M | 7.1 | 39.5 | 51.5 | | 1.9 | |

### 3.1.3. Conclusion from first round of screening

The formulas had normal appearances and good insoluble microparticle results, and there was no significant change in relative binding activity. After NaCl was added, the opalescence increased, and the aggregate content was high. As the pH increased, the SEC-HPLC aggregate content increased, and the nrCE-SDS fragment content decreased. Among them, FS1-3 had the lowest aggregate content and an acceptable fragment content. Thus, FS1-3, i.e., pH 6.0 histidine/histidine hydrochloride, was selected for the next round of formula screening.

### 3.2. Second round of formula screening

The objective of the second round of formula screening was to screen for a suitable stabilizer. JSKN003 and KN035 samples were mixed, and the resulting mixtures were buffer-exchanged into the formulas shown in Table 7, respectively. The viscosity and high-temperature stability (40 + 2 °C, 70% RH, 10 d) were investigated, and the appearance, insoluble microparticles, SEC-HPLC, nrCE-SDS, CEX-HPLC, and relative binding activity (HER2) were assessed.

**Table 7. A scheme for the second round of formula screening**

| Formula No. | KN035 Content | JSKN003 content | Buffer | Stabilizer | Antioxidant | Surfactant |
|---|---|---|---|---|---|---|
| FS2-1 | | | | 180 mM sucrose | 30 mM methionine | |
| FS2-2 | | | 10 mM histidine/histidine hydrochloride, pH 6.0 | 180 mM trehalose | | |
| FS2-3 | 100 mg/mL | 80 mg/mL | | 180 mM mannitol | | 0.02% polysorbate 80 |
| FS2-4 | | | | 180 mM sorbitol | | |
| FS2-5 | | | | 180 mM proline | | |
| FS2-6 | | | | 180 mM sucrose | / | |

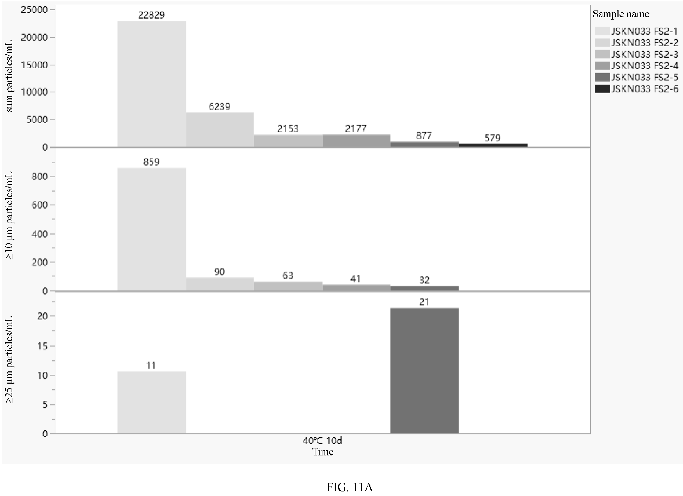

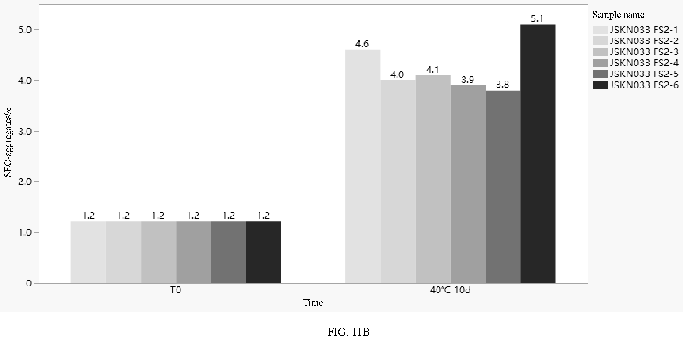

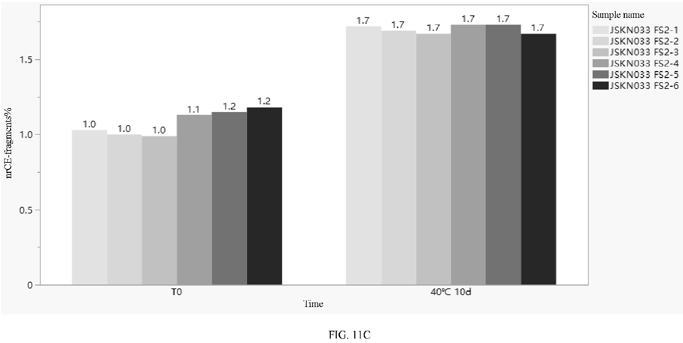

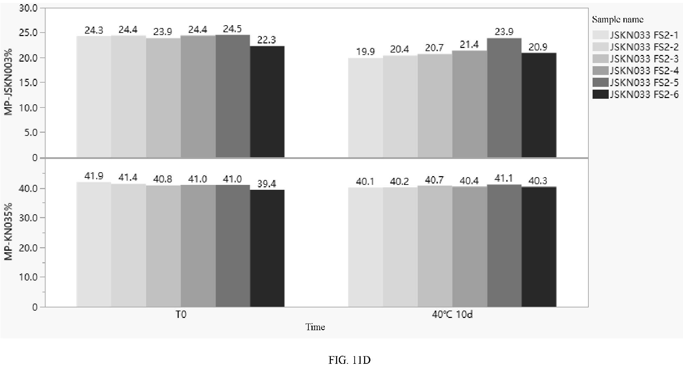

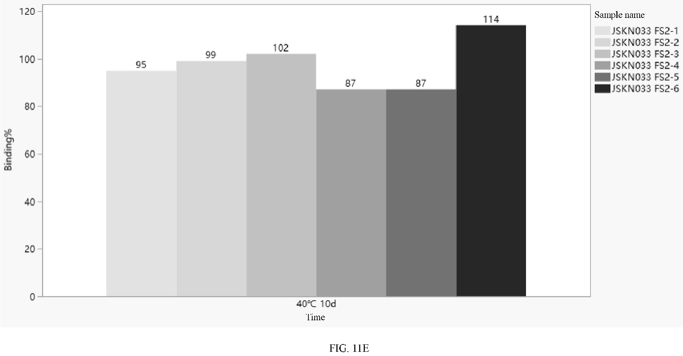

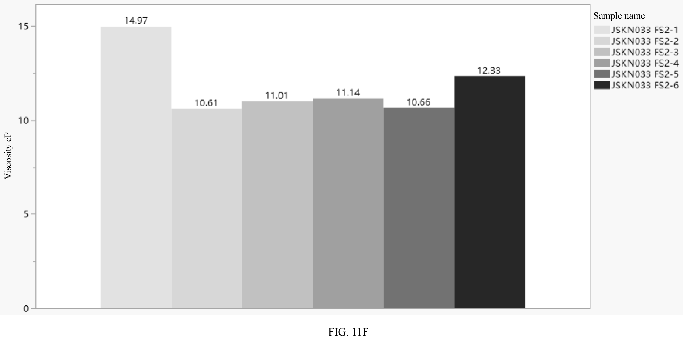

The test results are shown in Table 8 and FIGs. 11A-11F. After standing at a high temperature, FS2-5 had the lowest SEC-HPLC aggregate content and the highest CEX-HPLC main peak content. The appearance, viscosity, and relative binding activity of each formula were normal. Thus, FS2-5, i.e., proline and methionine, was selected for a third round of formula screening.

**Table 8. FS2-high-temperature stability test results**

| Formula No. | Appearance | | | | 40 °C, 10 d, insoluble microparticles (particles/mL) | | | | |
|---|---|---|---|---|---|---|---|---|---|
| | T0 | | 40 °C 10 d | | Total | | ≥10 µm | ≥25 µm | |
| FS2-1 | Slightly opalescent, yellowish | | Slightly opalescent, yellowish | | 22829.37 | | 858.88 | 10.60 | |
| FS2-2 | Slightly opalescent, yellowish | | Slightly opalescent, yellowish | | 6239.30 | | 90.42 | 0.00 | |
| FS2-3 | Slightly opalescent, yellowish | | Slightly opalescent, yellowish | | 2153.23 | | 62.59 | 0.00 | |
| FS2-4 | Slightly opalescent, yellowish | | Slightly opalescent, yellowish | | 2176.78 | | 41.46 | 0.00 | |
| FS2-5 | | Slightly opalescent, yellowish | | Slightly opalescent, yellowish | | 876.81 | | 32.08 | 21.39 |
| FS2-6 | | Slightly opalescent, yellowish | | Slightly opalescent, yellowish and slightly dark | | 579.23 | | 0.00 | 0.00 |

| Formula No. | SEC-aggregates (%) | | SEC-JSKN003 main peak (%) | SEC-KN035 main peak (%) | SEC-fragments (%) | nrCE-aggregate s (%) | nrCE-JSKN003 main peak (%) | nrCE-KN035 main peak (%) | nrCE-fragment s (%) |
|---|---|---|---|---|---|---|---|---|---|
| FS2-1 | 4.6 | | 48.1 | 47.1 | 0.2 | 2.8 | 46.2 | 49.3 | 1.7 |
| FS2-2 | 4.0 | | 48.3 | 47.5 | 0.2 | 2.2 | 46.7 | 49.4 | 1.7 |
| FS2-3 | 4.1 | | 48.3 | 47.5 | 0.2 | 2.2 | 46.8 | 49.3 | 1.7 |
| FS2-4 | 3.9 | | 48.4 | 47.5 | 0.2 | 2.2 | 46.3 | 49.8 | 1.7 |
| FS2-5 | 3.8 | | 48.5 | 47.5 | 0.2 | 2.0 | 46.1 | 50.2 | 1.7 |
| FS2-6 | 5.1 | | 47.8 | 46.8 | 0.2 | 2.8 | 46.5 | 49.0 | 1.7 |

### 3.3. Third round of formula screening

The objective of the third round of formula screening was to determine the pH, protein content, methionine content, and polysorbate 80 content. JSKN003 and KN035 samples were buffer-exchanged into the formulas shown in Table 9. After samples were prepared, the viscosity was measured, and freeze-thaw, shaking, light-exposure, and high-temperature stability tests were performed. See Table 10 for details. After each stability test was completed, the appearance, insoluble microparticles, rH and PDI, SEC-HPLC, nrCE-SDS, CEX-HPLC, Binding (HER2 and PD-L1), and Free-Drug of each sample were assessed. The results of the stability tests are shown in Tables 11-15.

**Table 9. A scheme for the third round of formula screening**

| Formula No. | JSKN003 content (mg/mL) | KN035 Content (mg/mL) | pH | Buffer system | Stabilizer | Antioxidant | Surfactant |
|---|---|---|---|---|---|---|---|
| FS3-1 | 80 | 100 | 6.0* | | | 15 mM Methionine | 0.02% polysorbate 80 |
| FS3-2 | 80 | 100 | | | | 30 mM Methionine | |
| FS3-3 | 100 | 100 | | 10 mM histidine/histidine hydrochloride | 180 mM Proline | | |
| FS3-4 | 60 | 90 | | | | | |
| FS3-6 | 80 | 100 | 5.5** | | | | |
| FS3-5 | 80 | 100 | 6.0* | | | 30 mM Methionine | 0.04% polysorbate 80 |

| | | | | | | | |
|---|---|---|---|---|---|---|---|
| *: The sample obtained after buffer exchange had a pH value of 6.0, and the replacement buffer had a pH of 5.5 before buffer exchange. **: The sample obtained after buffer exchange had a pH value of 5.5, and the replacement buffer had a pH of 5.0 before buffer exchange. | | | | | | | |

**Table 10. A stability scheme for the third round of formula screening**

| Stability test | Formula | Test conditions |
|---|---|---|
| Freeze-thaw stability | FS3-1, 3-2, 3-3, 3-4, 3-6 | Frozen at -40 °C and thawed at 2-8 °C, 5 cycles in total |
| Shaking stability | FS3-2, 3-5 | 25 + 2 °C, 200 rpm, 7 days |
| Light-exposure stability | FS3-1, 3-2, 3-3, 3-4 | Total illuminance: 0.6 MLxh |
| High-temperature stability | FS3-1, 3-2, 3-3, 3-4, 3-6 | 40 + 2 °C, RH 70 + 5%, 20 days |

**Table 11. FS3-viscosity test results**

| Formula No. | Viscosity (cP) |
|---|---|
| FS3-1 | 12.44 |
| FS3-2 | 11.41 |
| FS3-3 | 15.41 |
| FS3-4 | 6.621 |
| FS3-6 | 9.873 |

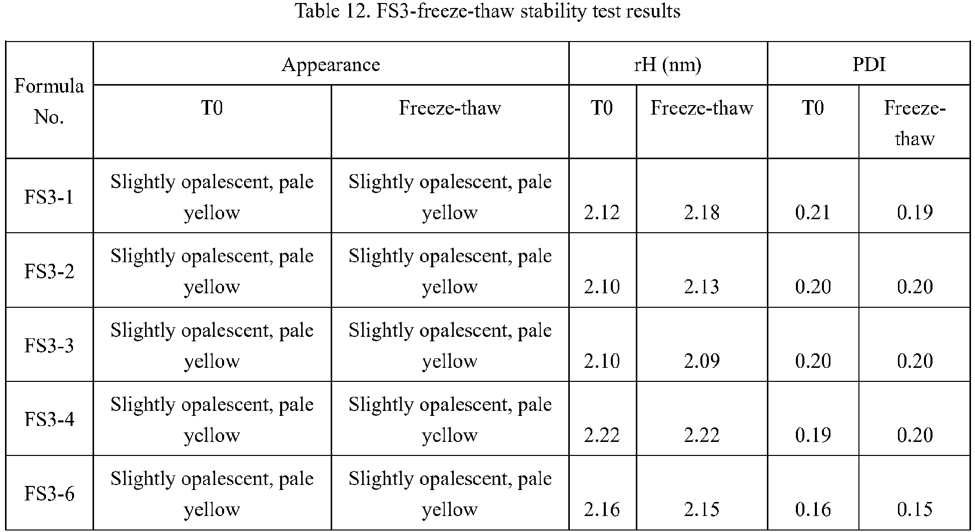

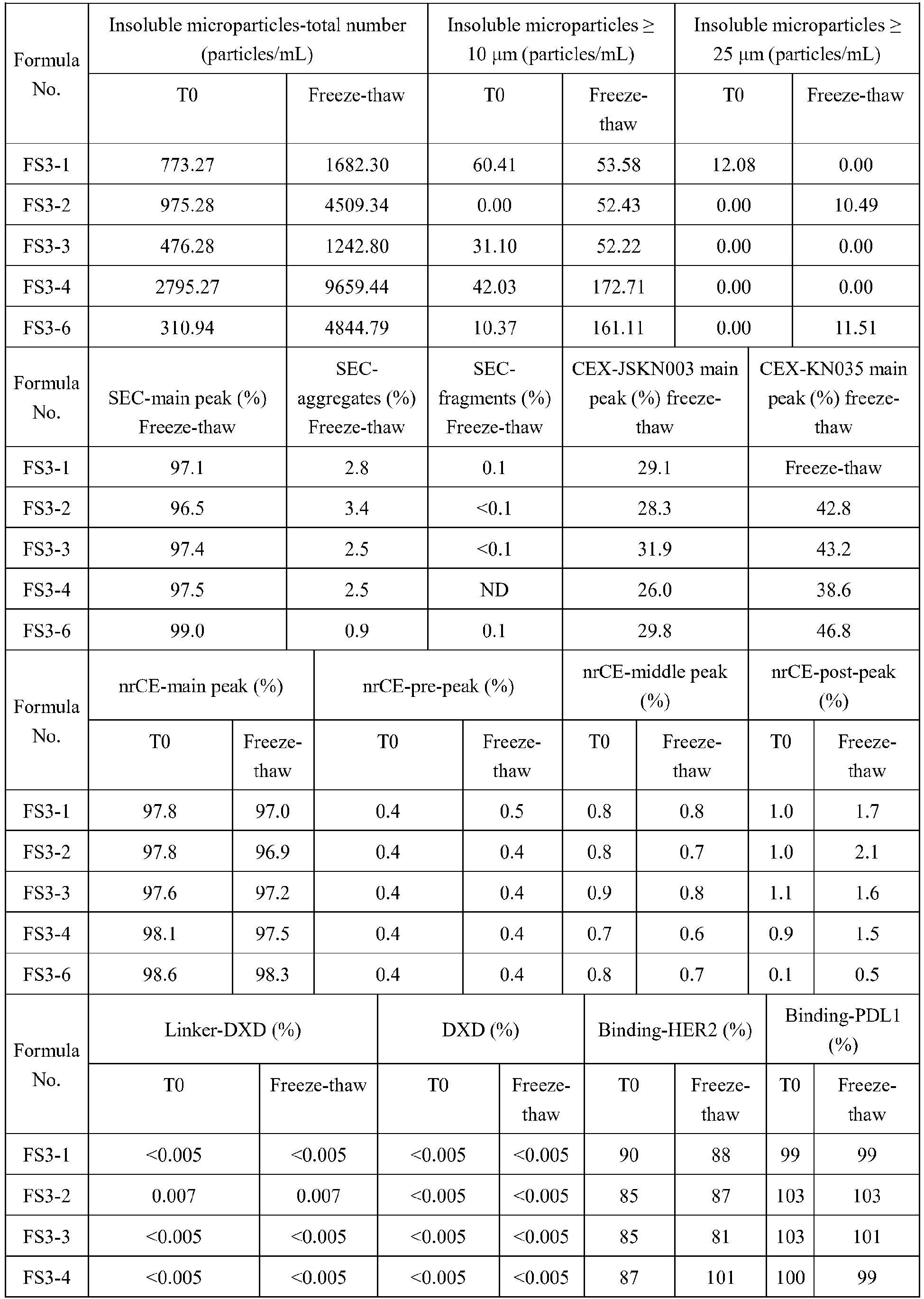

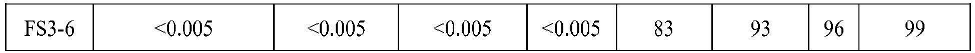

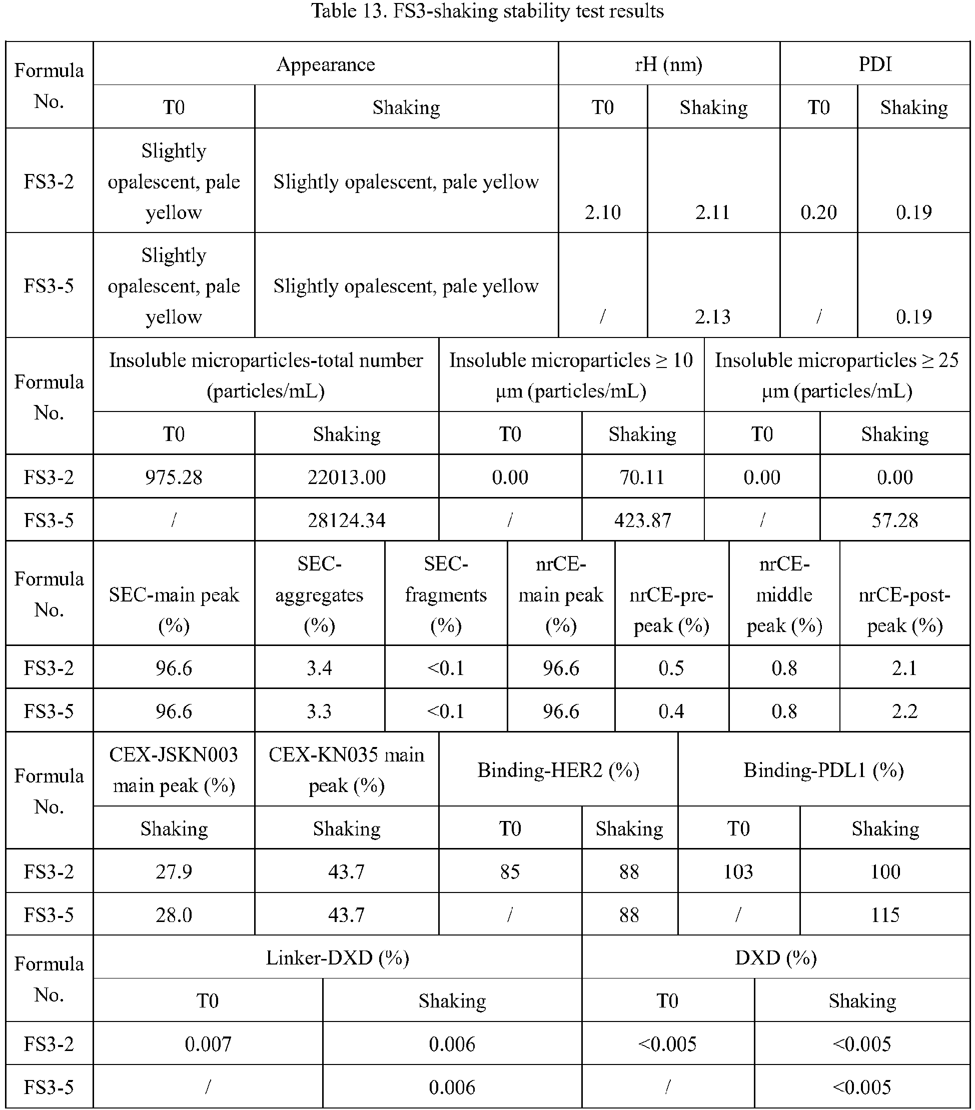

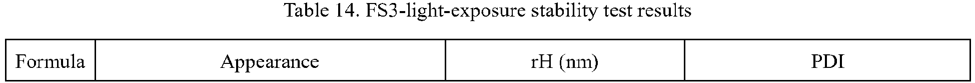

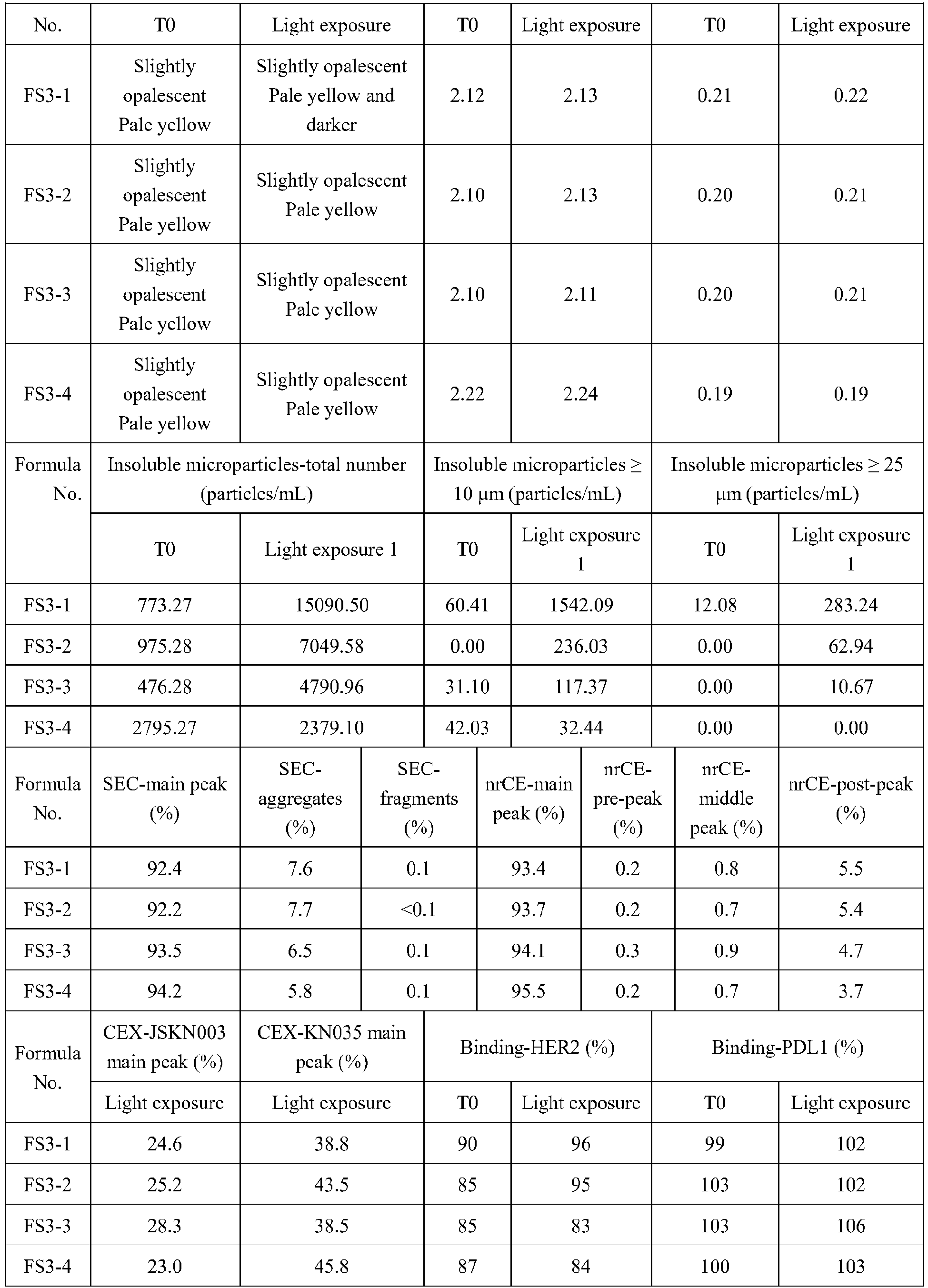

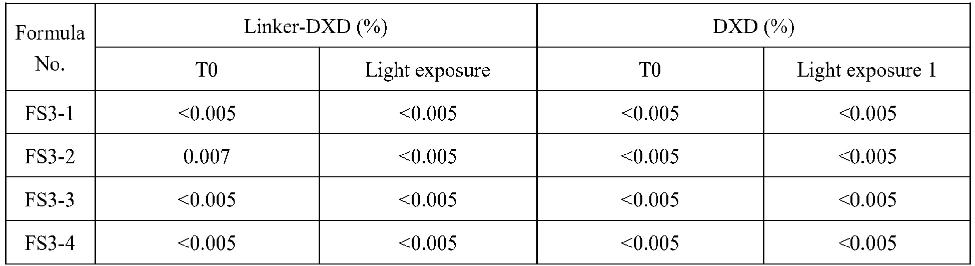

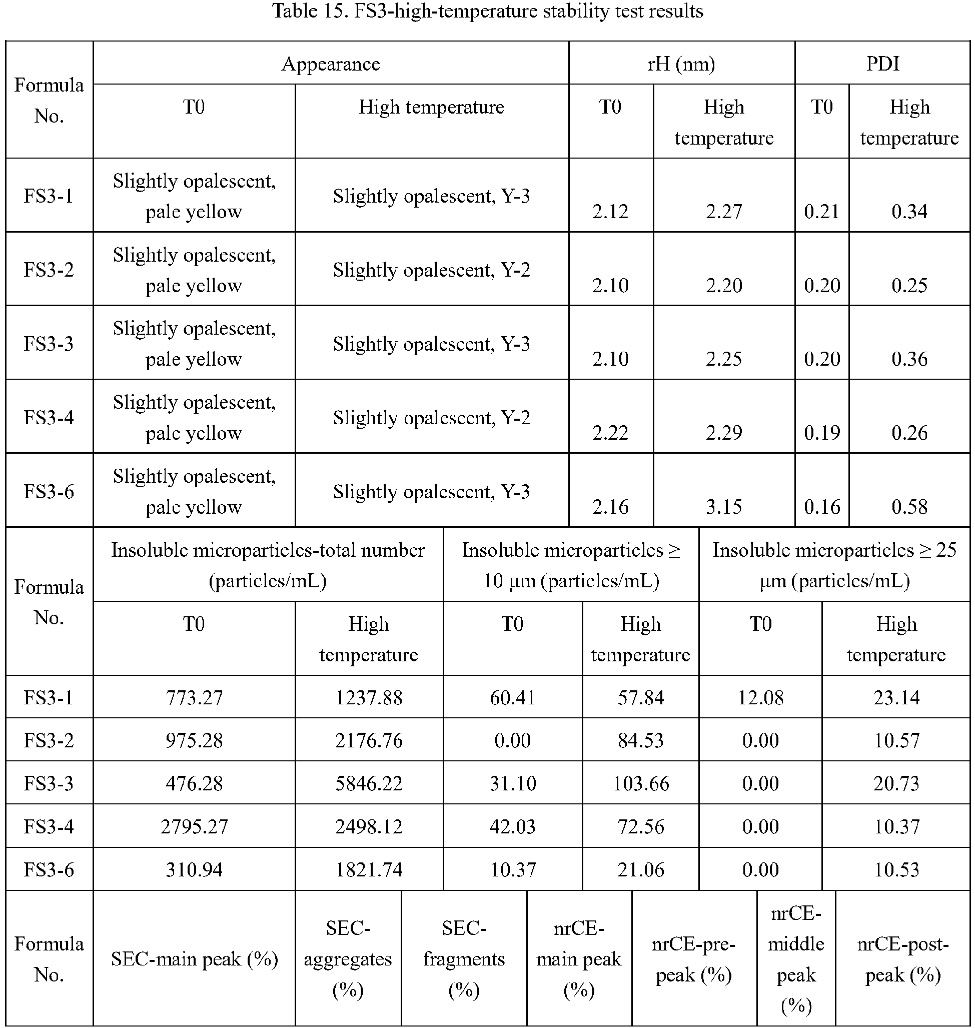

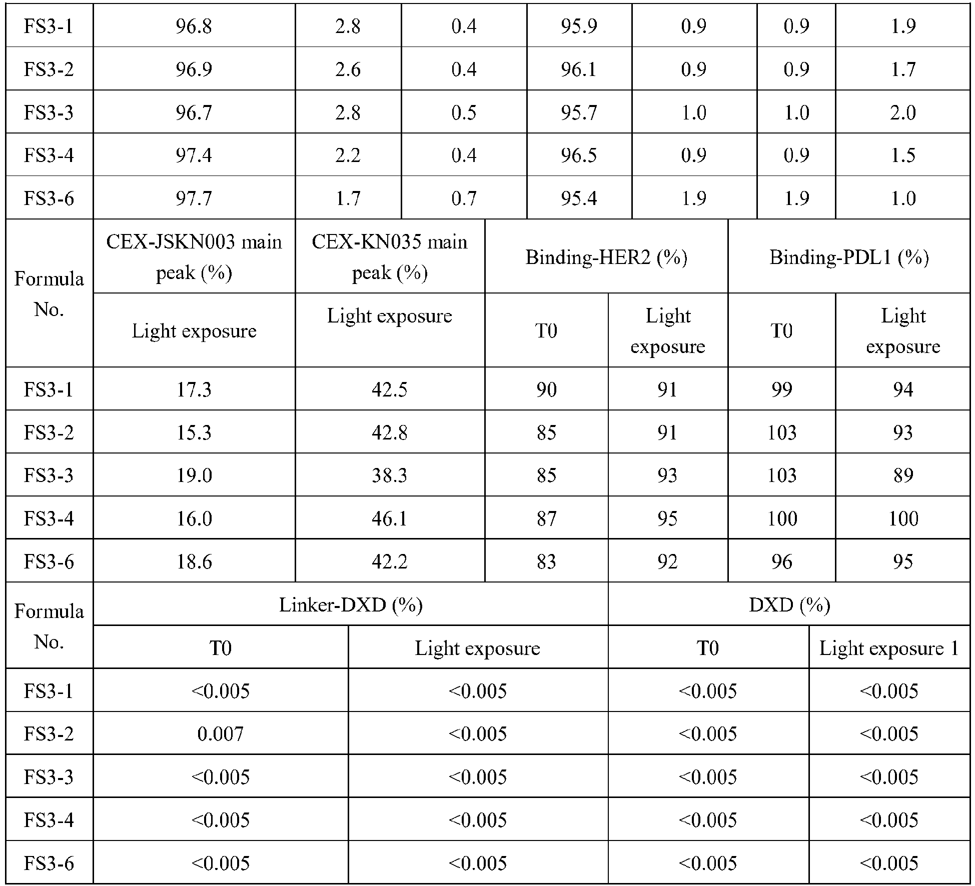

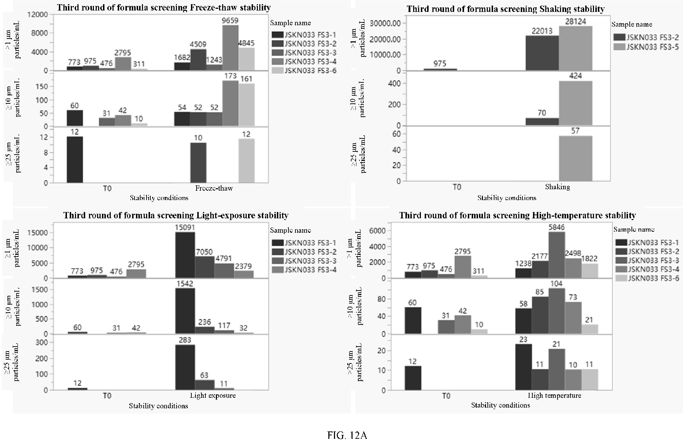

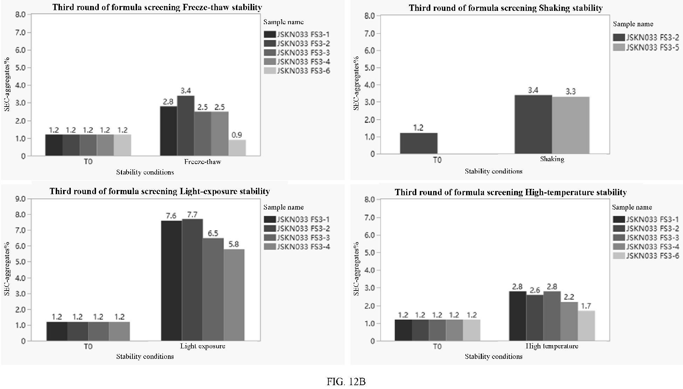

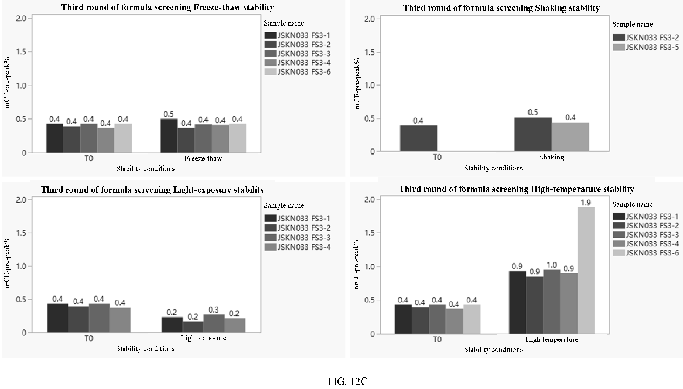

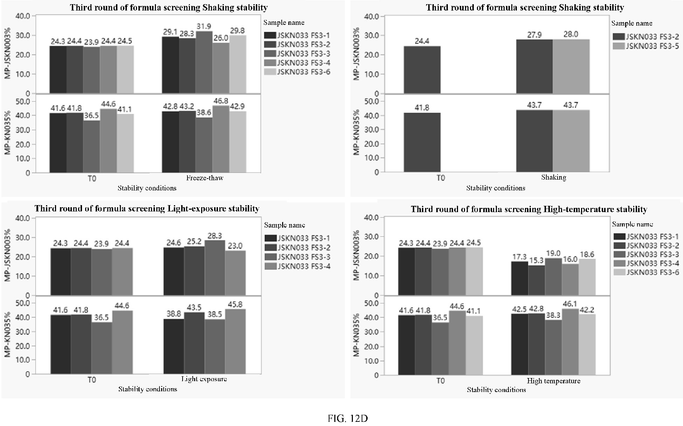

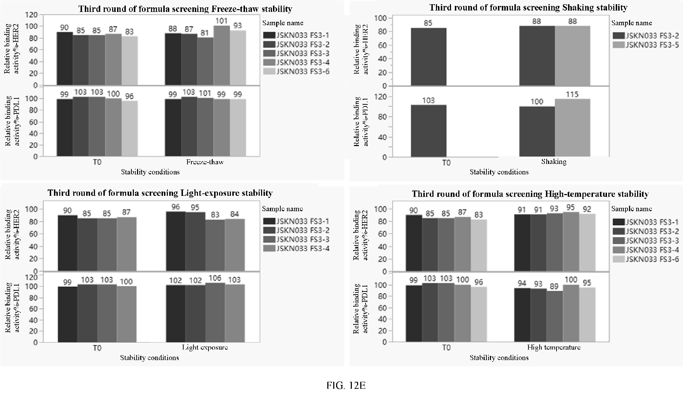

The results for the test items in the different stability tests are summarized in FIGs. 12A-12E. The study showed that FS3-6 had a low SEC-HPLC aggregate content under high-temperature and freeze-thaw conditions. Thus, the preferred pH was 5.5. After being exposed to light, FS3-1 had a significantly increased number of microparticles. Thus, the preferred methionine content was 30 mM. 0.04% polysorbate 80 showed no advantage. Thus, 0.02% polysorbate 80 was selected.

According to the above results, the preferred formula was FS3-6, i.e., 80 mg/mL JSKN003, 100 mg/mL KN035, pH 5.5, 10 mM histidine/histidine hydrochloride (the content of the replacement buffer; the actual histidine content after buffer exchange decreased somewhat; see below), 180 mM proline, 30 mM methionine, and 0.02% polysorbate 80. It exhibited good high-temperature stability, good freeze-thaw stability, and good shaking stability and had a viscosity of <10 cP, which is conducive to subcutaneous injection.

Given that the appropriate osmotic pressure for subcutaneous injection should be about 300 mOsmo/kg, according to calculations, the proline concentration was adjusted to 140 mM. The final preferred mixed formulation (i.e., JSKN033) formula was 80 mg/mL JSKN003 and 100 mg/mL KN035. The formulation formula was as follows: pH 5.5 + 0.5, 10 mM histidine/histidine hydrochloride, 140 mM proline, 30 mM methionine, and 0.02% polysorbate 80. The buffer system may also be formulated by using histidine and adding glacial acetic acid to adjust the pH.

### 3.4. Auxiliary material content confirmation

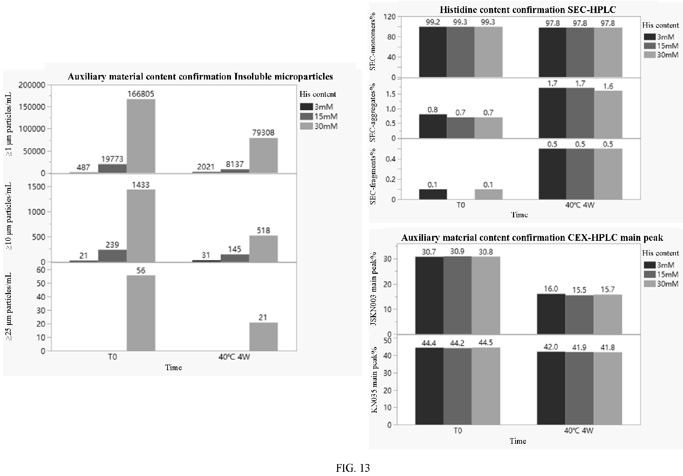

Samples containing 3-30 mM histidine/acetic acid and 140 mM proline were prepared for stability tests. The test results are shown in Table 16 and FIG. 13. The results show that the sample containing 15 mM histidine and the sample containing 30 mM histidine had relatively significant opalescence and an increased number of insoluble microparticles and did not differ in other test items. Thus, it was confirmed that the relatively appropriate histidine content was 3-5 mM (the replacement buffer had about 10 mM), the relatively appropriate proline content was 140-180 mM, and the relatively appropriate buffer system was a histidine/histidine hydrochloride or histidine/acetic acid buffer system.

**Table 16. The appearance test results from the histidine content confirmation experiment**

| **Formula No.** | **Buffer system** | **Appearance** | |
|---|---|---|---|
| | | **T0** | **40°C 4 W** |
| His 3 mM* | 3-30 mM histidine/acetic acid, pH 5.5, 140 mM proline, 30 mM methionine, and 0.02% polysorbate 80 | Least opalescent | Pale yellow Opalescent |
| His 15 mM** | | Opalescent | Pale yellow Opalescent |
| His 30 mM** | | Most opalescent | Pale yellow Opalescent |

| | | | |
|---|---|---|---|
| *: The replacement buffer had 10 mM, and the pH was adjusted with acetic acid. The found histidine content of the sample after buffer exchange was about 3.3 mM. **: An additional amount of buffer was added to the sample after His 3 mM buffer exchange to adjust the histidine content to the target value. | | | |

### 3.5. Long-term stability and accelerated stability of mixed formulation

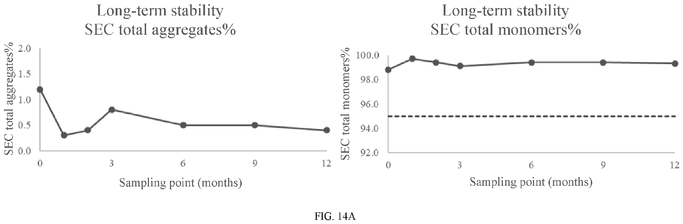

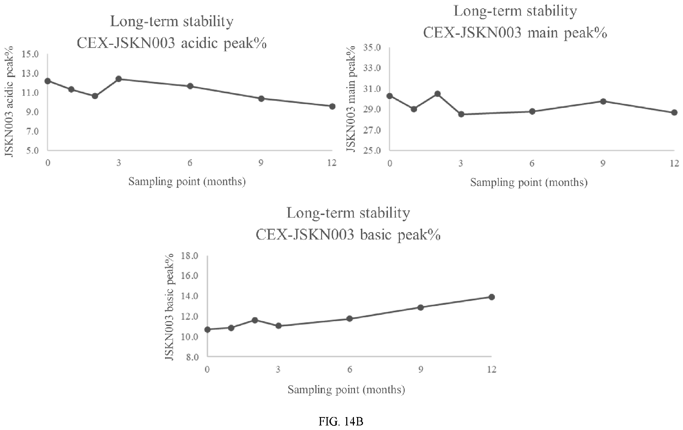

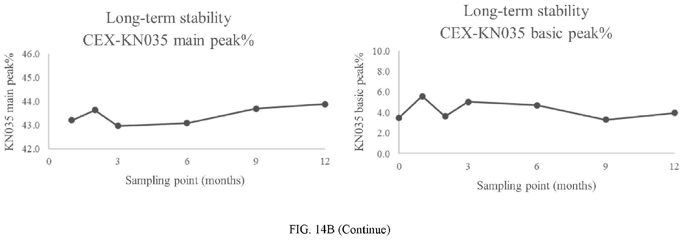

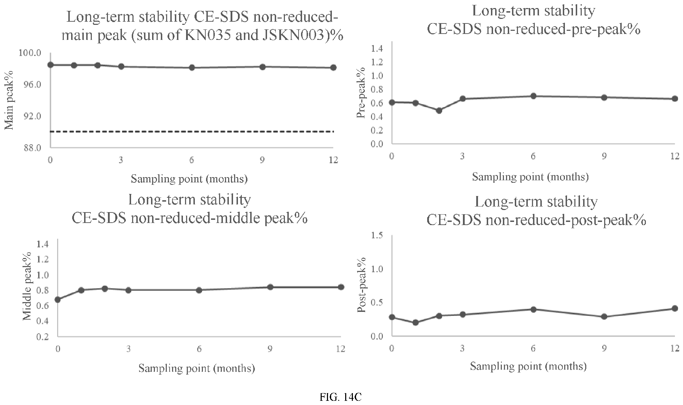

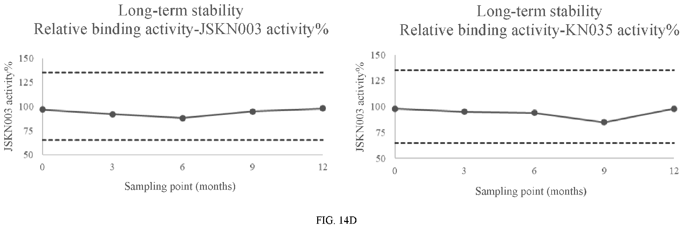

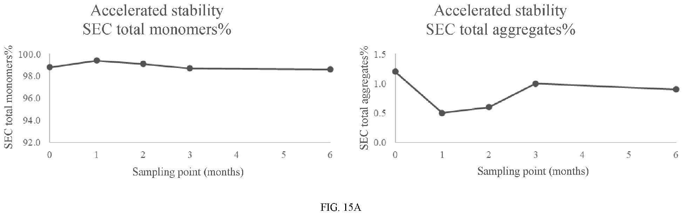

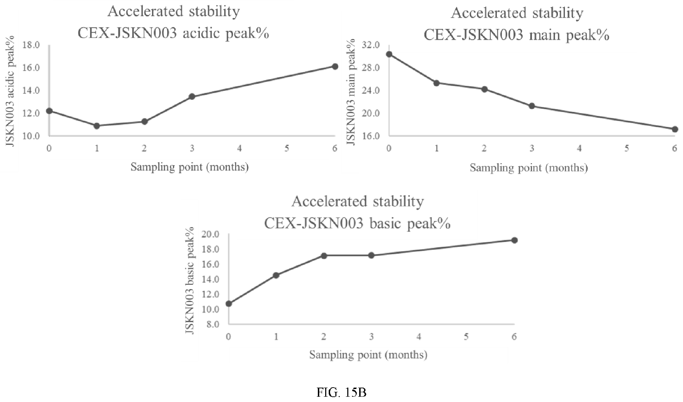

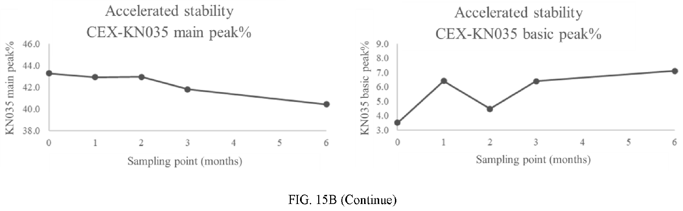

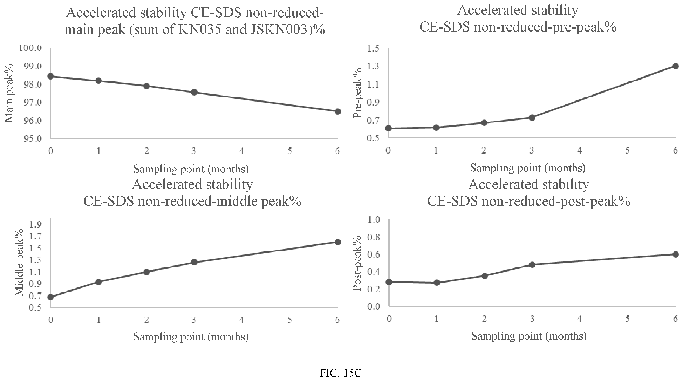

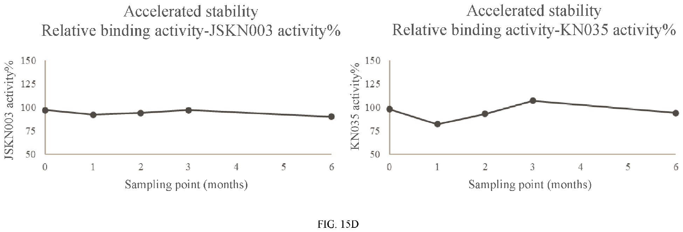

Scale-up production was performed according to the final formulation formula: 10 mM histidine/acetic acid (the histidine content of the replacement buffer was 10 mM, and the histidine content found after buffer exchange was about 4.3 mM), pH 5.5 (the replacement buffer had a pH of 5.0, and the pH after buffer exchange was 5.5), 140 mM proline, 30 mM methionine, and 0.02% polysorbate 80. The long-term stability (2-8 °C, inverted, 12 M) and accelerated stability (25 + 2 °C, RH 60 + 5%, inverted, 6 M) results are shown in FIGs. 14A-14D and FIGs. 15A-15D. The mixed formulation exhibited good long-term stability and good accelerated stability.

### Example 4. Subcutaneous Administration of Mixed Formulation

### 4.1. Pharmacokinetic study of single intravenous or subcutaneous administration to female Bama minipigs

Ten female Bama minipigs were selected and divided into 2 groups of 5. According to the following regimen, JSKN003 was administered by intravenous bolus injection or JSKN033 was administered by subcutaneous injection, and blood samples were collected at specific time points:

| Group | Number of animals | Test article | Target administration level (mg/kg) | Target administration volume (mg/kg) | Target administration concentration (mg/mL) | Vehicle | Route of administra tion | Blood collection time points |
|---|---|---|---|---|---|---|---|---|
| 1 | 5 Female | JSKN003 | 5 | 0.5 | 10 | Normal saline | Intravenou s bolus injection | 0 h (pre-dose), 0.083 h, 0.5 h, 1 h, 2 h, 4 h, 8 h, 12h, 24 h, 48 h, 72 h, 96 h, 144 h, 240 h, 336 h, 504 h, 672 h |
| 2 | 5 Female | JSKN033 | JSKN033 (11.6) JSKN003 (5) | 0.5 | JSKN033 (23.2) JSKN003 (10) | Normal saline | Subcutane ous injection | 0 h (pre-dose), 1 h, 4 h, 8 h, 12 h, 24 h, 48 h, 72 h, 96 h, 144 h, 192 h, 240 h, 288 h, 336 h, 504 h, 672 h |

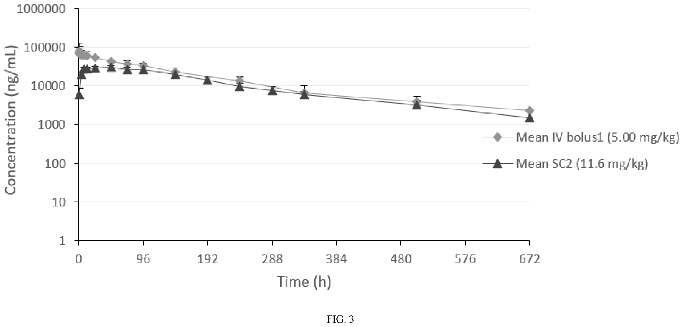

The drug concentration of JSKN003 in the serum of the samples was determined. The results are shown in FIG. 3. After subcutaneous injection of 11.6 mg/kg JSKN033 (equivalent to 5 mg/kg JSKN003), JSKN003 in the combination formulation was absorbed relatively rapidly. The maximum plasma concentration was reached 24-48 h after injection. The mean Cmax and AUC(0-last) values were 30,420 ng/mL and 7,499,765 ng.h/mL, respectively. The half-life following subcutaneous injection was 164 h, similar to that following intravenous bolus injection. The mean bioavailability of JSKN033 following subcutaneous injection was 73.4%.

### 4.2. Study on intermittent subcutaneous injection-induced irritation in rabbits

Eight rabbits were randomly assigned to 2 groups, with 2 females and 2 males in each group. The test article (left side) and the control article (right side) were administered by subcutaneous injection once weekly (days 1 and 8), and the irritation responses on different sides of the dorsal area of the same animal were compared to determine the potential local injection site irritation induced by JSKN033. Different doses (0.25 and 0.5 mL/site) of the test article were injected into the left side of the dorsal area, and the control article (an injectable solution of sodium chloride) was injected into the right side of the dorsal area. The dose concentration was 186.1 mg/mL. At the start of administration, male and female rabbits were approximately 5 months old, with body weights ranging from 3.2 to 3.4 kg for males and 3.1 to 3.3 kg for females. Animals were necropsied on day 11 (one animal/sex/group) and day 15 (another animal/sex/group). Evaluation criteria included mortality, clinical signs, body weight, injection site irritation response scores, and macroscopic and histopathological examination results.

The results showed that there were no unexpected deaths, and no test article-related clinical symptoms or body weight changes either. At the ends of the administration and recovery phases, no macroscopic or histopathological changes were observed.

For the control article (0.25 mL/site), the control article (0.5 mL/site), JSKN033 (0.25 mL/site), and JSKN033 (0.5 mL/site), the daily mean scores of the administration sites during both the administration phase and the recovery phase were 0.

The primary irritation index (PII) was derived from the daily mean administration site scores during the administration phase and the recovery phase. The results showed that during the administration phase and the recovery phase, the PII values of the sites to which the control article (0.25 mL/site), the control article (0.5 mL/site), JSKN033 (0.25 mL/site), and JSKN033 (0.5 mL/site) were administered were all 0, indicating that no irritation was observed at the administration sites in any treatment group.

### Example 5. Phase I/II Clinical Study for Assessing Safety, Tolerability, Pharmacokinetics/Pharmacodynamics, and Efficacy of JSKN033 in Patients with Advanced or Metastatic Solid Malignancies

### ➢ Study objectives and endpoints

Primary objectives and endpoints:

| Phase | Primary objectives | Primary endpoints |
|---|---|---|
| Phase I | • To assess the safety and tolerability of JSKN033 in eligible patients with advanced or metastatic solid malignancies | • Incidence and severity of treatment-emergent adverse events (TEAEs), treatment-related adverse events (TRAEs), and serious adverse events (SAEs) as assessed by NCI CTCAE version 5.0 |
| | • To determine the recommended Phase II dose (RP2D) of JSKN033 | |
| | | • Dose-limiting toxicities (DLTs) |
| Phase II | • To assess the efficacy of JSKN033 [based on objective response rate (ORR)] | • ORR |

Secondary objectives and endpoints:

| Phase | Secondary objectives | Secondary endpoints |
|---|---|---|
| Phase I | • To characterize the pharmacokinetic (PK) profile of JSKN033 | • Pharmacokinetic evaluation: including but not limited to Cmax, Tmax, AUC₀₋ₜ, AUC_{0-∞} (if applicable), t_{1/2}, MRT, Cₛₛ, max, Cₛₛ, min, AUCₛₛ, CL, Vₛₛ, Rac, etc., for JSKN003, total antibody (Tab) and payload of JSKN003, as well as envafolimab |
| | • To assess the immunogenicity of JSKN033 | • Status (positive or negative) and serum titers, as well as neutralizing capacity (if applicable), of anti-JSKN003 antibodies and anti-envafolimab antibodies |
| | • To assess the preliminary efficacy of JSKN033 | • ORR, disease control rate (DCR), time to response (TTR), duration of response (DOR), and progression-free survival (PFS) per Response Evaluation Criteria in Solid Tumors version 1.1 (RECIST v1.1); overall survival (OS) and 12-month OS rate |
| Phase II | • To characterize the pharmacokinetic (PK) profile of JSKN033 | • Pharmacokinetic evaluation: including but not limited to Cmax, Tmax, AUC₀₋ₜ, AUC_{0-∞} (if applicable), t_{1/2}, Cₛₛ, max, Cₛₛ, min, AUCₛₛ, CL, Vₛₛ, Rac, etc., for JSKN003, total antibody (Tab) and payload of JSKN003, as well as envafolimab |
| | • To assess the immunogenicity of JSKN033 | • Status (positive or negative) and serum titers, as well as neutralizing capacity (if applicable), of anti-JSKN003 antibodies and anti-envafolimab antibodies |
| | • To assess the safety of JSKN033 | • Incidence and severity of TEAEs, TRAEs, and serious adverse events (SAEs) as assessed by NCI CTCAE version 5.0 |
| | • To assess the efficacy of JSKN033 (based on other efficacy endpoints) | • DCR, TTR, DOR, PFS, OS, and 12-month OS rate |

### ➢ Study population

The study will enroll HER2-expressing patients with advanced unresectable or metastatic solid malignancies who are intolerant to standard therapy or have had a relapse during or after prior standard systemic therapy, including but not limited to gastric, colorectal, NSCLC, and other solid malignancy types. HER2-expressing is defined as immunohistochemistry (IHC) ≥ 1+. HER2 mutations in NSCLC patients are considered HER2 expression. For the dose expansion phase, only patients with HER2-expressing gastrointestinal tumors will be enrolled.

### ➢ Sample size

Dose escalation phase (Phase I): The sample size will be determined using a "3+3" design.

Dose escalation phase (Phase II): The SMC will select 1-2 dose groups for expansion, with 10 to 30 patients added to each group, to further explore the safety and effectiveness of JSKN033.

### ➢ Study drug

The strength of JSKN033 is 180 mg/1 mL/vial, containing 80 mg of JSKN003 and 100 mg of envafolimab. The route of administration for JSKN033 is subcutaneous injection, administered on D1 of each week, starting at 1.1 mg/kg, followed by 2.3 mg/kg, 4.5 mg/kg, 5.6 mg/kg, and 6.7 mg/kg.

### ➢ Study design

This is an open-label, multicenter, first-in-human, Phase I/II (dose escalation and dose expansion) study intended to assess the safety, tolerability, PK, immunogenicity, and efficacy of JSKN033 in patients with advanced unresectable or metastatic solid malignancies expected to be HER2-expressing (IHC ≥ 1+).

### • Dose escalation phase

The planned dose escalation scheme is as follows:

| Dose escalation level | JSKN033 dose |
|---|---|
| Dosage level 1 | 1.1 mg/kg, SC, QW |
| Dosage level 2 | 2.3 mg/kg, SC, QW |
| Dosage level 3 | 4.5 mg/kg, SC, QW |
| Dosage level 4 | 5.6 mg/kg, SC, QW |
| Dosage level 5 | 6.7 mg/kg, SC, QW |
| SC: subcutaneous injection | |

In the dose escalation phase, a single-patient accelerated dose titration (ADT) will be used for dosage level 1 (1.1 mg/kg, SC, QW) and dosage level 2 (2.3 mg/kg, SC, QW). For dosage level 3 (4.5 mg/kg, SC, QW), dosage level 4 (5.6 mg/kg, SC, QW), and dosage level 5 (6.7 mg/kg, SC, QW), a "3+3" design will be used for enrollment and monitoring, with the aim of determining the MTD/RP2D of JSKN033. The Safety Monitoring Committee (SMC) has the authority to decide on other doses (higher doses, intermediate doses, or lower doses, or other dose intervals) for dose escalation if deemed necessary.

Enrolled patients will be sequentially assigned to the planned dosage levels and be treated with JSKN033 SC QW per protocol to observe the occurrence of treatment-related AEs and dose-limiting toxicities (DLTs). The DLT observation period is 21 days starting from the first dose of JSKN033.

Accelerated dose titration dose escalation: To minimize potential undertreatment exposure in the early phase of dose escalation, the study will enroll only one patient at dosage level 1 and one at dosage level 2. If neither patient experiences a DLT, subsequent patients will be enrolled at dosage level 3 under the "3+3" design. If a patient experiences a DLT at dosage level 1 or dosage level 2, the SMC will decide whether to enroll more patients at that dosage level to assess the safety of JSKN033. When dosage level 2 is confirmed to be safe, dosage level 1 patients can be escalated to dosage level 2. When dosage level 3 is confirmed to be safe, dosage level 1 and dosage level 2 patients can be escalated to dosage level 3.

If a patient receives less than 80% of the planned dose during the DLT observation period, another patient will be enrolled. If a patient cannot receive an adequate dose of JSKN033 due to an adverse event, eligibility for DLT assessment will be determined by the SMC.

### • Dose expansion phase (Phase II)

After or during dose escalation, the SMC will select 1-2 dosage levels for expansion. An additional 10-30 patients with HER2-expressing gastrointestinal tumors will be enrolled at each dosage level to further explore the effectiveness and safety of JSKN033.

### ➢ Inclusion criteria

Patients must meet the following inclusion criteria to participate in the study:
1. Willing and able to provide a written informed consent form (ICF) for the trial.
2. Male or female, aged ≥18 years; willing and able to complete all required study procedures.
3. Eastern Cooperative Oncology Group performance status 0 or 1, with a life expectancy of ≥12 weeks.
4. A pathologically documented advanced/unresectable or metastatic solid malignancy (gastrointestinal tumor for the dose expansion phases) with HER2 expression (IHC ≥ 1+) that is refractory to or intolerant of standard therapy, or for which no effective standard therapy exists. HER2 mutations in NSCLC patients are also considered HER2 expression.
5. A disease measurable at baseline per RECIST 1.1. If target lesions located in previously irradiated areas have been shown to progress, such lesions are considered measurable.
6. Sufficient organ function as assessed within 7 days prior to the first trial [no blood transfusion, erythropoietin (EPO), granulocyte colony-stimulating factor (G-CSF), or other related medical support within 14 days prior to study product administration]:

| System | Laboratory value |
|---|---|
| Hematology | |
| Absolute neutrophil count | ≥1.5 x 10⁹/L |
| Platelets | ≥100 x 10⁹/L |
| Hemoglobin | ≥9 g/dL |

| Kidney | |
|---|---|
| Serum creatinine | ≤1.5 × upper limit of normal (ULN) |
| | or calculated creatinine clearance (CrCL) > 60 mL/min (Cockcroft-Gault formula) |

| Liver | |
|---|---|
| Total bilirubin | ≤1.5 x ULN |
| AST/ALT | ≤3 × ULN or, for patients with liver metastasis, ≤5 × ULN |
| Coagulation | |
| International normalized ratio (INR) or prothrombin time (PT) | ≤1.5 x ULN |
| Activated partial thromboplastin time (APTT) | ≤1.5 x ULN |

7. A sufficient treatment washout period prior to the first dose, defined as:

| Treatment | Washout period |
|---|---|
| Major surgery (except for diagnostic biopsy) | ≥4 weeks |
| Radiotherapy | ≥4 weeks (≥2 weeks for palliative stereotactic radiotherapy) |
| Hormone therapy | ≥1 week |
| Chemotherapy | ≥3 weeks (≥2 weeks for oral 5-fluorouracil and/or single-week paclitaxel chemotherapy) |
| Immunotherapy | ≥4 weeks |
| Other anti-tumor and investigational drugs | ≥4 weeks or 5 times half-life (not less than 2 weeks), whichever is shorter |
| Strong inhibitors of cytochrome P450(CYP)3A4 | ≥5 times inhibitor elimination half-life |

8. LVEF ≥ 50% as measured by echocardiography (ECHO) or multigated acquisition (MUGA) within 28 days prior to the first dose.
9. Female patients of childbearing potential or male patients agree to take highly effective contraceptive measures (with an annual failure rate of less than 1%) from the first dose to 180 days after the end of administration. Female subjects of childbearing potential (defined as premenopausal women without a documented tubal ligation or hysterectomy, or women within 1 year of reaching menopause) must have a negative pregnancy test result within 7 days prior to the first dose.

### ➢ Results

As of August 20, 2024, a total of ten patients had been enrolled (n = 4 with breast cancer, n = 2 with lung cancer, n = 2 with biliary tract cancer, n = 1 with colorectal cancer, and n = 1 with salivary gland cancer). Their HER2 expression/mutation status is shown in the table below. The patients were treated with JSKN033 at doses of 1.1 mg/kg (n = 1), 2.3 mg/kg (n = 1), 4.5 mg/kg (n = 3), 5.6 mg/kg (n = 3), and 6.7 mg/kg (n = 2). The most common treatment-related adverse events (TRAEs) were mild to moderate injection site reactions (grades 1-2). No grade 3 or higher TRAEs or serious adverse events were observed, and no TRAEs led to treatment discontinuation.

| Cancer type (n) | HER2 status | | | |
|---|---|---|---|---|
| | IHC1+ | IHC2+ | IHC3+ | Mutation |
| Breast cancer | 1 | 3 | 0 | 0 |
| Lung cancer | 0 | 0 | 0 | 2 |
| Biliary tract cancer | 0 | 1 | 1 | 0 |
| Colorectal cancer | 0 | 1 | 0 | 0 |
| Salivary gland cancer | 0 | 0 | 1 | 0 |

Eight patients were evaluable for efficacy. Among them, two showed partial responses and four showed stable disease, resulting in a disease control rate of 75%. Two patients treated at a dose of 5.6 mg/kg showed partial responses at the first post-baseline scan. Among them, one had HR-positive/HER2-low (IHC 2+, FISH-) breast cancer and had received at least fourth-line or higher-line treatment, and the other had HER2-mutant non-small cell lung cancer and had experienced disease progression after immunotherapy, chemotherapy, and anti-HER2 therapy.

In this first-in-human trial of JSKN033, the most common treatment-related adverse events (TRAEs) were mild to moderate injection site reactions, indicating a favorable safety profile. In the 5.6 mg/kg dose group, two heavily pretreated patients achieved partial responses, consistent with estimates from clinical pharmacology data. These results encourage further clinical development of JSKN033. In addition, this formulation technique may be applicable to other antibody-drug conjugate (ADC) therapies.

### Example 6. Study on Stability of Mixed Formulations of Other ADCs and KN035

(1) Samples of DS8201 (purchased from Daiichi Sankyo/AstraZeneca, trade name: Enhertu^{®}, product lot No. 397496) and KN035 were subjected to buffer exchange using dialysis cassettes, concentrated using ultrafiltration tubes, and mixed at expected concentrations to formulate the formulas shown in Table 17.

**Table 17. DS8201 mixed formulation formulas**

| Sample name | DS8201 content | KN035 content | Buffer system | Surfactant |
|---|---|---|---|---|
| DS8201-25 | 25 mg/mL | / | 10 mM histidine-acetic acid, pH 5.5 | 0.02% polysorbate 80 |
| DS8201EN-25 | 25 mg/mL | 25 mg/mL | 10 mM histidine-acetic acid, pH 5.5 | 0.02% polysorbate 80 |
| DS8201-50 | 50 mg/mL | / | 10 mM histidine-acetic acid, pH 5.5 | 0.02% polysorbate 80 |
| DS8201EN-50 | 50 mg/mL | 100 mg/mL | 10 mM histidine-acetic acid, pH 5.5 | 0.02% polysorbate 80 |

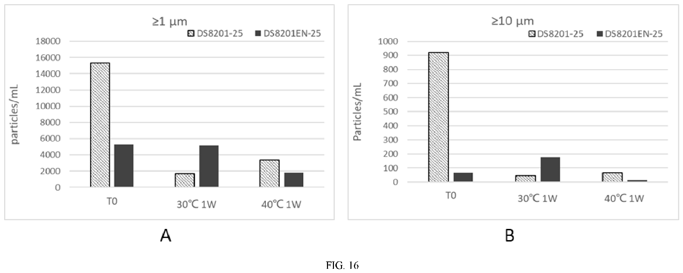

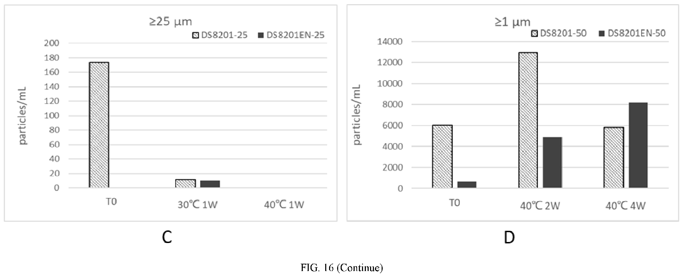

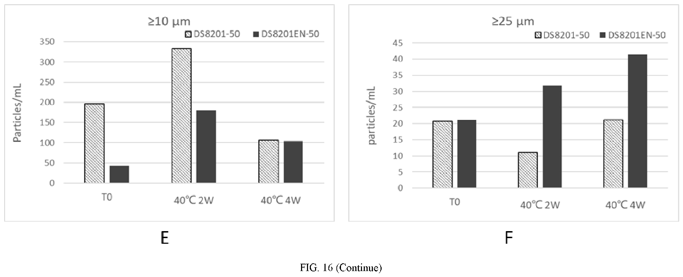

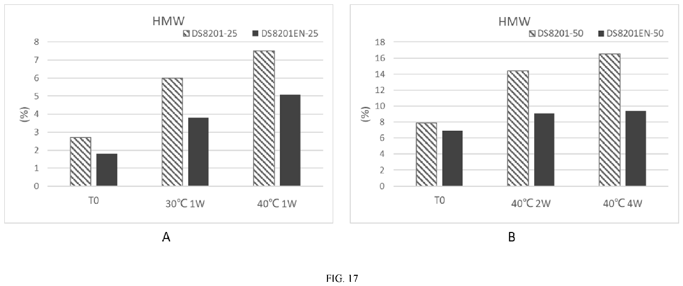

After the preparation was completed, samples were placed in a constant temperature and humidity chamber (40 + 2 °C, 70% RH) for 4 weeks, and the insoluble microparticle content and SEC-HPLC purity of each formula were determined. The results are shown in Table 18 and FIGs. 16 and 17.
The test results show that after DS8201 was mixed with KN035 (different concentrations), the high-temperature stability was good, the samples had no visible particles, there was no significant difference in insoluble microparticle content, and there was a decrease in SEC-HPLC aggregate (HMW) content.

**Table 18. Test results for DS8201 mixed formulations**

| Sample name | Insoluble microparticles (particles/mL) | T0 | 30 °C | 40 °C | SEC-HPLC purity (%) | T0 | 30 °C | 40 °C |
|---|---|---|---|---|---|---|---|---|
| | | | 1 W | 1 W | | | 1 W | 1 W |
| DS8201-25 | ≥1 µm | 15334.62 | 1645.04 | 3377.27 | HMW | 2.7 | 6.0 | 7.5 |
| | ≥10 µm | 921.81 | 45.38 | 66.88 | Main | 97.1 | 93.7 | 92.0 |
| | ≥25 µm | 173.52 | 11.35 | 0.00 | LMW | 0.2 | 0.3 | 0.5 |
| DS8201E N-25 | ≥1 µm | 5291.16 | 5193.19 | 1762.03 | HMW | 1.8 | 3.8 | 5.1 |
| | ≥10 µm | 63.62 | 176.22 | 11.52 | Main | 98.1 | 95.9 | 94.5 |
| | ≥25 µm | 0.00 | 10.37 | 0.00 | LMW | 0.1 | 0.3 | 0.4 |
| | | | | | | | | |

| Sample name | Insoluble microparticles (particles/mL) | T0 | 40 °C | 40 °C | SEC-HPLC purity (%) | T0 | 40 °C | 40 °C |
|---|---|---|---|---|---|---|---|---|
| | | | 2 W | 4 W | | | 2 W | 4 W |
| DS8201-50 | ≥1 µm | 6047.17 | 12926.81 | 5806.09 | HMW | 7.9 | 14.4 | 16.5 |
| | ≥10 µm | 196.95 | 333.17 | 105.96 | Main | 90.9 | 83.2 | 79.8 |
| | ≥25 µm | 20.73 | 11.11 | 21.19 | LMW | 1.2 | 2.4 | 3.8 |
| DS8201E N-50 | ≥1 µm | 657.42 | 4867.01 | 8209.59 | HMW | 6.9 | 9.1 | 9.4 |
| | ≥10 µm | 42.41 | 180.26 | 103.66 | Main | 92.9 | 90.5 | 89.8 |
| | ≥25 µm | 21.21 | 31.81 | 41.46 | LMW | 0.2 | 0.4 | 0.8 |

(2) Samples of RC48 (purchased from Remegen, trade name: Aidixi^{®}, product lot No. RC48-X1-202206016/RC48-X1-202311012) and KN035 were subjected to buffer exchange using dialysis cassettes, concentrated using ultrafiltration tubes, and mixed at expected concentrations to formulate the formulas shown in Table 19.

**Table 19. RC48 mixed formulation formulas**

| Sample name | RC48 content | KN035 content | Buffer system | Surfactant |
|---|---|---|---|---|
| RC48-80 | 80 mg/mL | / | 10 mM histidine-acetic acid, pH 5.5 | 0.02% polysorbate 80 |
| RC48EN-80 | 80 mg/mL | 100 mg/mL | 10 mM histidine-acetic acid, pH 5.5 | 0.02% polysorbate 80 |

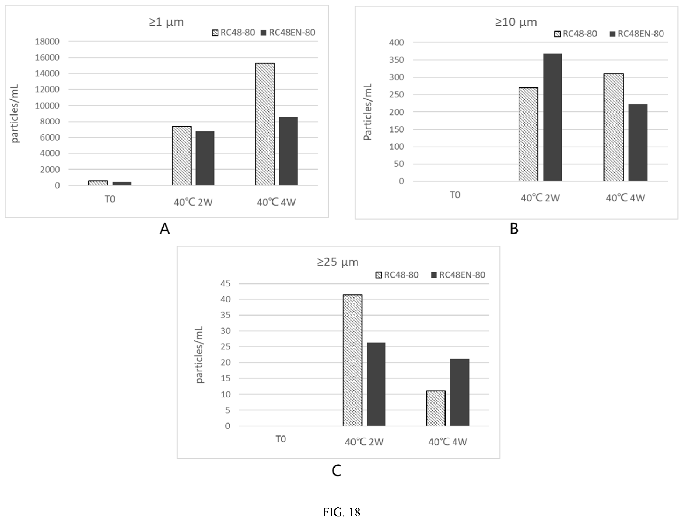

After the preparation was completed, samples were placed in a constant temperature and humidity chamber (40 + 2 °C, 70% RH) for 4 weeks, and the insoluble microparticle content and SEC-HPLC purity of each formula were determined. The results are shown in Table 20 and FIGs. 18 and 19.
The test results show that after RC48 was mixed with KN035 (high concentration), the high-temperature stability was good, the samples had no visible particles, there was no significant difference in insoluble microparticle content, and there was a decrease in SEC-HPLC aggregate (HMW) content.

**Table 20. Test results for RC48 mixed formulations**

| Sample name | Insoluble microparticles (particles/mL) | T0 | 40 °C | 40 °C | SEC-HPLC purity (%) | T0 | 40 °C | 40 °C |
|---|---|---|---|---|---|---|---|---|
| | | | 2 W | 4 W | | | 2 W | 4 W |
| RC48-80 | ≥1 µm | 551.38 | 7411.43 | 15292.28 | HMW | 0.3 | 6.9 | 10.2 |
| | ≥10 µm | 0.00 | 269.51 | 310.95 | Main | 96.7 | 89.0 | 84.9 |
| | ≥25 µm | 0.00 | 41.46 | 11.11 | LMW | 3.0 | 4.1 | 4.1 |
| RC48EN-80 | ≥1 µm | 466.88 | 6744.75 | 8482.81 | HMW | 0.3 | 6.2 | 8.7 |
| | ≥10 µm | 0.00 | 367.42 | 222.67 | Main | 99.5 | 93.4 | 90.7 |
| | ≥25 µm | 0.00 | 26.24 | 21.21 | LMW | 0.2 | 0.4 | 0.6 |

| **Sequence description** | **Sequence No.** | **Specific sequence** |
|---|---|---|
| PD-L1 VHH | SEQ ID NO: 1 | QVQLVESGGGLVQPGGSLRLSCAASGKMSSRRCMAWFRQAPGKE RERVAKLLTTSGSTYLADSVKGRFTISRDNSKNTVYLQMNSLRAED TAVYYCAADSFEDPTCTLVTSSGAFQYWGQGTLVTVSS |
| Fc region | SEQ ID NO: 2 | EPKSCDKTHTCPPCPAPELLGGPSVFLFPPKPKDTLMISRTPEVTCV VVDVSHEDPEVKFNWYVDGVEVHNAKTKPREEQYNSTYRVVSVL TVLHQDWLNGKEYKCKVSNKALPAPIEKTISKAKGQPREPQVYTL PPSRDELTKNQVSLTCLVKGFYPSDIAVEWESNGQPENNYKTTPPV LDSDGSFFLYSKLTVDKSRWQQGNVFSCSVMHEALHNHYTQKSLS LSPGK |
| Fc region | SEQ ID NO: 3 | EPKSSDKTHTCPPCPAPELLGGPSVFLFPPKPKDTLMISRTPEVTCV VVAVSHEDPEVKFNWYVDGVEVHNAKTKPREEQYNSTYRVVSVL TVLHQDWLNGKEYKCKVSNKALPAGIEKTISKAKGQPREPQVYTL PPSRDELTKNQVSLTCLVKGFYPSDIAVEWESNGQPENNYKTTPPV LDSDGSFFLYSKLTVDKSRWQQGNVFSCSVMHEALHNHYTQKSLS LSPGK |
| PD-L1 VHH-Fc | SEQ ID NO: 4 | QVQLVESGGGLVQPGGSLRLSCAASGKMSSRRCMAWFRQAPGKE RERVAKLLTTSGSTYLADSVKGRFTISRDNSKNTVYLQMNSLRAED TAVYYCAADSFEDPTCTLVTSSGAFQYWGQGTLVTVSSEPKSSDKT HTCPPCPAPELLGGPSVFLFPPKPKDTLMISRTPEVTCVVVAVSHED PEVKFNWYVDGVEVHNAKTKPREEQYNSTYRVVSVLTVLHQDWL NGKEYKCKVSNKALPAGIEKTISKAKGQPREPQVYTLPPSRDELTK NQVSLTCLVKGFYPSDIAVEWESNGQPENNYKTTPPVLDSDGSFFL YSKLTVDKSRWQQGNVFSCSVMHEALHNHYTQKSLSLSPGK |
| Kabat CDR1 | SEQ ID NO: 5 | RRCMA |
| Kabat CDR2 | SEQ ID NO: 6 | KLLTTSGSTYLADSVKG |
| Kabat CDR3 | SEQ ID NO: 7 | DSFEDPTCTLVTSSGAFQY |
| AbM CDR1 | SEQ ID NO: 8 | GKMSSRRCMA |
| AbM CDR2 | SEQ ID NO: 9 | KLLTTSGSTY |
| AbM CDR3 | SEQ ID NO: 10 | DSFEDPTCTLVTSSGAFQY |
| Chothia CDR1 | SEQ ID NO: 11 | GKMSSRR |
| Chothia CDR2 | SEQ ID NO: 12 | LTTSGS |
| Chothia CDR3 | SEQ ID NO: 13 | DSFEDPTCTLVTSSGAFQY |
| IMGT CDR1 | SEQ ID NO: 14 | GKMSSRRC |
| IMGT CDR2 | SEQ ID NO: 15 | LLTTSGST |
| IMGT CDR3 | SEQ ID NO: 16 | AADSFEDPTCTLVTSSGAFQY |
| First heavy chain variable region of HER2 bispecific antibody | SEQ ID NO: 17 | |
| Second heavy chain variable region of HER2 bispecific antibody | SEQ ID NO: 18 | |
| Light chain variable region of HER2 bispecific antibody | SEQ ID NO: 19 | |
| First heavy chain of HER2 bispecific antibody | SEQ ID NO: 20 | EVQLVESGGGLVQPGGSLRLSCAASGFTFTDYTMDWVRQAPGKG LEWVADVNPNSGGSIYNQRFKGRFTLSVDRSKNTLYLQMNSLRAE DTAVYYCARNLGPSFYFDYWGQGTLVTVSSASTKGPSVFPLAPSSK STSGGTAALGCLVKDYFPEPVTVSWNSGALTSGVHTFPAVLQSSGL YSLSSVVTVPSSSLGTQTYICNVNHKPSNTKVDKKVEPKSCDKTHT CPPCPAPELLGGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPSRDELTKN QVSLWCLVKGFYPSDIAVEWESNGQPENNYKTTPPVLDSDGSFFLY SALTVDKSRWQQGNVFSCSVMHEALHNHYTQKSLSLSPGK |
| Second heavy chain of HER2 bispecific antibody | SEQ ID NO: 21 | EVQLVESGGGLVQPGGSLRLSCAASGFNIKDTYIHWVRQAPGKGL EWVARIYPTNGYTRYADSVKGRFTISADTSKNTAYLQMNSLRAEDT AVYYCSRWGGDGFYAMDYWGQGTLVTVSSASTKGPSVFPLAPSSK STSGGTAALGCLVKDYFPEPVTVSWNSGALTSGVHTFPAVLQSSGL YSLSSVVTVPSSSLGTQTYICNVNHKPSNTKVDKKVEPKSCDKTHT CPPCPAPELLGGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPSRDELTKN QVSLSCAVKGFYPSDIAVEWESNGQPENNYKTTPPVLDSDGSFKLV SKLTVDKSRWQQGNVFSCSVMHEALHNHYTQKSLSLSPGK |
| Light chain of HER2 bispecific antibody | SEQ ID NO: 22 | DIQMTQSPSSLSASVGDRVTITCRASQDVNTAVAWYQQKPGKAPKL LIYSASFLYSGVPSRFSGSRSGTDFTLTISSLQPEDFATYYCQQHYTT PPTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |
| Kabat CDR1 of first heavy chain variable region of HER2 bispecific antibody | SEQ ID NO: 23 | DYTMD |
| Kabat CDR2 of first heavy chain variable region of HER2 bispecific antibody | SEQ ID NO: 24 | DVNPNSGGSIYNQRFKG |
| Kabat CDR3 of first heavy chain variable region of HER2 bispecific antibody | SEQ ID NO: 25 | NLGPSFYFDY |
| AbM CDR1 of first heavy chain variable region of HER2 bispecific antibody | SEQ ID NO: 26 | GFTFTDYTMD |
| AbM CDR2 of first heavy chain variable region of HER2 bispecific antibody | SEQ ID NO: 27 | DVNPNSGGSI |
| AbM CDR3 of first heavy chain variable region of HER2 bispecific antibody | SEQ ID NO: 28 | NLGPSFYFDY |
| Chothia CDR1 of first heavy chain variable region of HER2 bispecific antibody | SEQ ID NO: 29 | GFTFTDY |
| Chothia CDR2 of first heavy chain variable region of HER2 bispecific antibody | SEQ ID NO: 30 | NPNSGG |
| Chothia CDR3 of first heavy chain variable region of HER2 bispecific antibody | SEQ ID NO: 31 | NLGPSFYFDY |
| IMGT CDR1 of first heavy chain variable region of HER2 bispecific antibody | SEQ ID NO: 32 | GFTFTDYT |
| IMGT CDR2 of first heavy chain variable region of HER2 bispecific antibody | SEQ ID NO: 33 | VNPNSGGS |
| IMGT CDR3 of first heavy chain variable region of HER2 bispecific antibody | SEQ ID NO: 34 | ARNLGPSFYFDY |
| Kabat CDR1 of second heavy chain variable region of HER2 bispecific antibody | SEQ ID NO: 35 | DTYIH |
| Kabat CDR2 of second heavy chain variable region of HER2 bispecific antibody | SEQ ID NO: 36 | RIYPTNGYTRYADSVKG |
| Kabat CDR3 of second heavy chain variable region of HER2 bispecific antibody | SEQ ID NO: 37 | WGGDGFYAMDY |
| AbM CDR1 of second heavy chain variable region of HER2 bispecific antibody | SEQ ID NO: 38 | GFNIKDTYIH |
| AbM CDR2 of second heavy chain variable region of HER2 bispecific antibody | SEQ ID NO: 39 | RIYPTNGYTR |
| AbM CDR3 of second heavy chain variable region of HER2 bispecific antibody | SEQ ID NO: 40 | WGGDGFYAMDY |
| Chothia CDR1 of second heavy chain variable region of HER2 bispecific antibody | SEQ ID NO: 41 | GFNIKDT |
| Chothia CDR2 of second heavy chain variable region of HER2 bispecific antibody | SEQ ID NO: 42 | YPTNGY |
| Chothia CDR3 of second heavy chain variable region of HER2 bispecific antibody | SEQ ID NO: 43 | WGGDGFYAMDY |
| IMGT CDR1 of second heavy chain variable region of HER2 bispecific antibody | SEQ ID NO: 44 | GFNIKDTY |
| IMGT CDR2 of second heavy chain variable region of HER2 bispecific antibody | SEQ ID NO: 45 | IYPTNGYT |
| IMGT CDR3 of second heavy chain variable region of HER2 bispecific antibody | SEQ ID NO: 46 | SRWGGDGFYAMDY |
| Kabat CDR1 of light chain variable region of HER2 bispecific antibody | SEQ ID NO: 47 | RASQDVNTAVA |
| Kabat CDR2 of light chain variable region of HER2 bispecific antibody | SEQ ID NO: 48 | SASFLYS |
| Kabat CDR3 of light chain variable region of HER2 bispecific antibody | SEQ ID NO: 49 | QQHYTTPPT |
| AbM CDR1 of light chain variable region of HER2 bispecific antibody | SEQ ID NO: 50 | RASQDVNTAVA |
| AbM CDR2 of light chain variable region of HER2 bispecific antibody | SEQ ID NO: 51 | SASFLYS |
| AbM CDR3 of light chain variable region of HER2 bispecific antibody | SEQ ID NO: 52 | QQHYTTPPT |
| Chothia CDR1 of light chain variable region of HER2 bispecific antibody | SEQ ID NO: 53 | RASQDVNTAVA |
| Chothia CDR2 of light chain variable region of HER2 bispecific antibody | SEQ ID NO: 54 | SASFLYS |
| Chothia CDR3 of light chain variable region of HER2 bispecific antibody | SEQ ID NO: 55 | QQHYTTPPT |
| IMGT CDR1 of light chain variable region of HER2 bispecific antibody | SEQ ID NO: 56 | QDVNTA |
| IMGT CDR2 of light chain variable region of HER2 bispecific antibody | SEQ ID NO: 57 | SAS |
| IMGT CDR3 of light chain variable region of HER2 bispecific antibody | SEQ ID NO: 58 | QQHYTTPPT |
| GalT1 | SEQ ID NO: 59 | GSNSAAAIGQSSGELRTGGARPPPPLGASSQPRPGGDSSPVVDSGPG PASNLTSVPVPHTTALSLPACPEESPLLVGPMLIEFNMPVDLELVAKQ NPNVKMGGRYAPRDCVSPHKVAIIIPFRNRQEHLKYWLYYLHPVLQ RQQLDYGIYVINQAGDTIFNRAKLLNVGFQEALKDYDYTCFVFSD VDLIPMNDHNAYRCFSQPRHISVAMDKFGFSLPYVQLFGGVSALSK QQFLTINGFPNNYWGWGGEDDDIFNRLVFRGMSISRPNAVVGRCR MIRHSRDKKNEPN PQRFDRIAHTKETMLSDGLNSLTYQVLDVQRYPLYTQITVDIGTPS |
| human GalT1 (Y285L) | SEQ ID NO: 60 | MRLREPLLSGSAAMPGASLQRACRLLVAVCALHLGVTLVYYLAGR DLSRLPQLVGVSTPLQGGSNSAAAIGQSSGELRTGGARPPPPLGASS QPRPGGDSSPVVDSGPGPASNLTSVPVPHTTALSLPACPEESPLLVGP MLIEFNMPVDLELVAKQNPNVKMGGRYAPRDCVSPHKVAIIIPFRN RQEHLKYWLYYLHPVLQRQQLDYGIYVINQAGDTIFNRAKLLNVG FQEALKDYDYTCFVFSDVDLIPMNDHNAYRCFSQPRHISVAMDKF GFSLPYVQLFGGVSALSKQQFLTINGFPNNYWGWGGEDDDIFNRL VFRGMSISRPNAVVGRCRMIRHSRDKKNEPNPQRFDRIAHTKETML SDGLNSLTYQVLDVQRYPLYTQITVDIGTPS |
| bovine GalT1 (Y289L) | SEQ ID NO: 61 | MKFREPLLGGSAAMPGASLQRACRLLVAVCALHLGVTLVYYLAGR DLRRLPQLVGVHPPLQGSSHGAAAIGQPSGELRLRGVAPPPPLQNSS KPRSRAPSNLDAYSHPGPGPGPGSNLTSAPVPSTTTRSLTACPEESPL LVGPMLIEFNIPVDLKLVEQQNPKVKLGGRYTPMDCISPHKVAIIIPF RNRQEHLKYWLYYLHPILQRQQLDYGIYVINQAGESMFNRAKLLN VGFKEALKDYDYNCFVFSDVDLIPMNDHNTYRCFSQPRHISVAMD KFGFSLPYVQLFGGVSALSKQQFLSINGFPNNYWGWGGEDDDIYN RLAFRGMSVSRPNAVIGKCRMIRHSRDKKNEPNPQRFDRIAHTKET MLSDGLNSLTYMVLEVQRYPLYTKITVDIGTPS |

## Claims

1. Use of a programmed death ligand 1 (PD-L1)-binding molecule in improving the stability of a protein-drug conjugate formulation.

2. The use according to claim 1, wherein the formulation is a solid formulation, preferably a freeze-dried formulation.

3. The use according to claim 1, wherein the formulation is a liquid formulation, preferably an injectable solution.

4. The use according to claim 3, wherein the PD-L1-binding molecule is capable of reducing the amount of insoluble microparticles generated in the formulation.

5. The use according to claim 3 or 4, wherein the PD-L1-binding molecule is capable of reducing the amount of aggregates generated in the formulation.

6. The use according to any one of claims 1-5, wherein the PD-L1-binding molecule comprises an immunoglobulin single variable domain, and the immunoglobulin single variable domain comprises a CDR1, a CDR2, and a CDR3 from the VHH set forth in SEQ ID NO: 1.

7. The use according to claim 6, wherein the CDRs may be Kabat CDRs, AbM CDRs, Chothia CDRs, or IMGT CDRs.

8. The use according to claim 7, wherein the CDR1, the CDR2, and the CDR3 from the VHH set forth in SEQ ID NO: 1 are selected from any one of the following groups: SEQ ID NOs: 5-7, SEQ ID NOs: 8-10, SEQ ID NOs: 11-13, and SEQ ID NOs: 14-16.

9. The use according to any one of claims 1-8, wherein the immunoglobulin single variable domain comprises the amino acid sequence set forth in SEQ ID NO: 1.

10. The use according to any one of claims 6-9, wherein the PD-L1-binding molecule further comprises an immunoglobulin Fc region.

11. The use according to claim 10, wherein the immunoglobulin Fc region is a human immunoglobulin Fc region, preferably, an Fc region of human IgG1, human IgG2, human IgG3, or human IgG4, or a variant thereof.

12. The use according to claim 11, wherein the amino acid sequence of the immunoglobulin Fc region is set forth in SEQ ID NO: 2 or SEQ ID NO: 3.

13. The use according to any one of claims 10-12, wherein the immunoglobulin single variable domain is located at the N-terminus of the immunoglobulin Fc region, and the two are linked directly or by a peptide chain selected from one or more of the following amino acid sequences: (GS)ₙ, (GG)ₙ, (GGS)ₙ, (GGGS)ₙ, (GGSG)ₙ, (GGGGS)ₙ, and (GAP)ₙ, wherein n is an integer from 1 to 10.

14. The use according to any one of claims 1-13, wherein the amino acid sequence of the PD-L1-binding molecule is set forth in SEQ ID NO: 4.

15. The use according to any one of claims 1-14, wherein the protein-drug conjugate has the structure of formula I:
P-(L-D)n (I)
wherein P is a protein, L is a linking unit, D is a substance having biological activity, and n is an integer from 1 to 20.

16. The use according to claim 15, wherein P is an antibody.

17. The use according to claim 16, wherein the antibody targets one or more of the following antigens: 4-1BB, 4-1BBL, A33, adenosine A2a receptor, Akt, ALK, androgen receptor, Ang-1, Ang-2, Annexin A3, Aurora A, Aurora B, B7-H3, B7-H4, Bcl-2, Ber-Abl, BRAF, BTK, BTLA, BTN2A1, CA-125, CAIX, CCR4, CD105/endoglin, CD109, CD123, CD155, CD16, CD160, CD19, CD20, CD200, CD200R, CD22, CD24, CD25, CD27, CD28, CD30, CD33, CD36, CD37, CD38, CD40, CD40L, CD47, CD48, CD52, CD70, CD79b, CD80, CD86, CD96, CDK4, CDK6, CDK9, CEA, CEACAM1, ChK1, ChK2, c-KIT, c-Met, COX2, CSF-1R, CSF2, CTLA-4, CXCR2, CXCR4, DDR2, DLL3, DLL4, DNAM-1, DR5, EGFR, EpCAM, EPHA3, EphB4, ERK1, ERK2/p38 MAPK, FAK, FAP, FGF-2, FGFR1, FGFR2, FGFR3, FGFR4, Flt-3, Gal-9, GITR, GITRL, Glypican-3, HDAC1, HDAC2, HDAC3, HDAC4, HDAC5, HDAC7, HDAC8, HDAC9, HER2, HER3, HER4/ERBB4, HGF, HHLA2, HIF-1α, HSP27, HSP90, HVEM, ICOS, ICOS Ligand, IDO, IGF1R, IL-13, IL-6, JAK1, JAK2, JAK3, KRAS, LAG-3, LIGHT, MDM2, MEK1, MEK2, MMP-1, MMP-10, MMP-11, MMP-13, MMP-2, MMP-7, MMP-9, mTOR, Mucin 1, Myc, NF-κB, NKG2A, NRAS, NTRK1, NTRK2, NTRK3, OX40, OX40L, p53, PAF, PARP1, PARP2, PD1, PDGFR-α, PDGFR-β, PD-L1, PD-L2, PI3Kα, PI3Kβ, PI3Kγ, PI3Kδ, PIM1, PIM3, PSMA, PTEN, RAF-1, RANKL, RET, S100A4, SIRPα, SLAMF7, SMO, Src, STAT3, STEAP-1, SYK, TDO, TGFβ, Tie-2, TIGIT, TIM-3, TLR8, TMIGD2, TNF-α, Toll-like receptor 3, TRAIL, TRAILR1, TROP-2, VEGF, VEGF-C, VEGFR-1, VEGFR-2, VEGFR-3, and VISTA.

18. The use according to any one of claims 15-17, wherein the linking unit L has the structure represented by formula II:
-L1-sp1-L2-sp2- (II)
wherein L1 is a linker for linking to P, sp1 is a first spacing unit, L2 is a cleavable linker, and sp2 is a second spacing unit and is linked to D.

19. The use according to any one of claims 15-17, wherein the linking unit L has the structure represented by formula II:
-L1-sp1-L2-sp2- (II)
wherein L1 is a linker for linking to P, sp1 is a first spacing unit, L2 is absent, and sp2 is a second spacing unit and is linked to D.

20. The use according to claim 18 or 19, wherein L1 is selected from: 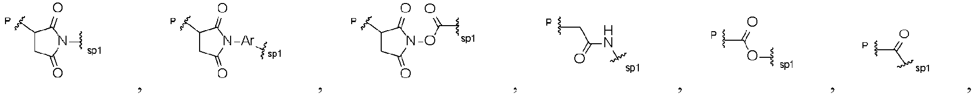 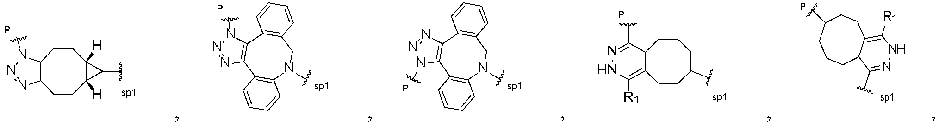 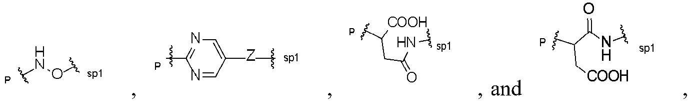 wherein Ar represents C₆₋₁₀ arylene optionally substituted with halogen or C₁₋₆ alkyl; R₁ is selected from hydrogen, halogen, and C₁₋₆ alkyl; Z is selected from a direct bond, C₂₋₆ alkynylene, C₂₋₆ alkenylene, C₆₋₁₀ arylene, 5-10 membered heteroarylene, amido, sulfonamido, imino, and CF₂.

21. The use according to any one of claims 18-20, wherein the structure of sp1 is: 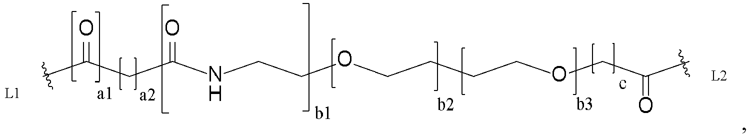 wherein a1 = 0 or 1, a2 = an integer from 0 to 6, b1 = 0 or 1, b2 = an integer from 0 to 16, b3 = an integer from 0 to 16, and c = an integer from 0 to 6, and at least one of b2 and b3 is 0.

22. The use according to claim 21, wherein:
(1) a1 = 0, a2 = 2, 3, 4, 5, or 6, b1 = 0, b2 = 0, b3 = 0, and c = 0;
(2) a1 = 0, a2 = 0, b1 = 0, b2 = 0, b3 = 0, and c = 2, 3, 4, 5, or 6;
(3) a1 = 1, a2 = 2, 3, 4, 5, or 6, b1 = 1, b2 = 2, 3, 4, 5, 6, 7, or 8, b3 = 0, and c = 0;
(4) a1 = 0, a2 = 2, 3, 4, 5, or 6, b1 = 1, b2 = 2, 3, 4, 5, 6, 7, or 8, b3 = 0, and c = 0; or
(5) a1 = 1, a2 = 0, b1 = 0, b2 = 0, b3 = 2, 3, 4, 5, 6, 7, or 8, and c = 2, 3, 4, 5, or 6.

23. The use according to any one of claims 18 and 20-22, wherein L2 is a dipeptide, tripeptide, or tetrapeptide residue.

24. The use according to claim 23, wherein L2 is selected from the following dipeptide residues: -Phe-Lys-, -Val-Ala-, -Val-Lys-, -Val-Cit-, -Ala-Lys-, -Phe-Cit-, -Leu-Cit-, -Ile-Cit-, -Phe-Arg-, -Trp-Cit-, -Gly-Gly-, -Ala-Ala-, -Gly-Val-, and -Gly-Glu-; the left side of the dipeptide residue is linked to sp1, and the right side thereof is linked to sp2.

25. The use according to claim 23, wherein L2 is selected from the following tripeptide residues: -Glu-Val-Ala-, - Glu-Val-Cit-, -αGlu-Val-Ala-, -αGlu-Val-Cit-, -Val-Lys-Gly, and -Val-Cit-Gly-; the left side of the tripeptide residue is linked to sp1, and the right side thereof is linked to sp2.

26. The use according to claim 23, wherein L2 is selected from the following tetrapeptide residues: -Gly-Gly-Phe-Gly- and -Gly-Phe-Gly-Gly-; the left side of the tetrapeptide residue is linked to sp1, and the right side thereof is linked to sp2.

27. The use according to any one of claims 18-26, wherein sp2 is absent, or sp2 is selected from: 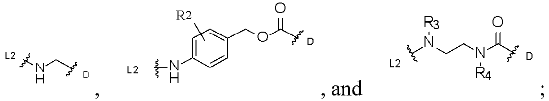
R₂ is independently selected from hydrogen, C₁₋₆ alkyl, hydroxy, amino, halogen, nitro, cyano, 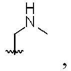 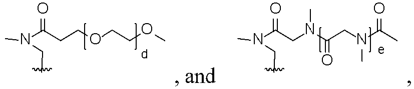 d is an integer from 1 to 20, and e is an integer from 1 to 20; R₃ and R₄ are each independently selected from hydrogen and C₁₋₆ alkyl;
the alkyl may be optionally substituted with hydroxy, amino, halogen, nitro, and cyano.

28. The use according to any one of claims 15-27, wherein the linking unit L is selected from the following structures: 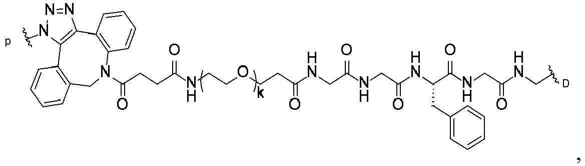 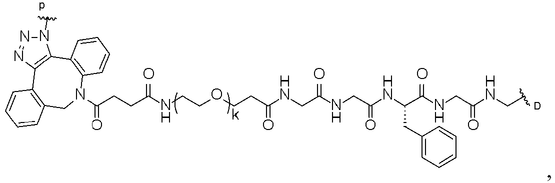 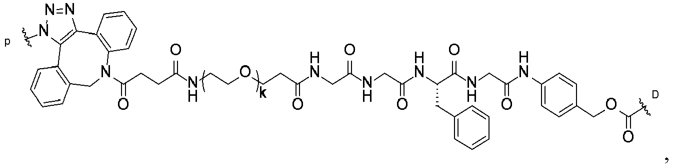 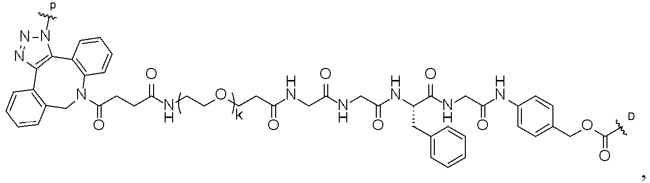 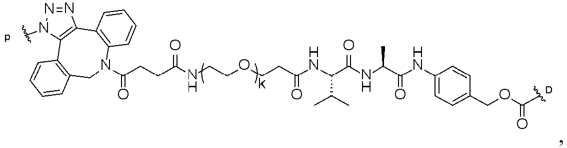 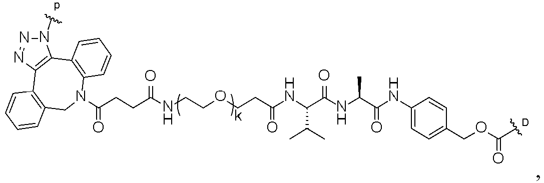 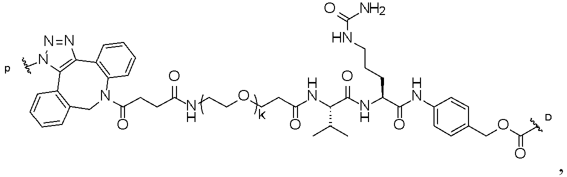 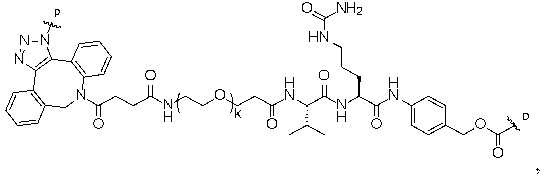 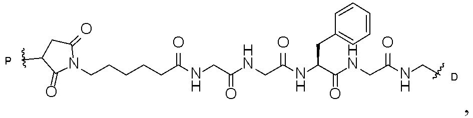 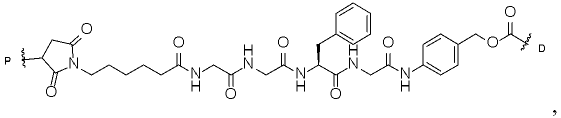 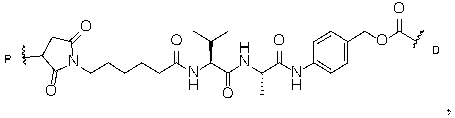 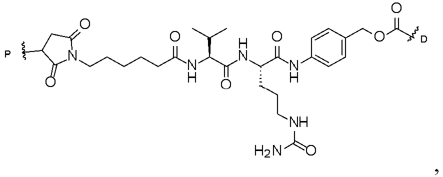 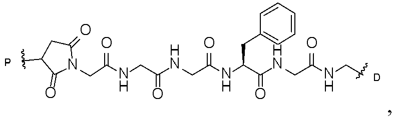 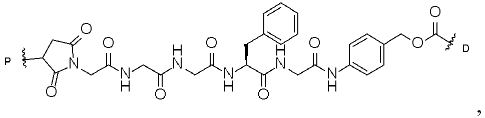 and 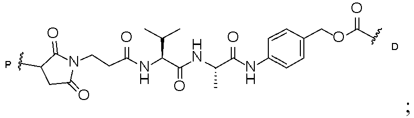 k is an integer from 1 to 20, e.g., 2, 3, 4, 5, 6, 7, 8, 9, or 10.

29. The use according to any one of claims 15-28, wherein D is selected from a cytotoxin, a protein kinase inhibitor, an immune agonist, a glucocorticoid, an oligonucleotide, a radioisotope, a polypeptide, and any combination thereof.

30. The use according to claim 29, wherein D is a cytotoxin selected from a DNA alkylating agent, a DNA deconstructing agent, a topoisomerase I inhibitor, a topoisomerase II inhibitor, a microtubule inhibitor, a ribosome inhibitor, and any combination thereof.

31. The use according to claim 30, wherein D is selected from an auristatin derivative, a maitansine derivative, an eribulin derivative, a tubulysin derivative, a pyrrolobenzodiazepine (PDB) derivative, a duocarmycin derivative, a calicheamicin derivative, PNU-159682 and a derivative thereof, a camptothecin derivative, an amatoxin derivative, and any combination thereof.

32. The use according to claim 30, wherein D has the structure represented by formula III: 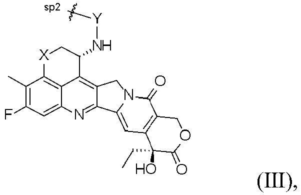
wherein X is selected from CH₂, NH, O, S, and SO₂;
Y is absent, or Y has the structure represented by 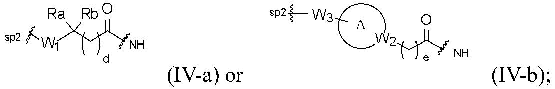
W₁ and W₃ are each independently selected from O, S, and NH, and W₂ is selected from C, CH, and N;
Rₐ and R_{b} are each independently selected from hydrogen, deuterium, halogen, C₁₋₆ alkyl, C₁₋₆ alkoxy, C₃₋₆ cycloalkyl, 3-6 membered heterocycloalkyl, hydroxy, amino, cyano, and nitro; or, Rₐ and R_{b}, together with the carbon atom to which they are attached, form 3-6 membered cycloalkyl, 3-6 membered heterocycloalkyl, or carbonyl; or, Rₐ is linked to the N atom of the amide moiety to form 3-6 membered heterocycloalkyl, and R_{b} is hydrogen;
ring A is selected from the following groups: 5-10 membered cycloalkylene, 5-10 membered heterocycloalkylene, 6-10 membered arylene, and 5-10 membered heteroarylene;
the alkyl, alkoxy, cycloalkyl, heterocycloalkyl, cycloalkylene, heterocycloalkylene, arylene, and heteroarylene are each independently, optionally, and further substituted with a group selected from deuterium, halogen, hydroxy, amino, cyano, carbonyl, and nitro;
d and e are each independently selected from integers from 0 to 5.

33. The use according to claim 32, wherein D has the structure represented by formula III-a: 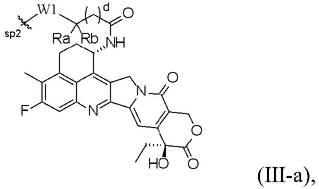
wherein W₁ is O or NH;
Rₐ and R_{b} are each independently selected from hydrogen, deuterium, halogen, C₁₋₆ alkyl, C₁₋₆ alkoxy, C₁₋₆ cycloalkyl, 3-6 membered heterocycloalkyl, hydroxy, amino, cyano, and nitro; or, Rₐ and R_{b}, together with the carbon atom to which they are attached, form 3-6 membered cycloalkyl, 3-6 membered heterocycloalkyl, or carbonyl; or, Rₐ is linked to the N atom of the amide moiety to form 3-6 membered heterocycloalkyl, and R_{b} is hydrogen;
the alkyl, alkoxy, cycloalkyl, and heterocycloalkyl are each independently, optionally, and further substituted with a group selected from deuterium, halogen, hydroxy, amino, cyano, and nitro;
d is an integer from 0 to 5.

34. The use according to claim 33, wherein W₁ is O.

35. The use according to claim 33 or 34, wherein d is 0, 1, or 2.

36. The use according to any one of claims 33-35, wherein Rₐ is selected from hydrogen, deuterium, halogen, C₁₋₆ alkyl, C₁₋₆ alkoxy, C₁₋₆ cycloalkyl, 3-6 membered heterocycloalkyl, hydroxy, and amino, and R_{b} is hydrogen.

37. The use according to any one of claims 33-35, wherein Rₐ and R_{b}, together with the carbon atom to which they are attached, form 3-6 membered cycloalkyl or 3-6 membered heterocycloalkyl.

38. The use according to claim 33, wherein D is selected from the following groups:

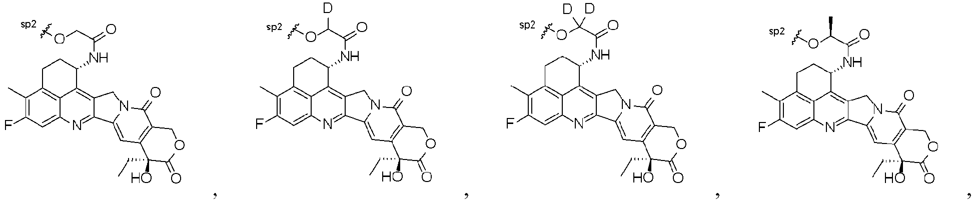

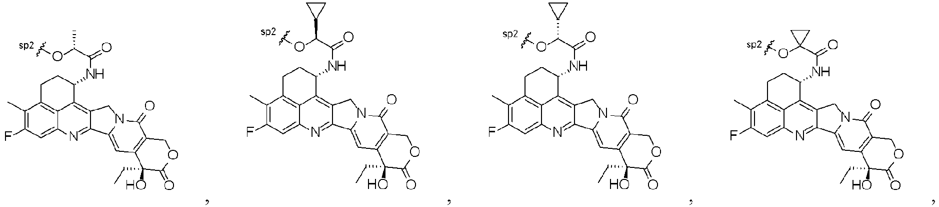

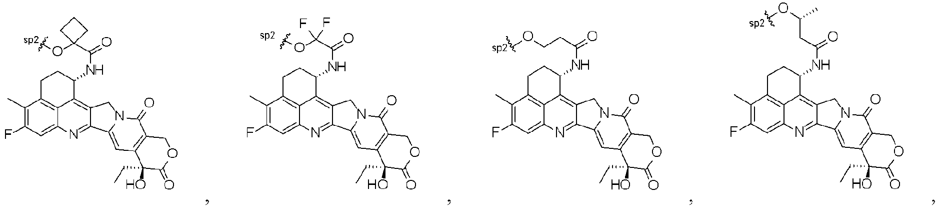

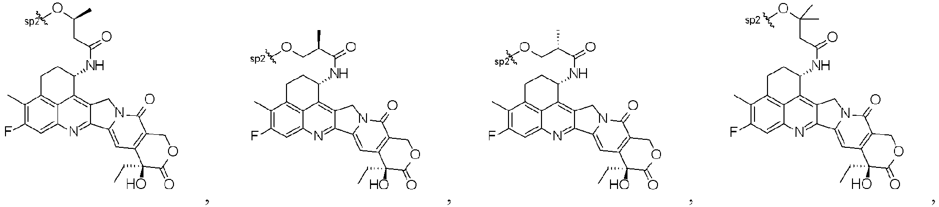

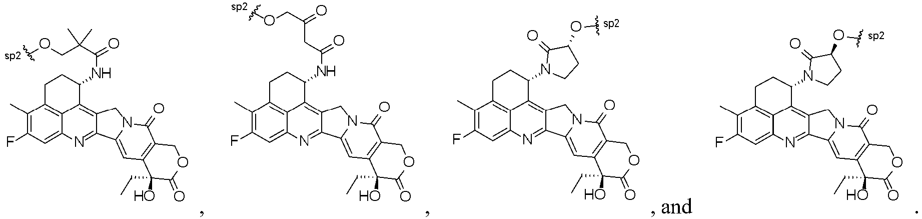

39. The use according to claim 32, wherein D has the structure represented by formula III-b: 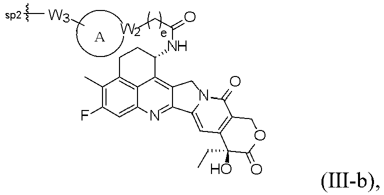
wherein W₃ is O or NH, and W₂ is C, CH, or N;
ring A is selected from the following groups: 5-10 membered cycloalkylene, 5-10 membered heterocycloalkylene, 6-10 membered arylene, and 5-10 membered heteroarylene;
the cycloalkylene, heterocycloalkylene, arylene, and heteroarylene are each independently, optionally, and further substituted with a group selected from deuterium, halogen, hydroxy, amino, cyano, carbonyl, and nitro;
e is an integer from 0 to 5.

40. The use according to claim 39, wherein W₃ is O.

41. The use according to claim 39 or 40, wherein W₂ is CH.

42. The use according to any one of claims 39-41, wherein e is 0, 1, or 2.

43. The use according to any one of claims 39-42, wherein ring A is 5-10 membered cycloalkylene.

44. The use according to claim 39, wherein D is selected from the following groups:

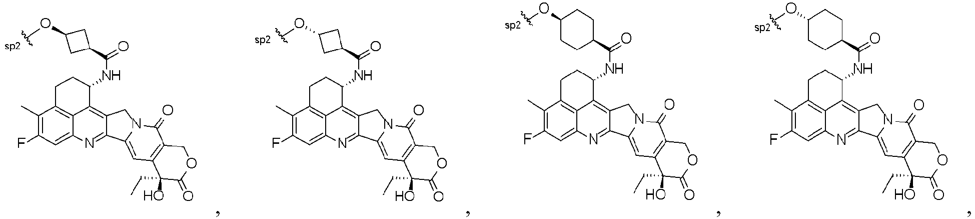

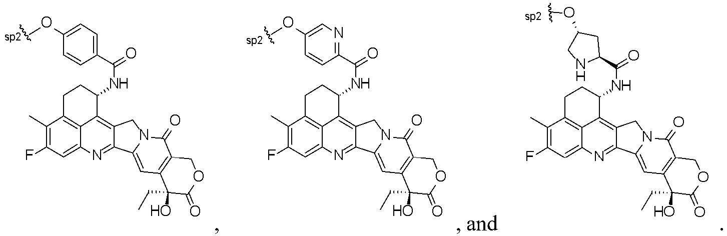

45. The use according to claim 31, wherein D is a camptothecin derivative and is preferably selected from the following structures:

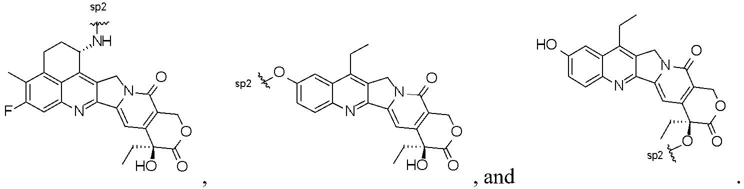

46. The use according to claim 31, wherein D is an auristatin derivative and is preferably selected from the following structures: 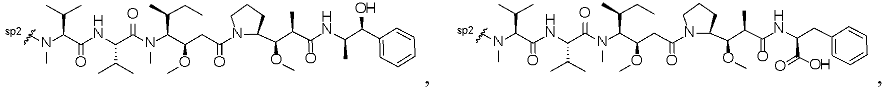 and

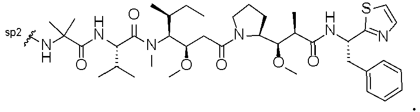

47. The use according to claim 31, wherein D is a maitansine derivative and is preferably selected from the following structures:

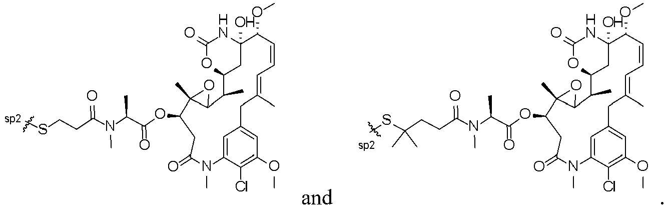

48. The use according to claim 31, wherein D is eribulin and a derivative thereof and is preferably selected from the following structures:

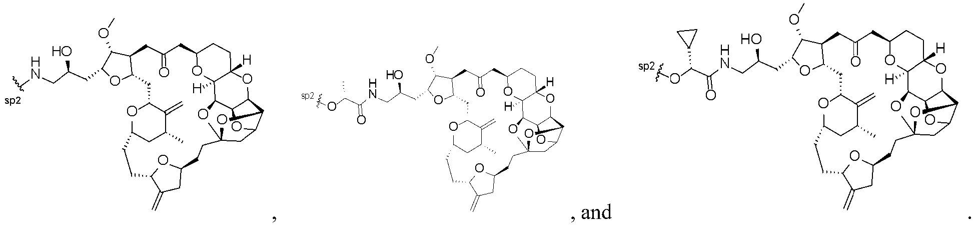

49. The use according to any one of claims 17-48, wherein the linking unit L is linked to the antibody by sulfhydryl, and the sulfhydryl is obtained by reducing a disulfide bond between heavy chains and/or a disulfide bond between a heavy chain and a light chain.

50. The use according to any one of claims 17-48, wherein the linking unit L is linked to the antibody by an oligosaccharide.

51. The use according to claim 50, wherein the oligosaccharide has the structure represented by formula V-a or formula V-b: 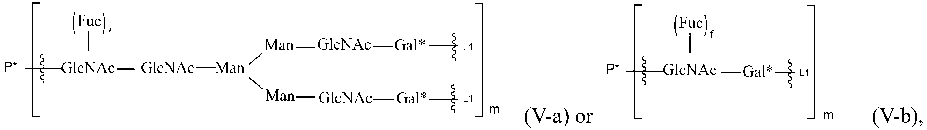
wherein P* is an antibody, e.g., the antibody defined in claim 17, GlcNAc is N-acetylglucosamine, Fuc is fucose, Man is mannose, f is 0 or 1, and m is 1 to 20;
Gal* is a modified galactose selected from the following structures: 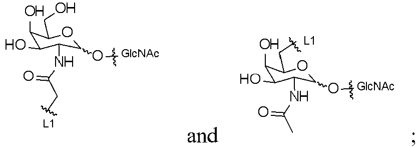
the oligosaccharide is linked to the antibody by the core GlcNAc.

52. The use according to claim 51, wherein the modified galactose is linked to GlcNAc by a β-1,4-glycosidic bond.

53. The use according to any one of claims 50-52, wherein the oligosaccharide is linked to the Fc fragment of the antibody, preferably to the CH2 domain of the Fc fragment, and more preferably to Asn297 (numbered according to the EU index of Kabat) of the Fc fragment.

54. The use according to any one of claims 1-53, wherein the protein-drug conjugate has the structure represented by formula VI: 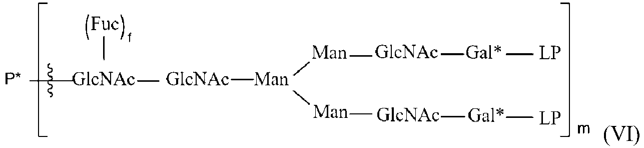
wherein P* is an antibody, e.g., the antibody defined in claim 17, GlcNAc is N-acetylglucosamine, Fuc is fucose, Man is mannose, f is 0 or 1, and m is 1 to 10;
Gal* is a modified galactose selected from the following structures: 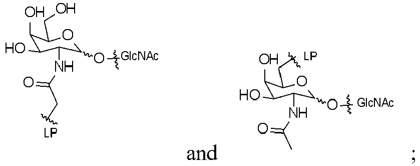
LP is selected from one of the following structures (a) to (g):
(a) 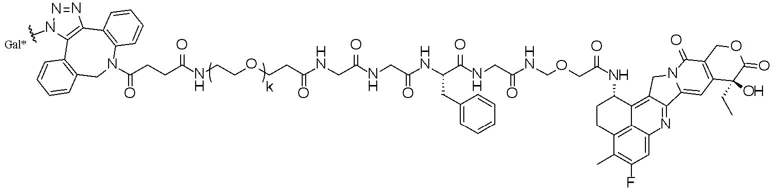 and/or 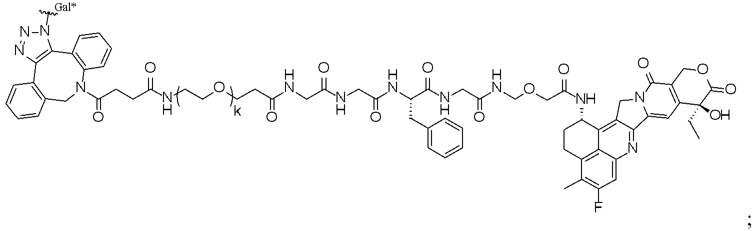
(b) 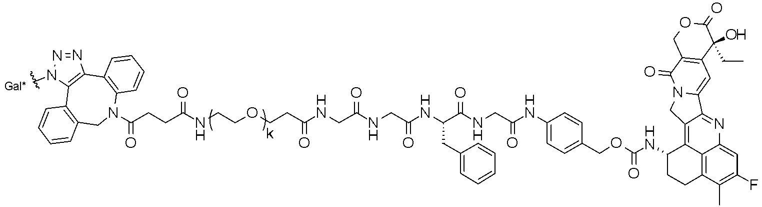 and/or 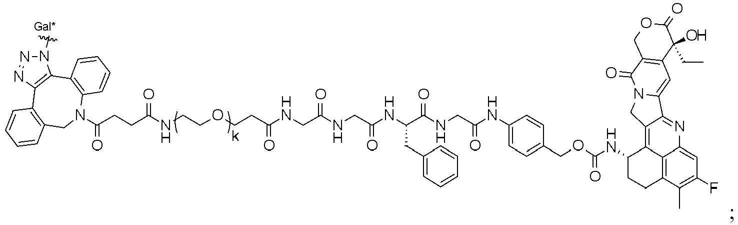
(c) 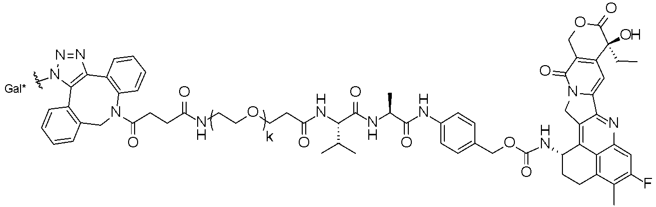 and/or 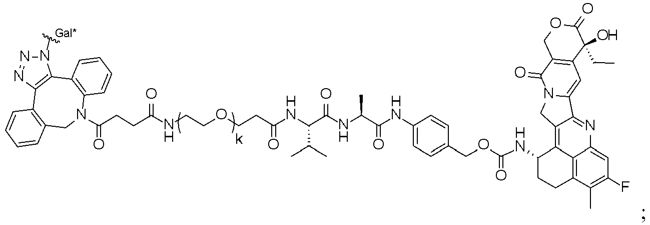
(d) 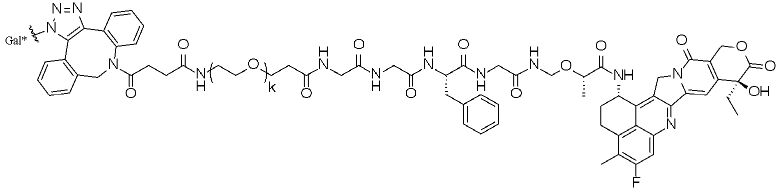 and/or 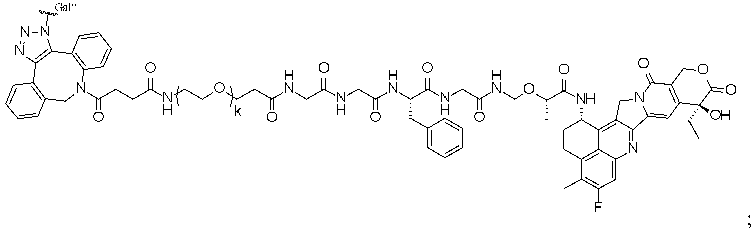
(e) 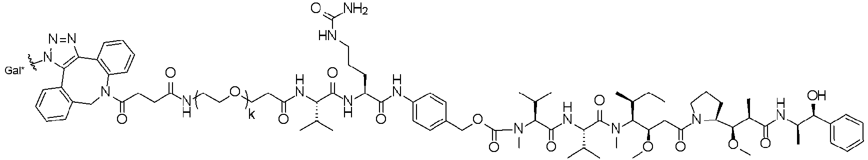 and/or 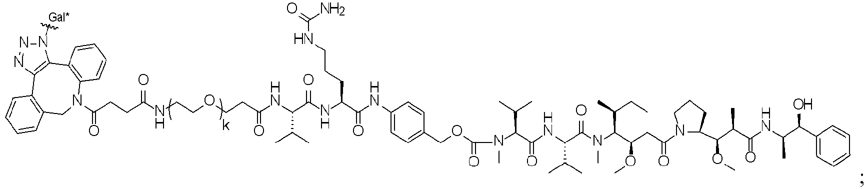
(f) 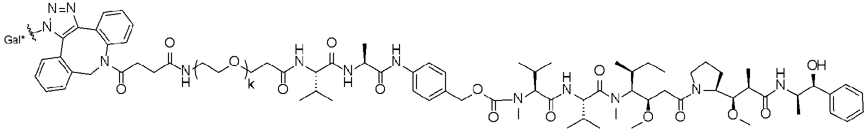 and/or 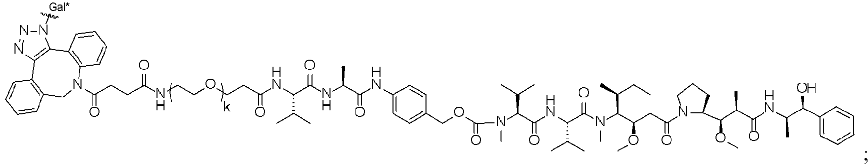 and
(g) 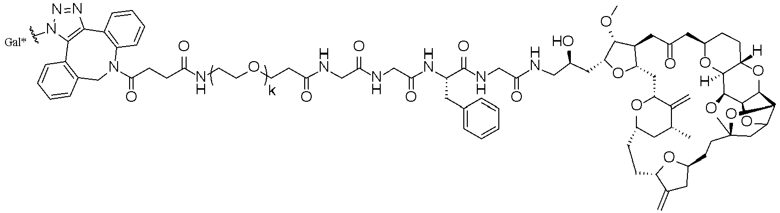 and/or 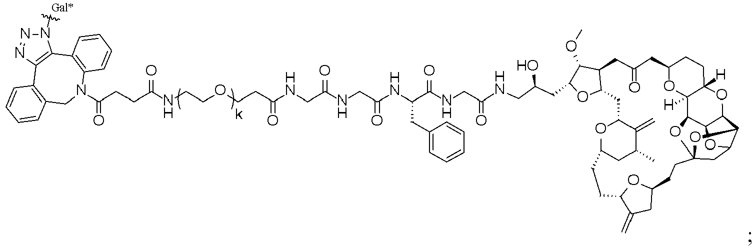 k is an integer from 1 to 20, e.g., 2, 3, 4, 5, 6, 7, 8, 9, or 10;
the core GlcNAc is directly linked to the antibody.

55. A pharmaceutical combination, comprising a programmed death ligand 1 (PD-L1)-binding molecule and a protein-drug conjugate, wherein the programmed death ligand 1-binding molecule is as defined in any one of claims 6-14, and the protein-drug conjugate is as defined in any one of claims 15-54.

56. A method for treating an advanced or metastatic solid malignancy, comprising administering to a patient in need thereof a pharmaceutical combination of a programmed death ligand 1 (PD-L1)-binding molecule and a protein-drug conjugate.

57. The method according to claim 56, wherein the PD-L1-binding molecule is as defined in any one of claims 6-14.

58. The method according to claim 56 or 57, wherein the protein-drug conjugate is as defined in any one of claims 15-54.

59. The method according to any one of claims 56-58, wherein the pharmaceutical combination is administered to the patient in need thereof once weekly, once every two weeks, once every three weeks, or once every four weeks.

60. The method according to any one of claims 56-59, wherein the pharmaceutical combination is administered by subcutaneous injection.

61. A pharmaceutical combination of a programmed death ligand 1 (PD-L1)-binding molecule and a protein-drug conjugate, being capable of being used for treating an advanced or metastatic solid malignancy.

62. Use of a programmed death ligand 1 (PD-L1)-binding molecule in combination with a protein-drug conjugate in the preparation of a medicament for treating an advanced or metastatic solid malignancy.
